(19) Europäisches Patentamt
European Patent Office
Office européen des brevets

(11) **EP 3 995 486 A1**

(12) **EUROPEAN PATENT APPLICATION**
published in accordance with Art. 153(4) EPC

(43) Date of publication:
**11.05.2022 Bulletin 2022/19**

(21) Application number: **20834188.3**

(22) Date of filing: **01.07.2020**

(51) International Patent Classification (IPC):
***C07C 231/12*** $^{(2006.01)}$ ***C07C 237/12*** $^{(2006.01)}$
***C07C 269/06*** $^{(2006.01)}$ ***C07C 271/22*** $^{(2006.01)}$
***C07K 1/06*** $^{(2006.01)}$ ***C07K 1/10*** $^{(2006.01)}$
***C07K 5/00*** $^{(2006.01)}$ ***C07K 5/08*** $^{(2006.01)}$
***C07K 5/083*** $^{(2006.01)}$

(52) Cooperative Patent Classification (CPC):
**C07C 231/12; C07C 237/12; C07C 269/06; C07C 271/22; C07K 1/06; C07K 1/10; C07K 5/00; C07K 5/08;** Y02P 20/55

(86) International application number:
**PCT/JP2020/025912**

(87) International publication number:
**WO 2021/002408 (07.01.2021 Gazette 2021/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.07.2019 JP 2019124016**
**20.11.2019 JP 2019209908**

(71) Applicants:
- **AGC INC.**
**Chiyoda-ku,**
**Tokyo 1008405 (JP)**
- **The University Of Tokyo**
**Tokyo 113-8654 (JP)**

(72) Inventors:
- **OKAZOE Takashi**
**Tokyo 100-8405 (JP)**
- **ISHIBASHI Yuichiro**
**Tokyo 100-8405 (JP)**
- **SANDO Shinsuke**
**Tokyo 113-8654 (JP)**
- **MORIMOTO Jumpei**
**Tokyo 113-8654 (JP)**
- **AIKAWA Kohsuke**
**Tokyo 113-8654 (JP)**
- **MIKAMI Toshiki**
**Tokyo 113-8654 (JP)**
- **ONO Takahiro**
**Tokyo 113-8654 (JP)**

(74) Representative: **Müller-Boré & Partner Patentanwälte PartG mbB**
**Friedenheimer Brücke 21**
**80639 München (DE)**

(54) **PEPTIDE AND METHOD FOR MANUFACTURING SAME**

(57) The present invention provides a peptide having a fluoroalkyl group as its side chain. The present invention provides a method for producing a fluoroalkyl group-containing peptide, which comprises condensing a compound represented by the formula (6-2) or (6-4):

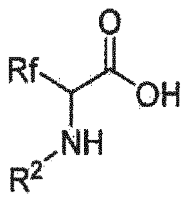
(6-2)

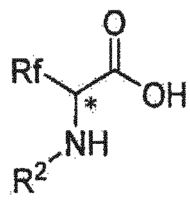
(6-4)

wherein * means that the asymmetric carbon atom marked with * has an absolute configuration of S or R, Rf is a $C_{1-30}$ alkyl group which is substituted with at least

EP 3 995 486 A1

two fluorine atoms, and which may further be substituted with a halogen atom other than a fluorine atom (when the $C_{1-30}$ alkyl group is a $C_{2-30}$ alkyl group, it may have 1 to 5 etheric oxygen atoms between carbon atoms), and $R^2$ is a protecting group for the amino group,
with a fluorinated amino acid having its carboxy group protected, an amino acid having its carboxy group protected, a fluorinated peptide having its C-terminal protected, or a peptide having its C-terminal protected.

Fig. 1

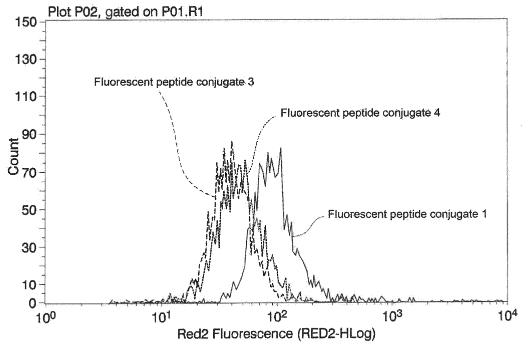

## Description

TECHNICAL FIELD

The present invention relates to a peptide containing an amino acid residue having a fluoroalkyl group introduced into its side chain, and a method for producing it.

The entire disclosures of Japanese Patent Application No. 2019-124016 filed on July 2, 2019 and Japanese Patent Application No. 2019-209908 filed on November 20, 2019, including specifications, claims, drawings and summaries are incorporated herein by reference in their entireties.

BACKGROUND ART

Antibody drugs, peptide drugs, nucleic acid drugs, etc., are excellent in that they have high specificity to target molecules existing in the cell and have less side-effects, but they do not easily reach the target molecules. In order to overcome this problem, various means have been studied. Particularly, cell penetrating peptides (CPPs) are expected to be promising. As CPPs, peptides from HIV Tat protein (Patent Document 1) and polyarginine peptides (Patent Document 2) are mentioned as representative examples. These peptides are bonded to therapeutic peptides, which are taken up by target cells (for example, Patent Document 3, Non-Patent Document 1).

Further, fluorinated amino acids are reported to exhibit specific physiological activities and attract attention. For example, 3,3,3-trifluoroalanine and its derivative are reported to act as suicide inhibitors of pyridoxal enzymes (Non-Patent Document 2). Further, it was reported that alanine racemase of gram-negative bacterium Salmonella typhimurium and gram-positive bacterium Bacillus stearothermophilus are inactivated by 3,3,3-trifluoroalanine (Non-Patent Document 3). Fluorinated amino acids and peptides containing them are expected to be useful in medical fields as physiologically active substances.

A compound having a polyfluoro structure is known to be stable and is less toxic in the body and is excellent in uptake by the cells and transport out of endosomes (Non-Patent Document 4). It has been reported that by utilizing such properties, a peptide dendrimer using lysine having an amino acid group as its side chain perfluoroacylated as the constituting amino acid, can be used to deliver the genes (Non-Patent Document 5). However, since it is a dendrimer, it can not form a hybrid by connecting with a therapeutic peptide, a nucleic acid or a protein to be an antibody drug, like CPPs.

PRIOR ART DOCUMENTS

PATENT DOCUMENTS

Patent Document 1: U. S. Patent No. 6,316,003
Patent Document 2: U. S. Patent No. 6,306,993
Patent Document 3: WO2008/089491

NON-PATENT DOCUMENTS

Non-Patent Document 1: Miyaji et al., Drug Metabolism and Disposition, 2011, vol. 39, p. 1946-1953.
Non-Patent Document 2: Sakai et al., Tetrahedron, 1996, vol. 52 (1), p. 233-244.
Non-Patent Document 3: Faraci and Walsh, Biochemistry, 1989, vol. 28 (2), p. 431-437.
Non-Patent Document 4: Zhang et al., MRS Communications, 2018, vol. 8, p. 303-313.
Non-Patent Document 5: Cai et al., ACS Applied Materials and Interfaces, 2016, vol. 8, p. 5821-5832.

DISCLOSURE OF INVENTION

TECHNICAL PROBLEM

The CPPs disclosed in Patent Document 1 and the like have various problems such as transport efficiency into the cells, and decomposition by peptidase in the body.

The object of the present invention is to provide a peptide containing an amino acid residue having a fluoroalkyl

group introduced into its side chain, and a method for producing it.

SOLUTION TO PROBLEM

The present inventors have conducted studies to produce a peptide containing an amino acid residue having a fluoroalkyl group introduced into its side chain and as a result, found that such a peptide is excellent in cell permeability and accomplished the present invention.

That is, the present invention provides the following.

[1] A method for producing a fluoroalkyl group-containing peptide, which comprises condensing a compound represented by the following formula (6-2) or (6-4):

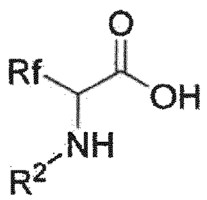
(6-2)

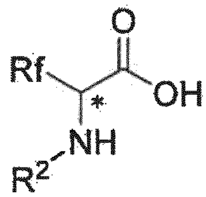
(6-4)

wherein asterisk means that the asymmetric carbon atom marked with the asterisk has an absolute configuration of S or R,

Rf is a $C_{1-30}$ alkyl group which is substituted with at least two fluorine atoms, and which may further be substituted with a halogen atom other than a fluorine atom (when the $C_{1-30}$ alkyl group is a $C_{2-30}$ alkyl group, it may have 1 to 5 etheric oxygen atoms between carbon atoms), and
$R^2$ is a protecting group for the amino group,
with a fluorinated amino acid having its carboxy group protected, an amino acid having its carboxy group protected, a fluorinated peptide having its C-terminal protected, or a peptide having its C-terminal protected.

[2] A method for producing a fluoroalkyl group-containing peptide, which comprises condensing a compound represented by the following formula (6-1) or (6-3):

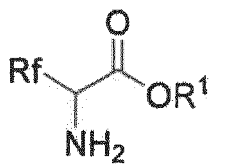
(6-1)

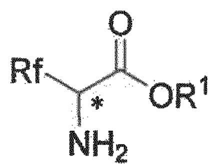
(6-3)

wherein asterisk means that the asymmetric carbon atom marked with the asterisk has an absolute configuration of S or R,

Rf is a $C_{1-30}$ alkyl group which is substituted with at least two fluorine atoms, and which may further be substituted with a halogen atom other than a fluorine atom (when the $C_{1-30}$ alkyl group is a $C_{2-30}$ alkyl group, it may have 1 to 5 etheric oxygen atoms between carbon atoms), and
$R^1$ is a protecting group selected from a group represented by the following formula (p-1):

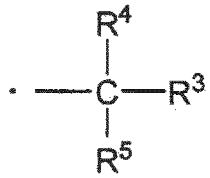 (p-1)

(wherein $R^3$ is a $C_{6-14}$ aryl group which may be substituted, $R^4$ and $R^5$ are each independently a hydrogen atom or a $C_{6-14}$ aryl group which may be substituted, and the black circle means a binding site), a 2-(9,10-dioxo)anthrylmethyl group, a benzyloxymethyl group and a phenacyl group,
with a fluorinated amino acid having its amino group protected, an amino acid having its amino group protected, a fluorinated peptide having its N-terminal protected, or a peptide having its N-terminal protected.

[3] A method for producing a fluoroalkyl group-containing peptide, which comprises protecting, of a compound represented by the following formula (7) or (7-1):

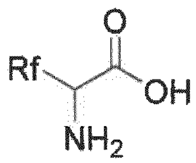
(7)

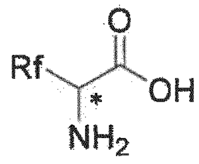
(7-1)

wherein asterisk means that the asymmetric carbon atom marked with the asterisk has an absolute configuration of S or R, and

Rf is a $C_{1-30}$ alkyl group which is substituted with at least two fluorine atoms, and which may further be substituted with a halogen atom other than a fluorine atom (when the $C_{1-30}$ alkyl group is a $C_{2-30}$ alkyl group, it may have 1 to 5 etheric oxygen atoms between carbon atoms),
the amino group with a protecting group, and then condensing the compound with a fluorinated amino acid having its carboxy group protected, an amino acid having its carboxy group protected, a fluorinated peptide having its C-terminal protected, or a peptide having its C-terminal protected, or
the carboxy group with a protecting group, and then condensing the compound with a fluorinated amino acid having its amino group protected, an amino acid having its amino group protected, a fluorinated peptide having its N-terminal protected, or a peptide having its N-terminal protected.

[4] The method for producing a fluoroalkyl group-containing peptide according to any one of the above [1] to [3], which further comprises removing the protecting group for the amino group or for the carboxy group of the produced fluoroalkyl group-containing peptide by deprotection.
[5] A peptide having two or more amino acids bonded by a peptide bond, wherein at least one of amino acid residues constituting the peptide has, as its side chain, a $C_{1-30}$ alkyl group substituted with at least two fluorine atoms (when the $C_{1-30}$ alkyl group is a $C_{2-30}$ alkyl group, it may have 1 to 5 etheric oxygen atoms between carbon atoms).
[6] The peptide according to the above [5], wherein the $C_{1-30}$ alkyl group substituted with at least two fluorine atoms may further be substituted with a halogen atom other than a fluorine atom.
[7] The peptide according to the above [5] to [6], wherein the side chain consisting of the $C_{1-30}$ alkyl group substituted with at least two fluorine atoms is a group represented by the following formula (f-1) or (f-2):

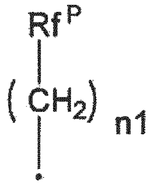
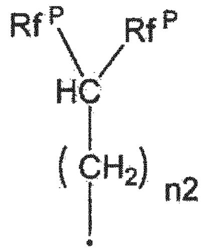

(f-1) (f-2)

wherein $Rf^P$ is a perhalogenated $C_{1-10}$ alkyl group containing at least two fluorine atoms (when the $C_{1-10}$ alkyl group has two or more carbon atoms, it may have an etheric oxygen atom between carbon atoms), n1 is an integer of from 0 to 10, n2 is an integer of from 0 to 9, and the black circle means a binding site.

[8] The peptide according to any one of the above [5] to [7], of which the C-terminal or the N-terminal may be protected with a protecting group.

[9] The peptide according to any one of the above [5] to [8], which is a tripeptide represented by the following formula (101) or (102):

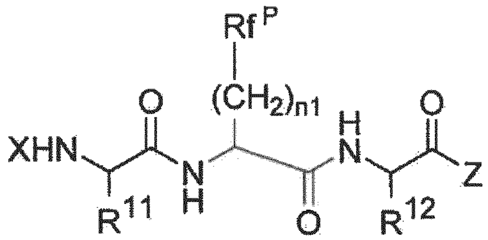
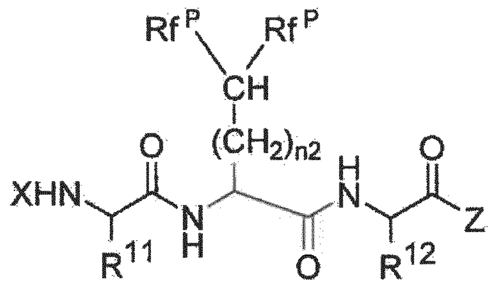

(101) (102)

wherein $Rf^P$ is a perhalogenated $C_{1-10}$ alkyl group containing at least two fluorine atoms (when the $C_{1-10}$ alkyl group has two or more carbon atoms, it may have an etheric oxygen atom between carbon atoms), n1 is an integer of from 0 to 10, n2 is an integer of from 0 to 9, $R^{11}$ and $R^{12}$ are each independently a $C_{1-6}$ alkyl group or a benzyl group, X is a 9-fluorenylmethyloxycarbonyl group or a tert-butoxycarbonyl group, and Z is a $C_{1-6}$ alkoxy group.

[10] The peptide according to any one of the above [5] to [9], which is a cell penetrating peptide.

## ADVANTAGEOUS EFFECTS OF INVENTION

By the production method according to the present invention, it is possible to produce a peptide having a fluoroalkyl group introduced into its side chain.

Further, the peptide according to the present invention, which has a fluoroalkyl group introduced into its side chain, is excellent in cell permeability. Accordingly, the peptide is expected to be useful in medical fields as a physiologically active substance.

## BRIEF DESCRIPTION OF DRAWINGS

Fig. 1 is a chart illustrating the results of flow cytometry of HeLa cells treated with fluorescent peptide conjugate 1 (Alexa-Ala-RFAA-Phe-OMe), fluorescent peptide conjugate 3 (Alexa-Ala-Nle-Phe-OMe) and fluorescent peptide conjugate 4 (Alexa-Ala-Ala-Phe-OMe) in Test Example 1.

Fig. 2 is a chart illustrating the results of flow cytometry of HeLa cells treated with fluorescent peptide conjugate 1 (Alexa-Ala-RFAA-Phe-OMe), fluorescent peptide conjugate 2 (Alexa-Ala-RFAA (C8)-Phe-OMe), fluorescent peptide conjugate 3 (Alexa-Ala-Nle-Phe-OMe) and fluorescent peptide conjugate 4 (Alexa-Ala-Ala-Phe-OMe) in Test Example 2.

DESCRIPTION OF EMBODIMENTS

In the present invention and in this specification, a "fluorinated amino acid" means an amino acid having at least two fluorine atoms in its side chain. A "fluorinated peptide" means a peptide having an amino acid having at least two fluorine atoms in its side chain.

In the present invention and in this specification, "$C_{p1-p2}$" (p1 and p2 are positive integers which satisfy p1<p2) means a group having p1 to p2 carbon atoms.

In the present invention and in this specification, a "$C_{1-10}$ alkyl group" means an alkyl group having from 1 to 10 carbon atoms and may be linear or branched. A "$C_{2-10}$ alkyl group" means an alkyl group having from 2 to 10 carbon atoms and may be linear or branched. The $C_{1-10}$ alkyl group may, for example, be a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group or a decyl group.

In the present invention and in this specification, a "$C_{1-30}$ alkyl group" is an alkyl group having from 1 to 30 carbon atoms and may be linear or branched. A "$C_{2-30}$ alkyl group" is an alkyl group having from 2 to 30 carbon atoms and may be linear or branched. The $C_{1-30}$ alkyl group may, for example, be a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a tricosyl group, a tetracosyl group, a pentacosyl group, a hexacosyl group, a heptacosyl group, an octacosyl group, a nonacosyl group or a triacontyl group.

In the present invention and in this specification, a "$C_{1-6}$ alkyl group" is an alkyl group having from 1 to 6 carbon atoms and may be linear or branched. The $C_{1-6}$ alkyl group may, for example, be a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group or a hexyl group.

In the present invention and in this specification, a "$C_{6-14}$ aryl group" means an aromatic hydrocarbon group having from 6 to 14 carbon atoms and is particularly preferably a $C_{6-12}$ aryl group. The $C_{6-14}$ aryl group may, for example, be a phenyl group, a naphthyl group, an anthryl group or a 9-fluorenyl group, and is particularly preferably a phenyl group.

In the present invention and in this specification, a "$C_{6-14}$ aryl group which may be substituted" is a group having one or more, preferably from 1 to 3, hydrogen atoms bonded to a carbon atom of a $C_{6-14}$ aryl group replaced with another functional group. When the aryl group has two or more substituents, the substituents may be of the same type or different types. The substituent may, for example, be a nitro group, a halogen atom (a fluorine atom, a chlorine atom, a bromine atom or an iodine atom), a $C_{1-6}$ alkyl group, a $C_{1-6}$ alkoxy group, or a methylenedioxy group (-O-CH2-O-). The "$C_{6-14}$ aryl group which may be substituted" may, for example, be a phenyl group, a naphthyl group, an anthryl group, a 4-nitrophenyl group, a 4-methoxyphenyl group, a 2,4-dimethoxyphenyl group, a 3,4-dimethoxyphenyl group, a 4-methylphenyl group, a 2,6-dimethylphenyl group, a 3-chlorophenyl group or a 1,3-benzodioxol-5-yl group.

In the present invention and in this specification, a "$C_{6-14}$ aryl-$C_{1-6}$ alkyl group" is a group having one hydrogen atom bonded to a carbon atom of a $C_{1-6}$ alkyl group replaced with a $C_{6-14}$ aryl group. The $C_{6-14}$ aryl group in the $C_{6-14}$ aryl-$C_{1-6}$ alkyl group may, for example, be a phenyl group, a naphthyl group, an anthryl group or a 9-fluorenyl group, and is particularly preferably a phenyl group or a 9-fluorenyl group. The $C_{1-6}$ alkyl group in the $C_{6-14}$ aryl-$C_{1-6}$ alkyl group is preferably a $C_{1-4}$ alkyl group. The $C_{6-14}$ aryl-$C_{1-6}$ alkyl group may, for example, be a benzyl group, a diphenylmethyl group, a triphenylmethyl group, a 2-phenylethyl group, a 9-anthrylmethyl group or a 9-fluorenylmethyl group.

In the present invention and in this specification, a "halogen atom" means a fluorine atom, a chlorine atom, a bromine atom or an iodine atom. A "halogen atom other than a fluorine atom" means a chlorine atom, a bromine atom or an iodine atom. The "halogen atom other than a fluorine atom" is preferably a chlorine atom or a bromine atom, particularly preferably a chlorine atom.

In the present invention and in this specification, a "$C_{1-6}$ alkoxy group" means a group having an oxygen atom bonded to the bond terminal of a $C_{1-6}$ alkyl group having from 1 to 6 carbon atoms. The $C_{1-6}$ alkoxy group may be linear or branched. The $C_{1-6}$ alkoxy group may, for example, be a methoxy group, an ethoxy group, a propoxy group, a butoxy group, a tert-butoxy group, a pentyloxy group or a hexyloxy group.

In the present invention and in this specification, an "etheric oxygen atom" means an oxygen atom linking carbon atoms, and does not include oxygen atoms directly bonded in series. The number of etheric oxygen atom which an alkyl group having Nc (Nc is an integer of 2 or more) carbon atoms may have is Nc-1 at the most.

Further, in the following, "compound n" means a compound represented by the formula (n).

<Reaction for synthesis of fluoroalkyl group-containing amino acid>

A fluoroalkyl group-containing amino acid which is an amino acid having a fluoroalkyl group introduced into its side chain may be produced, for example, by the following synthesis reaction.

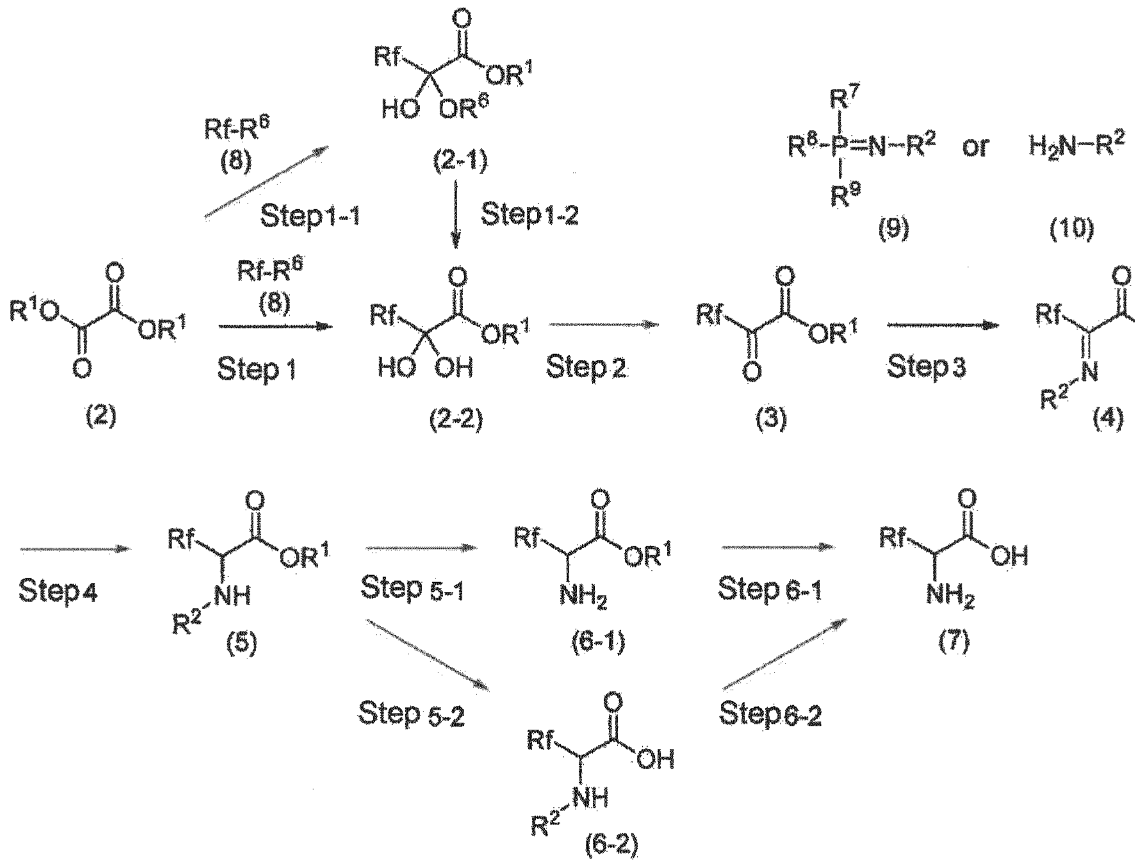


Rf is a $C_{1-30}$ alkyl group, in which at least two of hydrogen atoms bonded to a carbon atom are replaced with a fluorine atom, and in which at least one of hydrogen atoms bonded to a carbon atom may further be replaced with a halogen atom other than a fluorine atom. The $C_{1-30}$ alkyl group in Rf is preferably a $C_{1-20}$ alkyl group, more preferably a $C_{1-10}$ alkyl group, further preferably a $C_{2-10}$ alkyl group, still more preferably a $C_{2-8}$ alkyl group. When the $C_{1-30}$ alkyl group is a $C_{2-30}$ alkyl group, it may have 1 to 5 etheric oxygen atoms between carbon atoms. In Rf, the number of hydrogen atoms replaced with a fluorine atom is not particularly limited so long as it is 2 or more, and for example, preferably 3 or more, more preferably 6 or more, further preferably 7 or more.

Rf may, for example, be a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, a perfluorodecyl group, a difluoromethyl group, a 1,1-difluoroethyl group, a 2,2-difluoroethyl group, a 1,1,2,2-tetrafluoroethyl group, a 1,1,2,2,3,3-hexafluoropropyl group, a 1,1,2,3,3,3-hexafluoropropyl group, a 1,1,2,2,3,3-hexafluorohexyl group, a 1,1,2,2,3,3-hexafluorooctyl group, a 1,1,2,2,3,3-hexafluorodecyl group, a 1,1,2,2,3,3-hexafluorooctadecyl group or a 1,1,2,2,3,3-hexafluorohexacosyl group.

When Rf is a group having 2 carbon atoms, Rf in compound 2 is preferably a group having at least 4 hydrogen atoms bonded to a carbon atom replaced with a fluorine atom, such as a pentafluoroethyl group, rather than a 1,1,1-trifluoroethyl group ($CF_3$-$CH_2$-). Further, when Rf is a compound having 3 carbon atoms, Rf in the compound 2 is preferably a linear group, and when it is a branched group, it is preferably a group having no or one trifluoromethyl group, rather than a group having two trifluoromethyl groups, such as a 1,1,1,3,3,3-hexafluoropropan-2-yl group (($CF_3$)$_2$-CH-). When Rf is a group having 4 carbon atoms, Rf in the compound 2 is preferably a linear group, and when it is a branched group, it is preferably a group in which a hydrogen atom bonded to a carbon atom constituting the alkylene group moiety is replaced with a fluorine atom, or a perfluorinated group.

Rf is, specifically, preferably a group represented by the formula (f-1) or (f-2) shown hereinafter.

$R^1$ is a protecting group for a carboxy group and is specifically a protecting group selected form a group represented by the following formula (p-1), a 2-(9,10-dioxo)anthrylmethyl group, a benzyloxymethyl group and a phenacyl

group. In the formula (p-1), $R^3$ is a $C_{6-14}$ aryl group which may be substituted, and $R^4$ and $R^5$ are each independently a hydrogen atom or a $C_{6-14}$ aryl group which may be substituted. Further, the black circle means a binding site.

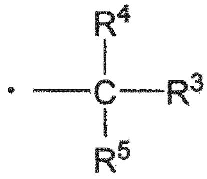

(p-1)

The protecting group for a carboxy group represented by $R^1$ may, for example, be a benzyl group, a diphenylmethyl group, a triphenylmethyl group, a 4-nitrobenzyl group, a 4-methoxybenzyl group, a 2,4-dimethoxybenzyl group, a 3,4-dimethoxybenzyl group, a 4-methylbenzyl group, a 2,6-dimethylbenzyl group, a 3-chlorobenzyl group, a 9-anthrylmethyl group, a piperonyl group, a 2-(9,10-dioxo)anthrylmethyl group, a benzyloxymethyl group or a phenacyl group. $R^1$ is preferably a benzyl group or a triphenylmethyl group, more preferably a benzyl group, which can be removed by deprotection under mild conditions.

The production method is advantageous in that by using an aralkyl protecting group such as a benzyl group or a triphenylmethyl group as the protecting group $R^1$ for a carboxy group, $R^1$ can be removed by deprotection under mild conditions, and synthesis of the fluorinated amino acid and synthesis of the fluorinated peptide can be conducted without decomposing the functional group of the amino acid.

$R^6$ is a silyl protecting group. $R^6$ may, for example, be a trimethylsilyl (TMS) group, a triethylsilyl (TES) group, a triisopropylsilyl (TIPS) group, a tert-butyldimethylsilyl (TBDMS) group or a tert-butyldiphenylsilyl (TBDPS) group. $R^6$ is preferably a trimethylsilyl (TMS) group.

$R^2$ is a protecting group for an amino group. $R^2$ is not particularly limited so long as it is a protecting group for an amino group to be used for peptide synthesis. The protecting group for an amino group may, for example, be a carbamate protecting group such as a tert-butoxycarbonyl (Boc) group, a 9-fluorenylmethyloxycarbonyl (Fmoc) group, a benzyloxycarbonyl (Cbz) group, an allyloxycarbonyl (Alloc) group or a 2,2,2-trichloroethoxycarbonyl (Troc) group. $R^2$ is preferably a tert-butoxycarbonyl (Boc) group or a 9-fluorenylmethyloxycarbonyl (Fmoc) group, which can be removed by deprotection under mild conditions.

[Step 1]

By reacting the compound 2 and compound 8 in the presence of a metal fluoride, compound 2-2 can be obtained. The compound 8 represented by Rf-$R^6$ of the formula (8) can be synthesized from an easily available Rf-I (fluoroalkyl iodide) in one step, and accordingly the range of the Rf groups which can be introduced is wide.

As the metal fluoride, an alkali metal fluoride such as cesium fluoride, lithium fluoride or sodium fluoride may be used, and cesium fluoride is preferred.

The reaction may be conducted in a solvent inert to the reaction. The solvent may be an inert solvent such as tetrahydrofuran (THF), dichloromethane (DCM), acetonitrile, benzene, toluene, diethyl ether, 1,4-dioxane, N,N-dimethylformamide or N,N-dimethylacetamide, and is preferably tetrahydrofuran.

The amount of the compound 8 is preferably from 0.5 to 10 moles per mole of the compound 2. The amount of the metal fluoride is preferably from 0.01 to 2 moles per mole of the compound 2. The reaction of step 1 is conducted preferably at a temperature of 10°C or lower. By conducting the reaction at a temperature of 10°C or lower, the compound 2-2 can be produced with a high yield. The reaction temperature is preferably from -78°C to 10°C, more preferably from -50°C to -10°C, particularly preferably from -40°C to -20°C. The reaction time is preferably from 1 to 48 hours, more preferably form 6 to 36 hours.

The compound 2 may be produced by diesterifying oxalic acid by a known method, or a commercial product may be used.

[Step 1-1]

In the reaction of step 1, compound 2-1 (a compound having one of hydroxy groups protected with $R^6$), or a mixture of the compound 2-2 and the compound 2-1 may be obtained in some cases. In such a case, the silyl protecting group $R^6$ for the compound 2-1 is removed by deprotection to obtain the compound 2-2.

The reaction of step 1-1 may be conducted in the same manner as in step 1.

[Step 1-2]

By removing the silyl protecting group $R^6$ for the compound 2-1 by deprotection, the compound 2-2 can be obtained.
Deprotection may be conducted in the presence of a fluoride such as tetrabutylammonium fluoride (TBAF), cesium fluoride or a hydrofluoride, or an acid such as hydrochloric acid, acetic acid or p-toluenesulfonic acid.
The reaction may be conducted in a solvent inert to the reaction. The solvent may be an inert solvent such as tetrahydrofuran, dichloromethane, acetonitrile, benzene, toluene, diethyl ether, 1,4-dioxane, N,N-dimethylformamide or N,N-dimethylacetamide, and is preferably tetrahydrofuran. It is preferred to add acetic acid.
The amount of the fluoride is, per mole of the compound 2-1 (in the case of a mixture of the compound 2-2 and the compound 2-1, per mole of the mixture), preferably from 0.1 to 10 moles. The amount of the acid is, per mole of the compound 2-1 (in the case of a mixture of the compound 2-2 and the compound 2-1, per mole of the mixture), preferably from 0.1 to 10 moles. The reaction of step 1-2 is conducted preferably at a temperature of 50°C or lower. By conducting the reaction at a temperature of 50°C or lower, the compound 2-2 can be produced with a high yield. The reaction temperature is preferably from -80°C to 50°C, more preferably from -40°C to 30°C, particularly preferably from -20°C to 30°C. The reaction time is preferably from 1 to 48 hours, more preferably from 6 to 36 hours.

[Step 2]

By subjecting the compound 2-2 to dehydration reaction, compound 3 can be obtained.
The dehydration reaction may be conducted in the presence of a dehydrating agent such as diphosphorus pentoxide, concentrated sulfuric acid, calcium chloride, sodium sulfate, magnesium sulfate, calcium sulfate, molecular sieve (synthetic zeolite) or silica gel. The dehydrating agent is preferably diphosphorus pentoxide. The amount of the dehydrating agent is preferably from 10 to 100 wt% to 100 wt% of the compound 2-2. The dehydrating reaction may be conducted by distilling the compound 2-2 in the presence of the dehydrating agent. Distillation is conducted preferably at a temperature of from 30°C to 150°C. If the distillation temperature is too high, the compound 3 may decompose. If the distillation temperature is too low, the compound 3 will not be condensed, and the recovery rate may decrease. Distillation may be conducted under any pressure of reduced pressure, normal pressure and elevated pressure, and may properly be determined so that the boiling point of the compound 3 will be within the above preferred temperature range. The pressure is preferably from 0.1 mmHg to 5 atm (3,800 mmHg).

[Step 3]

By reacting the compound 3 with compound 9 or compound 10, compound 4 can be obtained.
In the formula (9), $R^2$ is, as described above, a protecting group for an amino group. $R^7$, $R^8$ and $R^9$ are each independently a $C_{6-14}$ aryl group. The $C_{6-14}$ aryl group represented by $R^7$, $R^8$ or $R^9$ may, for example, be a phenyl group or a naphthyl group. $R^7$, $R^8$ and $R^9$ are each preferably a phenyl group.
The reaction may be conducted in a solvent inert to the reaction. The solvent may be an inert solvent such as diethyl ether, tetrahydrofuran, dichloromethane, acetonitrile, benzene, toluene, 1,4-dioxane, N,N-dimethylformamide or N,N-dimethylacetamide, and is preferably diethyl ether.
The amount of the compound 9 or the compound 10 is preferably from 0.5 to 10 moles per mole of the compound 3. The reaction temperature is preferably from -78°C to 100°C, more preferably from 0°C to 40°C. The reaction time is preferably from 1 minute to 24 hours, more preferably from 10 minutes to 4 hours.
In a preferred embodiment, by using a carbamate protecting group such as a tert-butoxycarbonyl group or a 9-fluorenylmethyloxycarbonyl group as the protecting group $R^2$ for an amino group, $R^2$ can be removed by deprotection under mild conditions, and synthesis of the fluorinated amino acid can be carried out while decomposition and racemization of the compound are suppressed.

[Step 4]

By subjecting the compound 4 to reduction reaction, compound 5 can be obtained.
The reduction reaction may be conducted by a method of using a reducing agent or a method of conducting reduction in the presence of a metal catalyst.

(1) Method using reducing agent

As the reducing agent, a borohydride reagent such as sodium borohydride, zinc borohydride, sodium cyanoborohydride, lithium triethylborohydride, lithium tri(sec-butyl)borohydride, potassium tri(sec-butyl)borohydride, lithium borohydride or sodium triacetoxyborohydride may be used. The reducing agent is preferably sodium borohydride or zinc borohydride, more preferably sodium borohydride. The amount of the reducing agent is preferably from 0.5 to 10 moles per mole of the compound 4.

The reaction may be conducted in a solvent inert to the reaction. The solvent may be an inert solvent such as diethyl ether, tetrahydrofuran, a hydrochlorofluorocarbon (HCFC) (for example, ASAHIKLIN (registered trademark) AK-225 (a mixture of 3,3-dichloro-1,1,1,2,2-pentafluoropropane and 1,3-dichloro-1,1,2,2,3-pentafluoropropane, AGC Inc.)), dichloromethane, acetonitrile, 1,4-dioxane, N,N-dimethylformamide, N,N-dimethylacetamide, and is preferably diethyl ether.

The reaction temperature is preferably from -78°C to 100°C, more preferably from -10°C to 40°C. The reaction time is preferably from 1 to 48 hours, more preferably from 6 to 36 hours.

(2) Method of conducting reduction in the presence of metal catalyst

The metal catalyst may, for example, be a palladium catalyst (such as palladium carbon, palladium hydroxide, Pearlman's catalyst, Lindlar's catalyst, silica gel-supported palladium catalyst, alumina-supported palladium catalyst or palladium oxide), a nickel catalyst (such as Raney nickel), a platinum catalyst (such as platinum carbon, platinum oxide, silica gel-supported platinum catalyst or alumina-supported platinum catalyst), a rhodium catalyst (such as rhodium carbon, alumina-supported rhodium catalyst or rhodium oxide), a ruthenium catalyst (such as ruthenium carbon, alumina-supported ruthenium catalyst or ruthenium oxide), or a cobalt catalyst (such as Raney cobalt), and is preferably a palladium catalyst. The amount of the metal catalyst is preferably from 0.0001 to 0.1 mole, more preferably from 0.0005 to 0.02 mole per mole of the compound 4.

The reaction may be conducted in a solvent inert to the reaction. The solvent may be an inert solvent such as methanol, ethanol, isopropanol, diethyl ether, tetrahydrofuran, ethyl acetate, dichloromethane, acetonitrile, 1,4-dioxane, N,N-dimethylformamide or N,N-dimethylacetamide.

The reduction reaction is conducted in the presence of hydrogen gas. The reduction reaction may be conducted under normal pressure or elevated pressure. The pressure of the hydrogen gas is preferably from 0.5 atm to 10 atm. The reaction temperature is preferably from 0°C to 100°C, more preferably from 10°C to 50°C. The reaction time is preferably from 1 to 48 hours, more preferably from 6 to 36 hours.

[Step 5-1]

By removing the protecting group $R^2$ for the compound 5 by deprotection, compound 6-1 can be obtained.

Deprotection may be conducted depending upon the type of the protecting group $R^2$.

When $R^2$ is a Boc group, deprotection may be conducted under acidic conditions. The acid used may, for example, be trifluoroacetic acid (TFA) or hydrochloric acid. The amount of the acid is preferably from 1 to 1,000 moles per mole of the compound 5.

The reaction may be conducted in a solvent inert to the reaction. The solvent may be an inert solvent such as diethyl ether, tetrahydrofuran, dichloromethane, acetonitrile, benzene, toluene, 1,4-dioxane, N,N-dimethylformamide or N,N-dimethylacetamide, and is preferably dichloromethane or N,N-dimethylformamide. An acid may be used as the solvent. The solvent may be an inorganic acid such as hydrochloric acid, acetic acid or trifluoroacetic acid, or an organic acid, and is preferably trifluoroacetic acid. The reaction temperature is preferably from -78°C to 50°C, more preferably from 0°C to 40°C. The reaction time is preferably from 1 to 48 hours, more preferably from 6 to 36 hours.

When $R^2$ is a Fmoc group, deprotection may be conducted under basic conditions. The base to be used may be a secondary amine such as piperidine, morpholine or pyrrolidine. The amount of the base is preferably from 1 to 100 moles per mole of the compound 5.

The reaction may be conducted in a solvent inert to the reaction. The solvent may be an inert solvent such as diethyl ether, tetrahydrofuran, dichloromethane, acetonitrile, benzene, toluene, 1,4-dioxane, N,N-dimethylformamide or N,N-dimethylacetamide. The reaction temperature is preferably from -20°C to 80°C, more preferably from 0°C to 40°C. The reaction time is preferably from 1 minute to 24 hours, more preferably from 5 minutes to 2 hours.

[Step 6-1]

By removing the protecting group $R^1$ for the compound 6-1 by deprotection, compound 7 can be obtained.

Deprotection may be conducted depending upon the type of the protecting group $R^1$. When $R^1$ is a benzyl group, a triphenylmethyl group, a 9-anthrylmethyl group, a piperonyl group, a 2-(9,10-dioxo)anthrylmethyl group, a benzyloxymethyl group or a phenacyl group, deprotection may be carried out by a method of conducting reduction in the presence of a metal catalyst. The reduction reaction may be conducted in the same manner as the method of conducting reduction in the presence of a metal catalyst in step 4.

[Step 5-2]

By removing the protecting group $R^1$ for the compound 5 by deprotection, compound 6-2 can be obtained. Deprotection may be conducted in the same manner as in step 6-1.

[Step 6-2]

By removing the protecting group $R^2$ for the compound 6-2 by deprotection, compound 7 can be obtained. Deprotection may be conducted in the same manner as in step 5-1.

By conducting asymmetric reduction of the imine represented by the formula (4) (compound 4), an optically active fluorinated amino acid (fluoroalkyl group-containing compound) can be synthesized. In the following reaction formula, the asterisk (*) means that the asymmetric carbon atom marked with the asterisk has an absolute configuration of S or R. Further, Rf, $R^1$, and $R^2$ are as defined above.

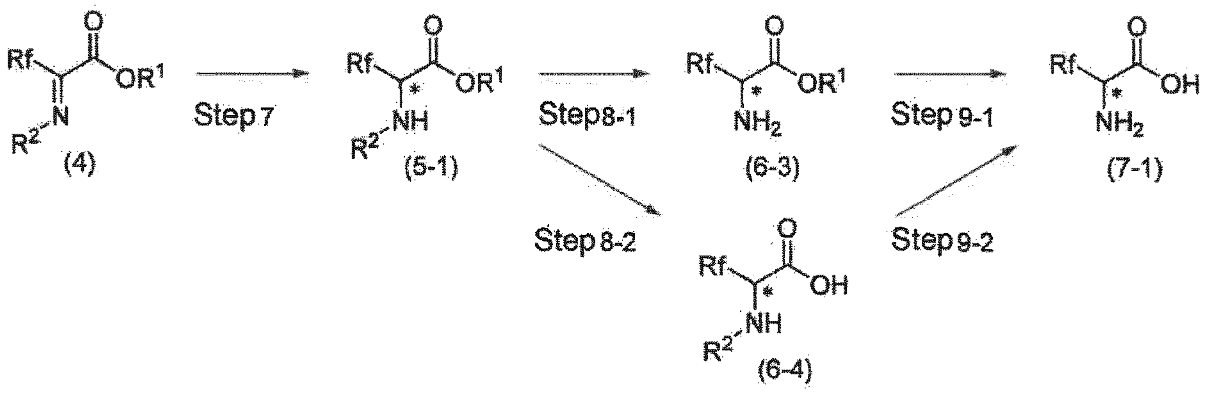


In the production method, use of an aralkyl protecting group such as a benzyl group or a triphenylmethyl group as the protecting group $R^1$ for a carboxy group, is advantageous in that $R^1$ can be removed by deprotection under mild conditions, and synthesis of the fluorinated amino acid and synthesis of the fluorinated peptide can be conducted while optical activity is maintained.

[Step 7]

By subjecting the compound 4 to asymmetric reduction reaction, compound 5-1 can be obtained.

Asymmetric reduction reaction may be conducted by reducing the compound 4 in the presence of an asymmetric reduction catalyst.

As the asymmetric reduction catalyst, a transition metal complex having an asymmetric ligand coordinated to a transition metal may be used. The transition metal may, for example, be palladium, rhodium, ruthenium, iridium, nickel, cobalt, platinum or iron. The transition metal complex may, for example, be a palladium complex, a rhodium complex, a ruthenium complex, an iridium complex or a nickel complex.

The asymmetric ligand may be DPEN (1,2-diphenylethylenediamine), DAIPEN (1,1-di(4-anisyl)-2-isopropyl-1,2-ethylenediamine) or an optically active phosphine ligand. The optically active phosphine ligand may, for example, be 2,2'-bis(diphenylphosphino)-1,1'-binaphthyl (BINAP), 2,2'-bis(diphenylphosphino)-5,5',6,6',7,7',8,8'-octahydro-1,1'-binaphthyl ($H_8$-BINAP), 2,2'-bis(di-p-tolylphosphino)-1,1'-binaphthyl (Tol-BINAP), 2,2'-bis[bis(3,5-dimethylphenyl)phosphino]-1,1'-binaphthyl (Xyl-BINAP), 2,2'-bis[bis(3,5-di-tert-butyl-4-methoxyphenyl)phosphino]-1,1'-binaphthyl (DTBM-BINAP), 1,2-bis(anisylphosphino)ethane (DIPAMP), 2,3-bis(diphenylphosphino)butane (CHIRAPHOS), 1-cyclohexyl-1,2-bis(diphenylphosphino)ethane (CYCPHOS), 1,2-bis(diphenylphosphino)propane (PROPHOS), 2,3-bis(diphenylphosphino)-5-norbornene (NORPHOS), 2,3-O-isopropylidene-2,3-dihydroxy-1,4-bis(diphenylphosphino)butane (DIOP), 1-[1',2-bis(diphenylphosphino)ferrocenyl]ethylamine (BPPFA), 1-[1',2-bis(diphenylphosphino)ferrocenyl]ethyl alcohol (BPPFOH), 2,4-bis-(diphenylphosphino)pentane (SKEWPHOS), 1,2-bis(substituted phosphorano)benzene (DuPHOS), 5,5'-bis(diphenylphosphino)-4,4'-bi-1,3-benzodioxole (SEGPHOS), 5,5'-bis[di(3,5-xylyl)phosphino]-4,4'-bi-1,3-benzodioxole (DM-SEGPHOS), 5,5'-bis[bis(3,5-di-tert-butyl-4-methoxyphenyl)phosphino]-4,4'-bi-1,3-benzodioxole (DTBM-SEGPHOS), 1-[2-(2-substituted phosphino)ferrocenyl]ethyl-2-substituted phosphine (Josiphos), or 1-[2-(2'-2-substituted phosphinophenyl)ferrocenyl]ethyl-2-substituted phosphine (Walphos).

The amount of the asymmetric reduction catalyst is preferably from 0.0001 to 0.1 moles, more preferably from 0.0005 to 0.02 moles per mole of the compound 4.

The reaction may be conducted in a solvent inert to the reaction. The solvent may be an inert solvent such as methanol, ethanol, isopropanol, diethyl ether, tetrahydrofuran, ethyl acetate, dichloromethane, acetonitrile, 1,4-dioxane, N,N-dimethylformamide, N,N-dimethylacetamide.

The reduction reaction is conducted in the presence of hydrogen gas. The reduction reaction may be conducted under normal pressure or elevated pressure. The pressure of the hydrogen gas is preferably from 0.5 atm to 10 atm. The reaction temperature is preferably from 0°C to 100°C, more preferably from 10°C to 50°C. The reaction time is preferably from 1 to 48 hours, more preferably from 6 to 36 hours.

[Step 8-1]

By removing the protecting group $R^2$ for the compound 5-1 by deprotection, compound 6-3 can be obtained. Deprotection may be conducted in the same manner as in step 5-1.

[Step 9-1]

By removing the protecting group $R^1$ for the compound 6-3 by deprotection, compound 7-1 can be obtained. Deprotection may be conducted in the same manner as in step 6-1.

[Step 8-2]

By removing the protecting group $R^1$ for the compound 5-1 by deprotection, compound 6-4 can be obtained. Deprotection may be conducted in the same manner as in step 6-1.

[Step 9-2]

By removing the protecting group $R^2$ for the compound 6-4 by deprotection, the compound 7-1 can be obtained. Deprotection may be conducted in the same manner as in step 5-1.

Synthesis of the optically active fluorinated amino acid (fluoroalkyl group-containing compound) may also be conducted by the following reaction. In the following reaction formula, the asterisk means that the asymmetric carbon atom marked with the asterisk has an absolute configuration of S or R. Further, Rf, $R^1$ and $R^2$ are as defined above.

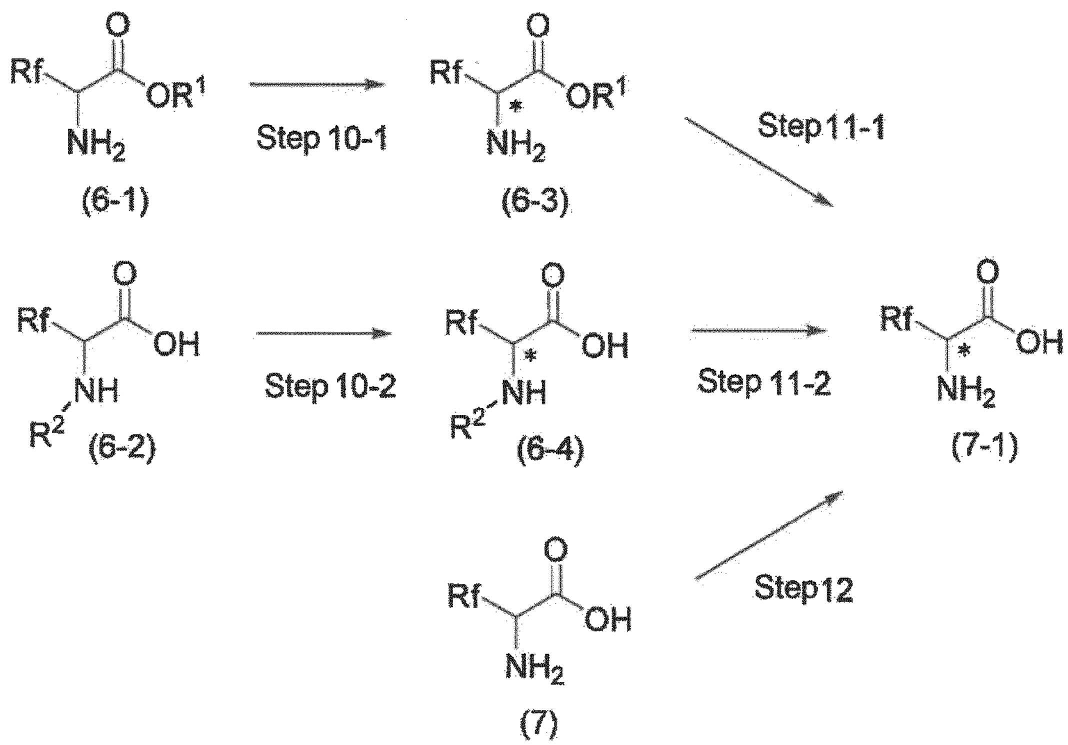

[Step 10-1]

By subjecting the compound 6-1 to optical resolution, the compound 6-3 can be obtained.
The optical resolution may be conducted by a known means. For example, a method of using a chiral column, a method by crystallization or a diastereomer method may, for example, be used.

(1) Method using chiral column

By liquid chromatography or supercritical fluid chromatography (SFC) using a chiral column, a racemic mixture may be resolved into optically active substances. As the chiral column, CHIRALPAK (registered trademark) (Daicel Corporation) and CHIRALCEL (registered trademark) (Daicel Corporation) may, for example, be used.

(2) Method by crystallization

A salt of a racemic mixture and an optically active amine or an optically active acid is formed and is induced to a crystalline diastereomer salt, followed by fractional crystallization. Recrystallization is repeatedly carried out, whereby a single diastereomer salt can be obtained. As the case requires, the diastereomer salt is neutralized to obtain a free optically active substance. The optically active amine may, for example, be brucine, cinchonidine, cinchonine or 1-phenethylamine. The optically active acid may, for example, be camphorsulfonic acid, tartaric acid or mandelic acid.

(3) Diastereomer method

An optically active reagent is reacted with the racemic mixture to obtain a diastereomer mixture, which is subjected to fractional crystallization and chromatography to isolate a single diastereomer. From the obtained single diastereomer, the optically active reagent is removed to obtain the desired optical isomer.

[Step 11-1]

By removing the protecting group $R^1$ for the compound 6-3 by deprotection, the compound 7-1 can be obtained. Deprotection may be conducted in the same manner as in step 6-1.

[Step 10-2]

By subjecting the compound 6-2 to optical resolution, the compound 6-4 can be obtained. Optical resolution may be conducted in the same manner as in step 10-1.

[Step 11-2]

By removing the protecting group $R^2$ for the compound 6-4 by deprotection, the compound 7-1 can be obtained. Deprotection may be conducted in the same manner as in step 5-1.

[Step 12]

By subjecting the compound 7 to optical resolution, the compound 7-1 can be obtained. Optical resolution may be conducted in the same manner as in step 10-1.

<Method for producing fluoroalkyl group-containing peptide>

The fluoroalkyl group-containing peptide may be produced from an amino acid having a fluoroalkyl group introduced into its side chain, as the raw material. For example, using the compound 6-1, the compound 6-2, the compound 6-3 or the compound 6-4 as the raw material, the fluoroalkyl group-containing peptide can be produced.
For example, the compound 6-2 or 6-4 is condensed with a fluorinated amino acid having its carboxy group protected, an amino acid having its carboxy group protected, a fluorinated peptide having its C-terminal protected, or a peptide having its C-terminal protected, whereby the fluoroalkyl group-containing peptide can be produced. Further, the compound 6-1 or the compound 6-3 is condensed with a fluorinated amino acid having its amino group protected, an amino acid having its amino group protected, a fluorinated peptide having its N-terminal protected, or a peptide having its N-terminal protected, whereby the fluoroalkyl group-containing peptide can be produced.
Further, the compound 7 or the compound 7-1, after having its amino group or carboxy group protected, may

be subjected to condensation in the same manner to produce the fluoroalkyl group-containing peptide. Specifically, after the amino group is protected with a protecting group, the compound is condensed with a fluorinated amino acid having its carboxy group protected, an amino acid having its carboxy group protected, a fluorinated peptide having its C-terminal protected, or a peptide having its C-terminal protected. Or, after the carboxy group is protected with a protecting group, the compound is condensed with a fluorinated amino acid having its amino group protected, an amino acid having its amino group protected, a fluorinated peptide having its N-terminal protected, or a peptide having its N-terminal protected.

Production of the peptide may be conducted by a conventional peptide synthesis method. For example, it may be conducted by solid phase peptide synthesis method. The fluoroalkyl group-containing peptide can easily be synthetized by using a peptide automatic synthetizing machine using an amino acid having a fluoroalkyl group introduced into its side chain as the raw material.

A peptide can be produced in such a manner that an amino acid having an amino group protected is sequentially condensed to an amino acid having its C-terminal bonded to a solid phase, and the resulting peptide is removed from the solid phase. The amino acid material is preferably one having its amino group protected with a Boc group or a Fmoc group. The amino acid material is preferably one having its side chain functional group protected with a protecting group. As the protecting group for the side chain functional group, a Boc group, a triphenylmethyl group, a benzyl group, and a 2,2,5,7,8-pentamethylchroman-6-sulfonyl (Pmc) group may, for example, be mentioned.

As a condensing agent for forming a peptide bond, for example, N,N-dicyclohexylcarbodiimide (DCC), 1-ethyl-3-(3'-dimethylaminopropyl)carbodiimide (WSC), benzotriazol-1-yloxy-trisdimethylaminophosphonium hexafluorophosphate (BOP), benzotriazol-1-yloxytrispyrrolidinophosphonium hexafluorophosphate (pyBOP), 2-(1H-benzotriazol-1-yl)-1,1,3,3-tetramethyluronium hexafluorophosphate (HBTU) or 2-(1H-benzotriazol-1-yl)-1,1,3,3-tetramethyluronium tetrafluoroborate may be mentioned. Further, N-hydroxybenzotriazole (HOBt) and the above condensing agent may be mixed at a preferred ratio.

For formation of the peptide bond, a method of activating the carboxy terminal may be employed, and in such a case, the activating agent may, for example, be N-hydroxysuccinimide, p-nitrophenylester or pentafluorophenylester. The base to be used when the peptide bond is formed, may, for example, be triethylamine or diisopropylethylamine (DIPEA). The solvent to be used for the peptide bond forming reaction may, for example, be chloroform, dichloromethane, acetonitrile, N,N-dimethylformamide (DMF) or dimethyl sulfoxide.

The Boc group and the Fmoc group, which are a protecting group for an amino-terminal amino group of the peptide or the amino acid, are respectively removed by trifluoroacetic acid and piperidine. The protecting group for the side chain functional group of the amino acid residue of the peptide may be removed, for example, by trifluoroacetic acid, hydrogen fluoride (HF) or trifluoromethanesulfonic acid.

Further, in the solid phase peptide synthesis method, as a method of removing a peptide or a peptide having a protecting group on the side chain functional group of the amino acid residue, from the solid phase peptide synthesis resin, for example, TFA may be used. Removal of the peptide from the solid phase peptide resin and removal of the protecting group on the side chain functional group of the amino acid residue, may be carried out simultaneously in the same reaction system. Otherwise, they may be carried out independently. As the solid phase peptide synthesis resin for the solid phase peptide synthesis, for example, commercial products such as a 4-hydroxymethyl-3-methoxyphenoxy butyric acid/benzhydrylamine/polystyrene resin, a p-benzyloxy benzyl alcohol/polystyrene resin, and an oxime resin may be used.

The desired peptide or its intermediate may be isolated and purified by various methods, such as ion chromatography, gel permeation chromatography, reversed phase chromatography, normal phase chromatography, recrystallization, extraction and fractional crystallization. Further, the peptide thus obtained may be converted into the corresponding salt by a conventional method.

The protecting group for an amino group or a carboxy group of the produced fluoroalkyl group-containing peptide may be removed by deprotection as the case requires. Deprotection may be conducted by a conventional method depending upon the type of the protecting group.

<Fluoroalkyl group-containing peptide>

The fluoroalkyl group-containing peptide according to the present invention is a peptide comprising two or more types of amino acids, and at least one of the amino acid residues constituting the peptide has, as its side chain, a $C_{1-30}$ alkyl group substituted with at least two fluorine atoms. The $C_{1-30}$ alkyl group substituted with at least two fluorine atoms may further be replaced with a halogen atom other than a fluorine atom. Further, when the $C_{1-30}$ alkyl group is a $C_{2-30}$ alkyl group, it may have 1 to 5 etheric oxygen atoms between carbon atoms.

As the fluoroalkyl group-containing peptide according to the present invention, a peptide comprising an amino acid residue having the above Rf as its side chain may be mentioned. At least one of the amino acid residues constituting the peptide, has Rf as its side chain, and all the amino acid residues may have Rf as their side chains. When one molecule of the peptide has two or more amino acid residues having Rf as their side chains, the plurality of Rf may be

of the same type or different type. Further, the amino acid residue having Rf as its side chain in the peptide may be on the N-terminal, may be on the C-terminal, or at a moiety other than the terminal.

Rf is preferably a group represented by the following formula (f-1) or (f-2). $Rf^P$ means a perhalogenated $C_{1-10}$ alkyl group containing at least two fluorine atoms. $Rf^P$ is a group in which all of hydrogen atoms in a $C_{1-10}$ alkyl group are replaced with a halogen atom, and at least two of such halogen atoms are fluorine atoms. When $Rf^P$ has 2 or more carbon atoms, that is, it is a perhalogenated $C_{2-10}$ alkyl group, it may have from 1 to 5 etheric oxygen atoms between carbon atoms. In the formula (f-2), the two $Rf^P$ may be groups of the same type or different type.

In the following formula (f-1) or (f-2), n1 is an integer of from 0 to 10, and n2 is an integer of from 0 to 9. When each of n1 and n2 is 0, such is meant for a single bond. That is, when n1 is 0, the group represented by the formula (f-1) is $Rf^P$-, and when n2 is 0, the group represented by the formula (f-2) is $(Rf^P)_2$-CH-.

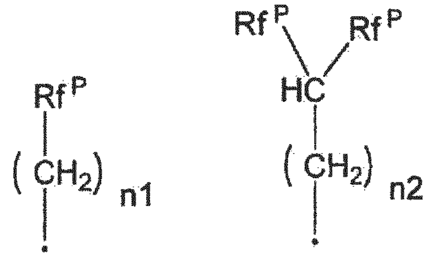

(f-1) (f-2)

When Rf is a group represented by the formula (f-1), Rf is preferably a group wherein $Rf^P$ is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group or a perfluorodecyl group, and n1 is an integer of from 0 to 4, more preferably a group wherein $Rf^P$ is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group or a perfluorodecyl group, and n1 is an integer of from 0 to 2, further preferably a group wherein $Rf^P$ is a trifluoromethyl group, a pentafluoroethyl group, a heptafluoropropyl group, a nonafluorobutyl group, a perfluoropentyl group or a perfluorohexyl group, and n1 is an integer of from 0 to 2 (excluding a group wherein n1 is 1, and $Rf^P$ is a trifluoromethyl group).

When Rf is a group represented by the formula (f-2), Rf is preferably a group wherein $Rf^P$ is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group or a perfluorodecyl group, and n2 is an integer of from 0 to 4, more preferably a group wherein $Rf^P$ is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group or a perfluorodecyl group, and n2 is an integer of from 0 to 2, further preferably a group wherein $Rf^P$ is a trifluoromethyl group, a pentafluoroethyl group, a heptafluoropropyl group, a nonafluorobutyl group, a perfluoropentyl group or a perfluorohexyl group, and n2 is an integer of from 0 to 2 (excluding a group wherein n2 is 0 or 1, and $Rf^P$ is a trifluoromethyl group).

As examples of Rf, a difluoromethyl group, a 1,1-difluoroethyl group, a 2,2-difluoroethyl group, a 1,1,2,2-tetrafluoroethyl group, a 1,1,2,2,3,3-hexafluoropropyl group and a 1,1,2,3,3,3-hexafluoropropyl group may be mentioned.

The peptide comprising 2 or more amino acids, is preferably a peptide comprising 3 or more amino acids. It is preferably a peptide comprising from 2 to 40 amino acids, more preferably a peptide comprising from 3 to 20 amino acids.

Of the fluoroalkyl group-containing peptide of the present invention, the C-terminal may be protected with a protecting group represented by $R^1$. $R^1$ is preferably a benzyl group. Further, of the fluoroalkyl group-containing peptide of the present invention, the N-terminal may be protected with a protecting group for an amino group, represented by $R^2$. $R^2$ is preferably a Boc group or a Fmoc group.

The fluoroalkyl group-containing peptide of the present invention may, for example, be a tripeptide represented by the following formula (101) or (102). In the formulae (101) and (102), $R^{11}$ and $R^{12}$ are each independently a $C_{1-6}$ alkyl group or a benzyl group, and are preferably each independently a methyl group or a benzyl group, and it is particularly preferred that $R^{11}$ is a methyl group and $R^{12}$ is a benzyl group. X is Fmoc or Boc. Z is a $C_{1-6}$ alkoxy group, and is particularly preferably a methoxy group.

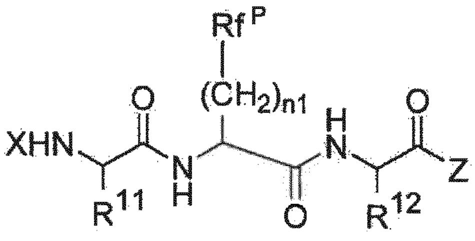

(101)

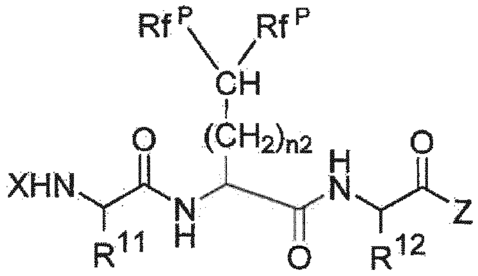

(102)

In the formula (101) and (102), $Rf^P$, n1 and n2 are as defined for the formulae (f-1) and (f-2). The group represented by the formula (101) or (102) is preferably a group wherein $Rf^P$ is a perfluorinated $C_{1-10}$ alkyl group, and n1 or n2 is an integer of from 0 to 4, more preferably a group wherein $Rf^P$ is a trifluoromethyl group, a pentafluoroethyl group, a heptafluoropropyl group, a nonafluorobutyl group, a perfluoropentyl group, a perfluorohexyl group, a perfluoroheptyl group or a perfluorooctyl group, and n1 or n2 is an integer of from 0 to 2, further preferably a group wherein $Rf^P$ is a nonafluorobutyl group, a perfluoropentyl group, a perfluorohexyl group, a perfluoroheptyl group or a perfluorooctyl group, and n1 or n2 is an integer of from 0 to 2.

A fluoroalkyl group has high affinity to the cell membrane. Accordingly, the fluoroalkyl group-containing peptide of the present invention is excellent in cell permeability. Further, since it has a structure significantly different from that of native peptides, it is hardly decomposed by peptidases. By virtue of these properties, the fluoroalkyl group-containing peptide of the present invention is expected to be useful in medical fields as a physiologically active substance. For example, the fluoroalkyl group-containing peptide of the present invention is expected to be useful as a DDS carrier which transports therapeutic components to target cells. For example, by adding, to a functional peptide which exhibits a certain physiological activity when taken by the target cells in the body, the fluoroalkyl group-containing peptide of the present invention so as not to impart the function, the uptake efficiency of the functional peptide by the target cells can be improved. Further, by replacing the side chain of a part of hydrophobic amino acid residues of the functional peptide having physiological activity, with Rf, preferably a group represented by the formula (101) or (102), within a range not to impart the function of the functional peptide, cell permeability and the retention time in the cells of the functional peptide can be improved.

EXAMPLES

Now, the present invention will be described in further detail with reference to Examples. However, it should be understood that the present invention is by no means restricted to such specific Examples.

The NMR apparatus used for analysis in Examples and Comparative Examples is JNM-ECZ400S (400 MHz) manufactured by JEOL Ltd. For $^1H$ NMR, the chemical shift of tetramethylsilane was assigned as 0 PPM, and for $^{19}F$ NMR, the chemical shift of $C_6F_6$ was assigned as -162 PPM.

In this specification, the following abbreviations are used.

Bn: benzyl
Boc: t-butoxycarbonyl
All: allyl
$Et_2O$: diethyl ether
Fmoc: 9-fluorenylmethyloxycarbonyl
THF: tetrahydrofuran
TMS: trimethylsilyl
$C_4F_9$: 1,1,2,2,3,3,4,4,4-nonafluorobutyl
$C_8F_{17}$: 1,1,2,2,3,3,4,4,5,5,6,6,7,7,8,8,8-heptadecafluorooctyl

[Production Example 1]

From trimethyl(nonafluorobutyl)silane and dibenzyl oxalate, 2-((t-butoxycarbonyl)amino)-3,3,4,4,5,5,6,6,6-nonafluorohexanoic acid was synthetized.

[Step 1]

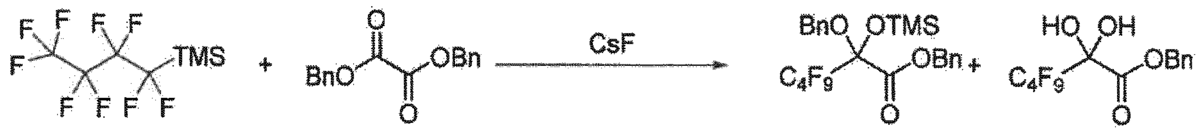


Into a 100 mL volume two-necked flask dried in an oven, a stirrer was put, and in a nitrogen atmosphere, dibenzyl oxalate (5.41 g, 20.0 mmol), cesium fluoride (255 mg, 1.68 mmol) and THF (54 mL) were added and stirred, and after cooling to -30°C, trimethyl(nonafluorobutyl)silane (4.50 mL, 20.2 mmol) was added, and stirring was continued at -30°C for 24 hours. To the reaction liquid, a saturated aqueous ammonium chloride solution (30 mL) was added, followed by extraction with ethyl acetate (3x50 mL). The resulting organic phase put together was dried over sodium sulfate and subjected to filtration, and the filtrate was distilled under reduced pressure to obtain a crude mixture of benzyl 2-(benzyloxy)-3,3,4,4,5,5,6,6,6-nonafluoro-2-((trimethylsilyl)oxy)hexanoate and benzyl 3,3,4,4,5,5,6,6,6-nonafluoro-2,2-dihydroxyhexanoate. The obtained crude product was subjected to the next step without being purified.

[Step 1-2]

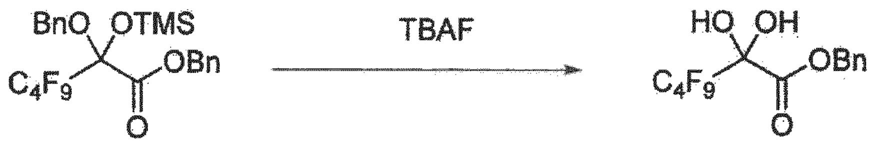


Into a 100 mL volume two-necked flask dried in an oven, a stirrer was put, and in a nitrogen atmosphere, the entire crude product obtained in step 1, a tetrabutylammonium fluoride (TBAF) 1 mol/L THF solution (10.5 mL, 10.5 mmol), acetic acid (1 mL) and THF (50 mL) were added and stirred at 0°C. Then, the temperature was raised to room temperature, followed by stirring for 24 hours, and a saturated aqueous sodium hydrogen carbonate solution (30 mL) was added for quenching, followed by extraction with ethyl acetate (3x50 mL). The resulting organic phase put together was washed with water (50 mL) and a saturated saline solution (50 mL), dried over sodium sulfate and subjected to filtration, and the filtrate was distilled under reduced pressure to obtain crude benzyl 3,3,4,4,5,5,6,6,6-nonafluoro-2,2-dihydroxyhexanoate. The obtained crude product was subjected to the next step without being purified.

$^{1}H$ NMR (400 MHz, CDCls) $\delta$7.39 (brs, 5H), 5.37 (s, 2H).

$^{19}F$ NMR (376 MHz, $CDCl_3$) $\delta$-80.79 (brs, 3F), -121.09 (brs, 2F), -121.24-121.26 (m, 2F), -126.12-126.21 (m, 2F).

[Step 2]

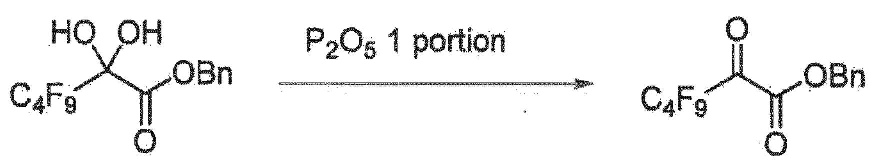


Into a 20 mL volume flask dried in an oven, a stirrer was put, and in a nitrogen atmosphere, the entire crude product obtained in step 2 and phosphorus pentoxide (22 wt% of the crude product) were added, followed by distillation under reduced pressure. The fractions obtained under 2 mmHg at 77°C were collected to obtain benzyl 3,3,4,4,5,5,6,6,6-nonafluoro-2-oxohexanoate as a colorless liquid (yield from step 1 through step 3: 73%).

$^{1}H$ NMR (400 MHz, $CDCl_3$) $\delta$7.41 (brs, 5H), 5.40 (s, 2H).

$^{19}F$ NMR (376 MHz, $CDCl_3$) $\delta$-80.79 (brs, 3F), -117.78-117.850 (t, 2F, $J_{F-F}$=13 Hz), -122.01 (brs, 2F), -125.58 (brs, 2F).

In the same manner as in step 1 to step 2 except that the temperature in step 1 was changed to 0°C, benzyl

3,3,4,4,5,5,6,6,6-nonafluoro-2-oxohexanoate was obtained as a colorless liquid. The yield from step 1 through step 2 was 69%.

[Step 3]

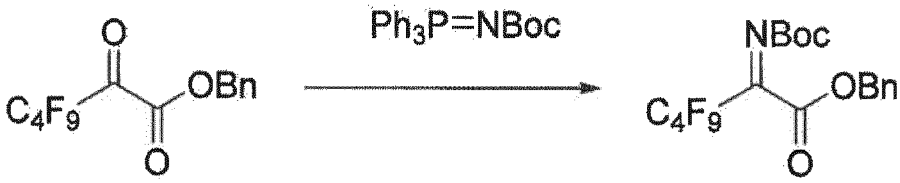


Into a 30 mL volume Schlenk flask dried in an oven, a stirrer was put, and in a nitrogen atmosphere, benzyl 3,3,4,4,5,5,6,6,6-nonafluoro-2-oxohexanoate (1 g, 2.6 mmol), t-butyl(triphenylphosphanylidene)carbamate (2.6 mmol) and $Et_2O$ (10 mL) were added. The reaction liquid was stirred at room temperature for 1 hour and subjected to filtration, and the solid residue was washed with $Et_2O$ (2x2 mL). The resulting organic phase put together was distilled under reduced pressure to obtain crude benzyl 2-((t-butoxycarbonyl)imino)-3,3,4,4,5,5,6,6,6-nonafluorohexanoate. The obtained crude product was purified by silica gel chromatography ($Et_2O$/hexane=1/4) to obtain 2-((t-butoxycarbonyl)imino)-3,3,4,4,5,5,6,6,6-nonafluorohexanoate as a colorless liquid (yield: 87%).

$^1H$ NMR (400 MHz, $CDCl_3$) $\delta$7.410-7.352 (m, 5H), 5.350 (s, 2H), 1.504 (s, 9H).

$^{19}F$ NMR (376 MHz, $CDCl_3$) $\delta$-80.76-80.78 (t, 3F, $J_{F-F}$=9 Hz), -112.37 (brs, 2F), -121.0 (brs, 2F), -125.36 (brs, 2F).

[Step 4]

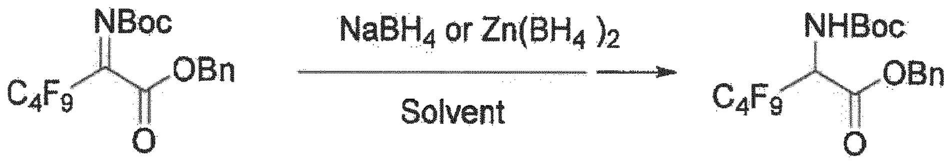


Into a 30 mL volume Schlenk flask dried in an oven, a stirrer was put, and in a nitrogen atmosphere, benzyl 2-((t-butoxycarbonyl)imino)-3,3,4,4,5,5,6,6,6-nonafluorohexanoate (0.2 g, 0.42 mmol) was dissolved in $Et_2O$ (15 mL), followed by stirring at 0°C. Sodium borohydride (0.46 mmol) was added dividedly in three times at 0°C, and the temperature was raised to room temperature, followed by stirring for 24 hours. Ice water was added for quenching, and 1 mol/L hydrochloric acid was added to adjust the pH to be less than 7. The aqueous phase was extracted with $Et_2O$ (2×10 mL), and the resulting organic phase put together was distilled under reduced pressure to obtain crude benzyl 2-((t-butoxycarbonyl)amino)-3,3,4,4,5,5,6,6,6-nonafluorohexanoate. The obtained crude product was purified by silica gel chromatography (ethyl acetate/hexane=1/4) to obtain benzyl 2-((t-butoxycarbonyl)amino)-3,3,4,4,5,5,6,6,6-nonafluorohexanoate as a colorless liquid (yield: 61%).

$^1H$ NMR (400 MHz, $CDCl_3$) $\delta$7.40-7.33 (m, 5H), 5.41-5.39 (d, 2H, $J_{H-H}$=10 Hz), 5.28-5.20 (m, 3H), 1.45 (s, 9H).

$^{19}F$ NMR (376 MHz, $CDCl_3$) $\delta$-80.86-80.89 (t, 3F, $J_{F-F}$=9 Hz), -115.36-118.55 (m, 2F), -121.50-123.17 (m, 2F), -125.00-126.77 (m, 2F).

The same reaction as in step 4 was carried out except that the solvent and reducing agent (equivalent) as identified in Table 1 were used instead of $Et_2O$ and sodium borohydride. The yield is shown in Table 1. In Table, "AK225" is "ASAHIKLIN (registered trademark) AK-225" (a mixture of 3,3-dichloro-1,1,1,2,2-pentafluoropropane and 1,3-dichloro-1,1,2,2,3-pentafluoropropane, AGC Inc.).

[Table 1]

| Solvent | Reducing agent (equivalent) | Yield (%) |
|---|---|---|
| $Et_2O$ | $NaBH_4$ (1.1) | 61 |
| THF | $Zn(BH_4)_2$ (1.0) | 37 |

(continued)

| Solvent | Reducing agent (equivalent) | Yield (%) |
|---|---|---|
| AK225 | $NaBH_4$ (1.1) | 35 |
| THF | $NaBH_4$ (1.1) | 19 |

[Step 5-2]

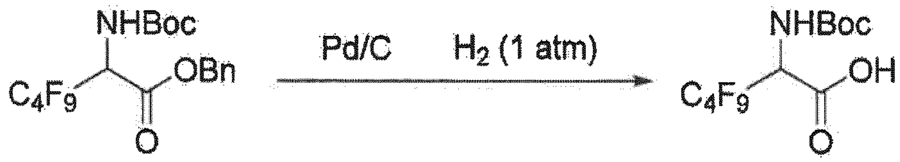


Into a 25 mL volume two-necked flask dried in an oven, a stirrer was put, and benzyl 2-((t-butoxycarbonyl)amino)-3,3,4,4,5,5,6,6,6-nonafluorohexanoate (73.6 mg, 0.15 mmol), palladium/carbon (Pd 5%, wetted with about 55% water, 20 mg), ethyl acetate (1 mL) and ethanol (7 mL) were added, followed by stirring at room temperature in a hydrogen atmosphere under normal pressure. After stirring at room temperature for 24 hours, the mixture was subjected to filtration through celite, the solid residue was washed with ethanol (3×5 mL), and the resulting organic phase put together was distilled under reduced pressure to obtain crude 2-((t-butoxycarbonyl)amino)-3,3,4,4,5,5,6,6,6-nonafluorohexanoic acid. The obtained crude product was purified by silica gel chromatography (ethyl acetate/hexane=1/1) to obtain 2-((t-butoxycarbonyl)amino)-3,3,4,4,5,5,6,6,6-nonafluorohexanoic acid as a colorless liquid (yield: 86%).

$^1$H NMR (400 MHz, $CDCl_3$) δ7.72 (br, 1H), 5.49-5.47 (d, 2H, $J_{H\text{-}H}$=10 Hz), 5.24-5.15 (m, 1H), 1.45 (s, 9H).

$^{19}$F NMR (376 MHz, $CDCl_3$) δ-80.30 (brs, 3F), -114.64-118.03 (m, 2F), -120.70-122.40 (m, 2F), -124.52-126.22 (m, 2F).

[Step 5-1]

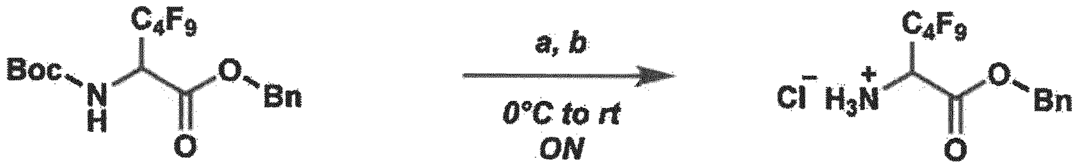


a: 4M HCl in 1,4 dioxane, 3 mL

*b*: quenched by $NaHCO_3$ aq, extracted with EA, dried with $Na_2SO_4$, evap, dried in vacuo

Into a 25 mL volume two-necked flask dried in an oven, a stirrer was put, and Boc-RFAA-OBn (376 mg, 0.78 mmol), and 4M HCl in 1,4-dioxane solution (3 mL) were added at 0°C. After stirring at room temperature for 18 hours, a saturated aqueous sodium carbonate solution was added to adjust the pH to be higher than 7, followed by extraction with ethyl acetate. The resulting organic phase was washed with a saturated saline solution and dried over sodium sulfate. The organic phase was subjected to filtration, and the filtrate was distilled under reduced pressure to obtain H-RFAA-OBn hydrochloride as a white solid (yield: 78%).

$^1$H NMR (400 MHz, $D_2O$) δ7.31 (brs, 2H), 6.81-6.77 (m, 5H), 5.27 (m, 1H), 3.71-3.67 (m, 2H).

$^{19}$F NMR (376 MHz, $D_2O$) δ-80.38 (t, 3F), -118.29-121.00 (m, 2F), -121.00-123.80 (m, 2F), -126.12-128.12 (m, 2F).

In the following, the amino acids may sometimes be represented by three-letter symbols. For example, "Phe" represents phenylalanine, and "Gly" represents glycine. Further, peptides are represented as (N side protecting group)-amino acid by three-letter symbol-(C side protecting group). "H-AA-OMe" means that the N-terminal side is not protected, and the C-terminal side is a methyl ester. When the C-terminal side is not protected, the C-terminal side is

represented as "OH" instead of "OMe".

[Example 1]

A dipeptide having a nonafluorobutyl group was synthesized.

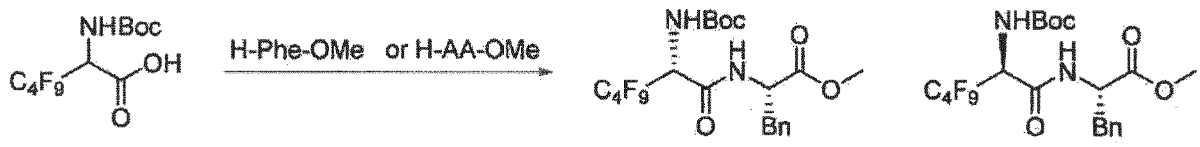


Boc-RfAA-Phe-OMe

Into a 25 mL volume two-necked flask dried in an oven, a stirrer was put, 2-((t-butoxycarbonyl)amino)-3,3,4,4,5,5,6,6,6-nonafluorohexanoic acid (33.4 mg, 0.085 mmol), DIPEA (0.13 mmol), DCM (3 mL), L-phenylalanine methyl ester (0.13 mmol) and benzotriazol-1-ol monohydrate (0.085 mmol) were added at room temperature, followed by cooling to 0°C, and BOP (0.085 mmol) was added. After stirring at room temperature for 24 hours, the solvent was distilled off under reduced pressure, the residue was diluted with ethyl acetate, and the resulting organic phase was washed with a saturated aqueous citric acid solution, a saturated aqueous sodium carbonate solution and a saturated saline solution and dried over sodium sulfate. The organic phase was subjected to filtration, and the filtrate was distilled under reduced pressure to obtain crude Boc-RFAA-Phe-OMe. The obtained crude product was purified by silica gel chromatography (ethyl acetate/hexane=1/4) to obtain two types of Boc-RFAA-Phe-OMe diastereomers respectively as colorless liquids (yield of the two types of diastereomers together: 22%).

Diastereomer A

$^1$H NMR (400 MHz, $CDCl_3$) $\delta$7.31-7.25 (m, 5H), 6.41-6.40 (d, N-H, $J_{H-H}$=7 Hz), 5.48-5.46 (d, 1H, $J_{H-H}$=9 Hz), 4.99-4.91 (m, 1H), 4.91-4.86 (m, 1H), 3.75 (s, 3H), 3.23-3.10 (m, 2H), 1.47 (s, 9H).

$^{19}$F NMR (376 MHz, $CDCl_3$) $\delta$-80.98 (t, 3F, $J_{F-F}$=7 Hz), -114.56-119.80 (m, 2F), -121.40-123.22 (m, 2F), -125.03-127.15 (m, 2F).

Diastereomer B

1H NMR (400 MHz, $CDCl_3$) $\delta$7.30-7.07 (m, 5H), 6.42-6.40 (d, N-H, $J_{H-H}$=9 Hz), 5.51-5.48 (d, 1H, $J_{H-H}$=8 Hz), 5.00-4.95 (m, 1H), 4.93-4.88 (m, 1H), 3.74 (s, 3H), 3.15-3.13 (m, 2H), 1.44 (s, 9H).

$^{19}$F NMR (376 MHz, $CDCl_3$) $\delta$-80.77 (t, 3F, $J_{F-F}$=9 Hz), -113.74-119.30 (m, 2F), -121.22-122.94 (m, 2F), -124.82-127.05 (m, 2F).

The same reaction was carried out by using the amino acid methyl ester as identified in Table 2 instead of L-phenylalanine methyl ester. The yield is shown in Table 2. In Table, "DCM" represents dichloromethane, "BOP" benzotriazol-1-yloxy-trisdimethylaminophosphonium hexafluorophosphate, and "EDC" 1-(3-dimethylaminopropyl)-3-ethylcarbodiimide hydrochloride.

[Table 2]

| H-AA-OMe | Solvent | Condensing agent | Yield (%) |
|---|---|---|---|
| Phe | DCM | BOP | 22 |
| Gly | DCM | EDC | 35 |

[Example 2]

The protecting group on the N-terminal side of the peptide synthesized in Example 1 was removed by deprotection.

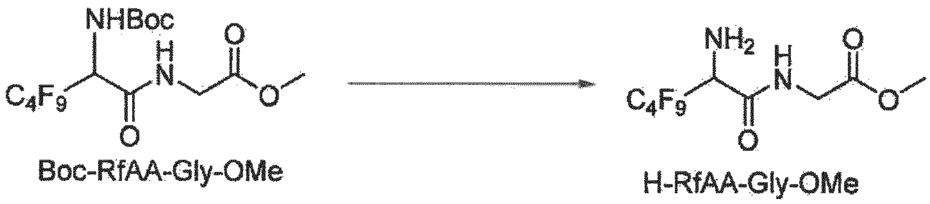


Into a 25 mL volume two-necked flask dried in an oven, a stirrer was put, and Boc-RFAA-Gly-OMe (21.2 mg, 0.05 mmol), DCM (1.5 mL) and TFA (0.4 mL) were added. After stirring at room temperature for 24 hours, a saturated aqueous sodium carbonate solution was added to adjust the pH to be higher than 7, followed by extraction with DCM. The resulting organic phase was washed with a saturated saline solution and dried over sodium sulfate. The organic phase was subjected to filtration, and the filtrate was distilled under reduced pressure to obtain crude H-RFAA-Gly-OMe (yield: 66%).

$^1$H NMR (400 MHz, $CDCl_3$) δ7.08 (brs, 1H), 4.15-4.07 (m, 2H), 3.79 (s, 3H).

$^{19}$F NMR (376 MHz, $CDCl_3$) δ-80.71 (t, 3F, $J_{F-F}$=7 Hz), -115.13-119.94 (m, 2F), -119.94-121.93 (m, 2F), -125.02-126.82 (m, 2F).

[Example 3]

A tripeptide having a nonafluorobutyl group was synthesized.

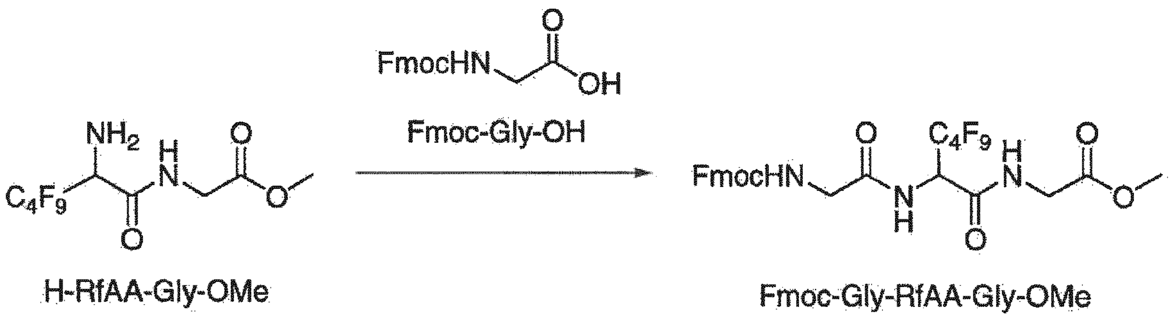


Into a NMR test tube dried in an oven, H-RFAA-Gly-OMe (10.9 mg, 0.03 mmol), DCM (0.5 mL), DIPEA (0.13 mmol), Fmoc-Gly-OH (0.03 mmol) and benzotriazol-1-yloxy-trisdimethylaminophosphonium salt (0.085 mmol) were added at room temperature. The mixture was left at rest at room temperature for 24 hours, the solvent was distilled off under reduced pressure, the residue was diluted with ethyl acetate, and the resulting organic phase was washed with a saturated aqueous citric acid solution, a saturated aqueous sodium carbonate solution and a saturated saline solution and dried over sodium sulfate. The organic phase was subjected to filtration, and the filtrate was distilled under reduced pressure to obtain crude Fmoc-Gly-RFAA-Gly-OMe.

$^1$H NMR (400 MHz, $CDCl_3$) δ7.78-7.27 (m, 8H), δ7.30 (brs, 1H), 7.13 (brs, 1H), 5.63-5.61 (d, 1H, $J_{H-H}$=7 Hz), 5.64-5.54 (m, 1H), 4.42-4.41 (d, 2H, $J_{H-H}$=7 Hz), 4.24-4.20 (m, 1H), 4.08-3.98 (m, 4H), 3.74 (s, 3H).

$^{19}$F NMR (376 MHz, $CDCl_3$) δ-80.78 (t, 3F, $J_{F-F}$=10 Hz), -114.61-118.74 (m, 2F), -121.22-123.09 (m, 2F), -124.89-126.90 (m, 2F).

[Example 4]

A tripeptide having a nonafluorobutyl group (Boc-Ala-RFAA-Phe-OMe) was synthesized.

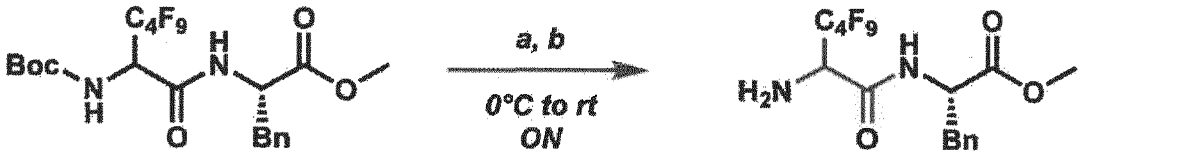


*a*: 20% TFA/DCM
*b*: quenched by $NaHCO_3$ aq, extracted with DCM, dried with $Na_2SO_4$, evap, dried in vacuo
*c*: column chromatography (Hex:EtOAc = 2 :1, $NEt_3$ 1%)

Into a 25 mL volume two-necked flask, a stirrer was put, and from one neck, Boc-RFAA-Phe-OMe diastereomer A (DR>95, 0.11 mmol) and DCM (5 mL) were added. The reaction mixture was cooled to 0°C, TFA (1.25 mL) was added, and the reaction mixture was allowed to room temperature. After stirring for 4 hours, an aqueous sodium hydrogen carbonate solution was added to terminate the reaction. The aqueous phase was extracted with DCM, and the resulting organic phase put together was distilled under reduced pressure and purified by silica gel column chromatography (hexane:ethyl acetate:triethylamine=2:1:1%) to obtain H-RFAA-Phe-OMe (33.9 mg, yield: 70.0%).

$^1H$ NMR (400 MHz, $CDCl_3$) δ=1.77 (brs, 2H), 3.10-3.14 (m, 2H), 3.74 (s, 3H), 3.93-3.99 (dd, 1H), 4.90-4.95 (dd, 1H), 6.81-6.83 (d, NH), 7.27 (m, 5H)

$^{19}F$ NMR (376 MHz, $CDCl_3$) δ=-126.2-125.7 (m, 2F), -121.2-119.7 (m, 2F), -120.5-114.4 (m, 2F), -80.7 (t, 3F)

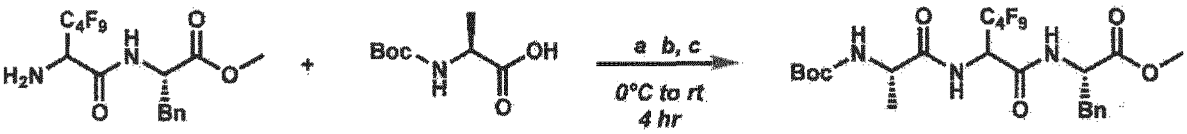


**H-R$_F$AA-Phe-OMe** — **17.6 mg, 0.04 mmol, dr >95**

**alanine** — **1.1 eq**

**Boc-Ala-R$_F$AA-Phe-OMe**
**Yield 98.1%**
**dr 85:15**

*a*: DCM 7 mL, HOAt (1.1 eq), DIPEA (1.3 eq), HATU (1.1 eq)
*b*: quenched by 1N HCl, extracted with DCM, evap, diluted with EtOAc, washed with 1N HCl, NaHCO3 aq, brine, dried with Na2SO4, evap, dried in vacuo
*c*: column chromatography (Hex:EtOAc = 3:1)

Into a 25 mL volume two-necked flask, a stirrer was put, and from one neck, 3 mL of DCM was added, and Boc-Ala-OH (1.1 equivalents), 1-hydroxy-7-azabenzotriazole (HOAt, 1.1 equivalents), DIPEA (1.3 equivalents), 1-[Bis(dimethylamino)methylene]-1H-1,2,3-triazolo[4,5-b]pyridinium 3-oxide hexafluorophosphate (HATU, 1.1 equivalents) and a dipeptide (H-RFAA-Phe-OMe, dr>95:5, 38 μmol) were added, and the mixture was cooled to 0°C. The mixture was allowed to room temperature and stirred for 1.5 hours. Then, HCl (1N) was added to terminate the reaction. The reaction liquid was subjected to liquid separation with HCl (1N) and DCM, and the resulting organic phase put together was distilled under reduced pressure and diluted with ethyl acetate. The organic phase was washed with HCl (1N), a saturated aqueous $NaHCO_3$ solution and brine, dried over $Na_2SO_4$, and evaporated to obtain a white solid. The obtained crude mixture was purified by silica gel column chromatography (hexane:ethyl acetate=2:1) to obtain a tripeptide (dr>95:5,18.2 mg, yield: 75.1%).

$^1H$ NMR (400 MHz, Acetone d6) δ=1.30 (d, 3H), 1.38 (s, 9H), 3.04-3.16 (m, 2H), 3.66 (s, 3H), 4.26-4.29 (m, 1H), 4.68-4.77 (m, 1H), 5.53-5.61 (m, 1H), 6.23 (d, N-H), 7.19-7.29 (m, 5H), 7.82 (d, N-H), 8.30 (d, N-H).

$^{19}F$ NMR (376 MHz, Acetone d6) δ=-126.9-126.3 (m, 2F), -123.1-121.9 (m, 2F), -120.1-115.0 (m, 2F), -81.5 (t, 3F)

[Example 5]

A tripeptide having a nonafluorobutyl group (H-Ala-RFAA-Phe-OMe) was synthetized.

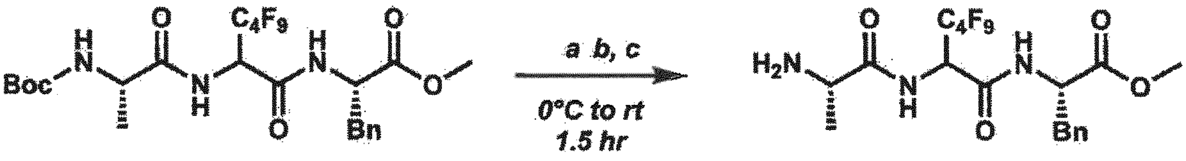


**Boc-Ala-R$_F$AA-Phe-OMe** **H-Ala-R$_F$AA-Phe-OMe**

*a*: DCM 2 mL, TFA 0.4 mL
*b*: quenched by $NaHCO_3$ aq, extracted with DCM, dried with $Na_2SO_4$, evap, dried in vacuo
*c*: column chromatography ($CHCl_3$:MeOH =10 :1)

Into a 25 mL volume two-necked flask, a stirrer was put, and from one neck, Boc-RFAA-Phe-OMe diastereomer A (DR>95, 29 μmol) and DCM (2 mL) were added. The reaction mixture was cooled to 0°C, TFA (0.4 mL) was added, and the mixture was allowed to room temperature. After stirring for 4 hours, an aqueous sodium hydrogen carbonate solution was added to terminate the reaction. The aqueous phase was extracted with DCM, and the resulting organic phase put together was distilled under reduced pressure and purified by silica gel column chromatography ($CHCl_3$:MeOH=10:1) to obtain H-Ala-RFAA-Phe-OMe (dr>9, 13.8 mg, yield: 90.6%).

$^1$H NMR (400 MHz, Acetone d6) δ=1.16 (d, 3H), 2.81 (br s, 2H), 3.00-3.14 (m, 2H), 3.67 (s, 3H), 3.94-3.99 (m, 1H), 4.68-4.74 (m, 1H), 5.49-5.56 (m, 1H), 7.19-7.27 (m, 5H), 8.16 (d, N-H), 8.33 (d, N-H).

$^{19}$F NMR (376 MHz, Acetone d6) δ=-126.9-125.6 (m, 2F), -123.1-122.1 (m, 2F), -120.0-115.3 (m, 2F), -81.7 (t, 3F)

[Example 6]

To the N-terminal of the tripeptide having a nonafluorobutyl group (H-Ala-RFAA-Phe-OMe) synthesized in Example 5, fluorescent material Alexa Fluor 647 was fused.

Into a 1.5 mL volume black tube, Alexa Fluor 647 (250 μg) dissolved in dry DMSO (15 μL), H-Ala-RFAA-Phe-OMe (1.5 equivalents) dissolved in dry DMSO (15 μL) and DIPEA (1.5 equivalents) were added. The mixture was kept being stirred at room temperature overnight. The mixture was purified by reversed-phase chromatography (acetonitrile/water/TFA=30:70:0.1 to 95:5:0.1) and freeze-dried to obtain fluorescent conjugate 1 as a blue solid (yield as calculated by fluorometer: 32.3%). The fluorescence was measured by Nano Drop (registered trademark) spectrophotometer ND-1000 at an emission wavelength=650 nm.

MALDI-TOF MS

$[M]^-$:m/z calcd.for $C_{55}H_{63}F_9N_5O_{17}S_4^-$ 1364.2964, found 1364.7252

[Example 7]

A tripeptide having a heptadecafluorooctyl group (H-Ala-RFAA (C8)-Phe-OMe) was synthesized.

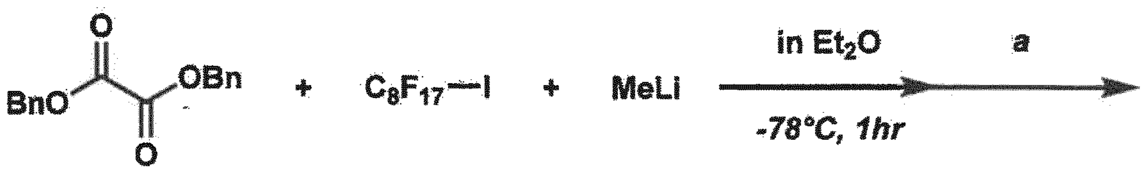


**1.50 g** **1.2 eq** **1.3 eq**
**5.55 mmol**

**a: quenched with 1N HCl,**
**extractd with Et$_2$O, dried over Na$_2$SO$_4$,**
**Evap, droed in vacuo**

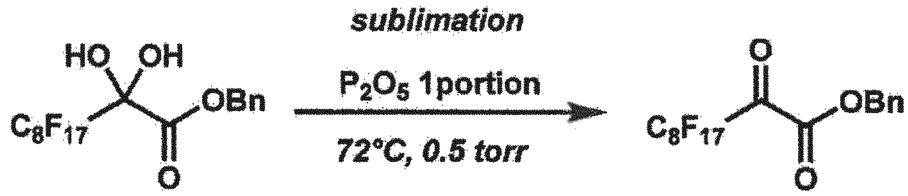


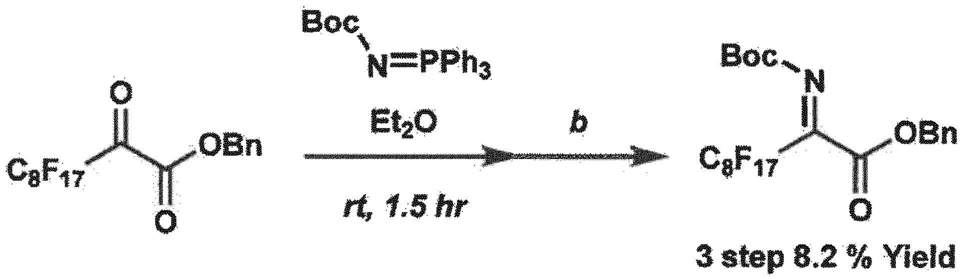


*b*: filtration,
column chromatography (Hex:EtOAc = 10:1 w/ $NEt_3$)

The perfluoroalkylation reaction was conducted in accordance with known literature (Journal of Fluorine Chemistry, 1984, vol. 26, p. 341-358).

The obtained crude product was purified by sublimation (72°C, 0.5 mmHg). The obtained white solid was directly put into a 100 mL volume three-necked round-bottom flask, dissolved in $Et_2O$ (10 mL) and reacted with tert-butyl(triphenylphosphoranylidene)carbamate (5.5 mmol) at room temperature for 1 hour. The crude product was subjected to filtration, and the filtrate was evaporated. The obtained white solid was purified by silica gel column chromatography (hexane:ethyl acetate=10:1w/0.4% $NEt_3$) to obtain $\alpha$-imino ester (311 mg, 3 step yield: 8.2%).

$^1H$ NMR (400 MHz, $CDCl_3$) $\delta$=1.50 (s, 9H), 5.36 (s, 2H), 7.36-7.38 (m, 5H)

$^{19}F$ NMR (376 MHz, $CDCl_3$) $\delta$=-126.3 (m, 2F), -122.8 (m, 2F), -121.8-122.0 (m, 4F), -121.2 (m, 2F), -120.2 (m, 2F), -112.6 (m, 2F), -81.0 (t, 3F)

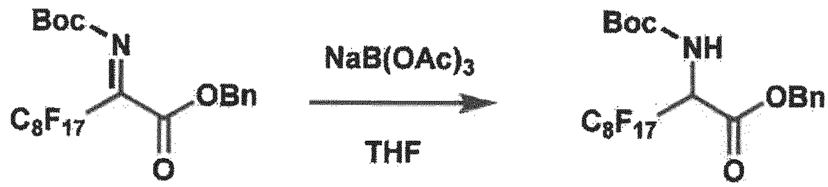


Into a 25 mL volume two-necked round-bottom flask, a stirrer was put, and the $\alpha$-imino ester (311 mg, 0.47 mmol) and THF (5 mL) were added. To the reaction mixture, sodium triacetoxyborohydride (0.59 mmol) was added at 0°C, followed by stirring at room temperature for 24 hours. The reaction mixture was directly evaporated, and partitioned between water and DCM. The resulting organic phase put together was distilled under reduced pressure to obtain a crude mixture. The crude mixture was purified by silica gel column chromatography (ethyl acetate/hexane=1:10) to obtain white solid (Boc-RFAA (C8)-OBn) (yield: 49.6%).

$^1H$ NMR (400 MHz $CDCl_3$) $\delta$=1.28 (s, 9H), 5.07 (s, 2H), 5.137 (m, 1H), 7.18 (m, 5H)

$^{19}F$ NMR (376 MHz $CDCl_3$) $\delta$=-126.3 (m, 2F), -122.9 (m, 2F), -121.5-122.1 (m, 8F), -115.3-118.8 (m, 2F), -81.0 (t, 3F)

The procedure for the tripeptide was the same as above. From 0.23 mmol of Boc-RFAA (C8)-OBn purified by HPLC, Boc-RFAA (C8)-OH was obtained in the same manner as in step 5-2 in Production Example 1, and Boc-RFAA (C8)-Pne-OMe was obtained from Boc-RFAA (C8)-OH in the same manner as in Example 1, and further, 46mg of a tripeptide (H-Ala-RFAA (C8)-Phe-OMe) was obtained from Boc-RFAA (C8)-Pne-OMe in the same manner as in Example 4 (total yield: 27.8%).

ESI-MS
$[M+H]^+$:m/z calcd.for 726.13, found 726.52

[Example 8]

To the N-terminal of the tripeptide having a heptadecafluorooctyl group (H-Ala-RFAA (C8)-Phe-OMe) synthesized in Example 7, fluorescent material Alexa Fluor 647 (manufactured by Thermo Fisher Scientific K.K.) was fused.

Into a 1.5 mL volume black tube, Alexa Fluor 647 (125 μg) dissolved in dry DMSO (15 μL), H-Ala-RFAA (C8)-Phe-OMe (1.5 equivalents) dissolved in dry DMSO (15 μL) and DIPEA (1.5 equivalents) were added. The mixture was kept being stirred at room temperature overnight. The mixture was purified by reversed-phase chromatography (acetonitrile/water/TFA=5:95:0.1 to 10:95:0.1) and freeze-dried to obtain fluorescent conjugate 2 as a blue solid (yield as calculated by fluorometer: 5.7%, emission wavelength=650 nm).

MALDI-TOF MS

$[M]^-$:m/z calcd.for $C_{55}H_{72}N_5O_{17}S_4^-$ 1564.2836, found 1564.5701

[Comparative Example 1]

A dipeptide having a butyl group (H-Nle-Phe-OMe) was synthesized.

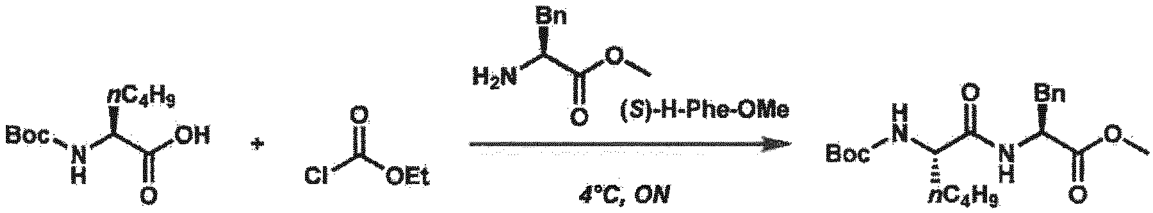


(*S*)-Boc-Nle-OH (*S, S*)-Boc-Nle-Phe-OMe

Boc-Nle-Phe-Ome was synthesized (amount obtained: 556 mg, yield: 40.2%) in accordance with known literature (Chemical and Pharmaceutical Bulletin, 1987, vol. 35, p. 468).

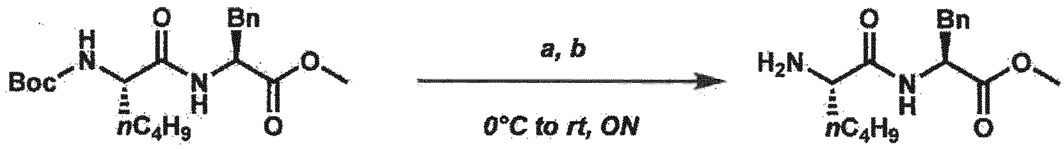


(*S, S*)-Boc-Nle-Phe-OMe (*S, S*)-Boc-Nle-Phe-OMe

*a*: TFA 2 mL, DCM 10 mL
*b*: wasehd with sat $NaHCO_3$ aq, extracted with DCM,
dried over $Na_2SO_4$, evap

Into a 25 mL volume two-necked flask, a stirrer was put, and from one neck, Boc-Nle-Phe-OMe (1.42 mmol) and DCM (10 mL) were added. The reaction mixture was cooled to 0°C, TFA (2 mL) was added, and the mixture was allowed to room temperature. After stirring for 4 hours, an aqueous sodium hydrogen carbonate solution was added to terminate the reaction. The aqueous phase was extracted with DCM, and the resulting organic phase put together was distilled under reduced pressure to obtain H-Nle-Phe-OMe in a stoichiometric amount. The product was used without being further purified.

$^1H$ NMR (400 MHz, ACETONE-D6)

Rotamer A

Δ8.09-7.88 (m, 1H), 7.42-7.04 (m, 5H), 4.67 (dd, J=11.0, 4.6 Hz, 1H), 4.59 (t, J=7.5 Hz, 1H), 3.69 (s, 3H), 3.22 (dd, J=13.7, 4.6 Hz, 1H), 3.00 (dd, J=14.0, 10.7 Hz, 1H), 2.00-1.80 (m, 2H), 1.48-1.18 (m, 2H), 0.83-0.73 (m, 3H)

Rotamer B

7.60 (t, J=7.8 Hz, 1H), 7.42-7.04 (m, 5H), 4.75 (t, J=6.9 Hz, 1H), 4.25 (t, J=6.4 Hz, 1H), 3.64 (s, 3H), 3.10 (t, J=7.3 Hz, 2H), 2.00-1.80 (m, 2H), 1.48-1.18 (m, 2H), 0.86 (t, J=7.3 Hz, 3H)

[Comparative Example 2]

A tripeptide having a butyl group (H-Ala-Nle-Phe-OMe) was synthesized.

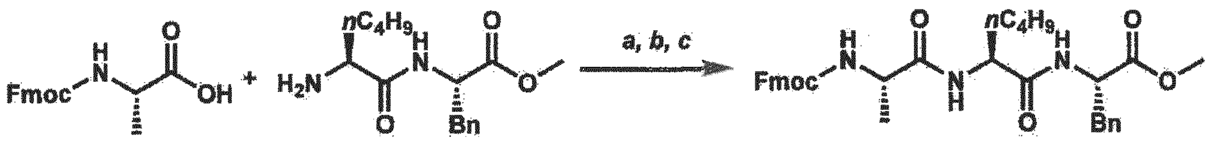


(*S*)-Fmoc-Ala-OH 1.1 eq

(*S*,*S*)-H-Nle-Phe-OMe 481 mg 1.64 mmol

(*S*,*S*,*S*)-Fmoc-Ala-Nle-Phe-OMe

*a*: DCM 7 mL, HOAt (1.1 eq), DIPEA (1.3 eq), HATU (1.1 eq), *rt, ON*
*b*: quenched by 1N HCl, extracted with DCM, evap, diluted with EtOAc, washed with 1N HCl, $NaHCO_3$ aq, brine, dried with $Na_2SO_4$, evap, dried in vacuo
*c*: column chromatography (Hex:EtOAc = 3:1)

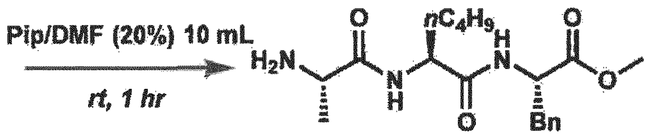


(*S*,*S*,*S*)-H-Ala-Nle-Phe-OMe
Yield 23.0 %

Into a 50 mL volume two-necked round-bottom flask, Fmoc-Ala-OH (1.1 equivalents), HOAt (1.1 equivalents) and DIPEA (1.3 equivalents) were added. HATU (1.1 equivalents) dissolved in 20 mL of DCM and a dipeptide (H-N1-Phe-OMe, 1.42 mmol) were added to the mixture at 0°C. The mixture was allowed to room temperature and then stirred for 1.5 hours, and HCl (1N) was added to terminate the reaction. The reaction mixture was partitioned between HCl (1N) and DCM. The resulting organic phase put together was distilled under reduced pressure and diluted with ethyl acetate. The organic phase was washed with HCl (1N), a saturated aqueous $NaHCO_3$ solution and brine, dried over $Na_2SO_4$ and evaporated to obtain a white solid. To the crude mixture, 25 mL of a 20% piperidine DMF solution was added, followed by stirring at room temperature for 1 hour. The solvent was removed by vacuum drying to obtain a white solid, which was purified by silica gel column chromatography ($Et_2O$:DCM=1:3) to obtain H-Ala-Nle-Phe-OMe (dr>95, 116 mg, yield: 23.0%).

MALDI-TOF MS

$[M+H]^+$:m/z calcd.for 364.22, found 364.07
$[M+H]^+$:m/z calcd.for 386.21, found 386.06

[Comparative Example 3]

To the N-terminal of the tripeptide having a butyl group (H-Ala-Nle-Phe-OMe) synthetized in Comparative Example 2, fluorescent material Alexa Fluor 647 was fused.

Into a 1.5 mL volume black tube, Alexa Fluor 647 (250 μg) dissolved in dry DMSO (15 μL), H-Ala-Nle-Phe-OMe (1.5 equivalents) dissolved in dry DMSO (15 μL) and DIPEA (1.5 equivalents) were added. The mixture was kept being stirred at room temperature overnight. The mixture was purified by reversed-phase chromatography (acetonitrile/water/TFA=30:70:0.1 to 95:5:0.1) and freeze-dried to obtain fluorescent conjugate 3 as a blue solid (yield as

calculated by fluorometer: 74.5%, emission wavelength=650 nm).

MALDI-TOF MS

$[M]^-$:m/z calcd.for $C_{55}H_{72}N_5O_{17}S_4^-$ 1202.3812, found 1202.1449

[Test Example 1]

The fluorescent peptide conjugate 1 (Alexa-Ala-RFAA-Phe-OMe) synthesized in Example 6 and the fluorescent peptide conjugate 3 (Alexa-Ala-Nle-Phe-OMe) synthesized in Comparative Example 3 were brought into contact with cultured cells to examine the uptake efficiency by the cells. Further, for comparison, fluorescent peptide conjugate 4 (Alexa-Ala-Ala-Phe-OMe) having fluorescent material Alexa Fluoro 647 fused to the N-terminal of a tripeptide having no butyl group (H-Ala-Ala-Phe-OMe), was also used.

HeLa cells were inoculated into a cover glass chamber 24 hours before the peptide treatment ($0.5\times10^5$ cells/well).

Cell uptake assay was conducted by changing a medium (DMEM low glucose medium containing 10% FBS and 1% penicillin-streptomycin solution) with a 0.4% DMSO medium (no additives) having the fluorescent peptide conjugate 1 or 2 added so that the final concentration would be 3.3 μM. The cells after the medium change were incubated at 37°C for 1 hour and washed with the cell culture medium and PBS (phosphate-buffered saline).

The cells were treated with TrypLETM Express (Gibco) and recovered, and analyzed by flow cytometry (guava easyCyte (trademark) 8). Red2 fluorescence (661/19 nm) was measured. The results are shown in Fig. 1. The vertical axis indicates the number of cells (count), and the horizontal axis indicates fluorescence intensity of the respective cells. As shown in Fig. 1, with the fluorescent peptide conjugate 1 (Alexa-Ala-RFAA-Phe-OMe), as compared with the fluorescent peptide conjugate 3, the proportion of cells emitting fluorescence of Alexa Fluor 647 was higher by twice or more. From the result, it was suggested that Alexa-Ala-RFAA-Phe-OMe, a peptide having a fluoroalkyl group, exhibits a higher cell uptake efficiency and is excellent in cell permeability, as compared with a peptide having no fluoroalkyl group.

[Test Example 2]

The cell uptake efficiency of the fluorescent peptide conjugate 2 (Alexa-Ala-RFAA (C8)-Phe-OMe) synthesized in Example 8 was examined. For comparison, the fluorescent peptide conjugate 1 (Alexa-Ala-RFAA-Phe-OMe), the fluorescent peptide conjugate 3 (Alexa-Ala-Nle-Phe-OMe) and the fluorescent peptide conjugate 4 (Alexa-Ala-Ala-Phe-OMe) were also used.

The HeLa cells were seeded on a 12-well cover glass chamber 24 hours before the peptide treatment ($1.0\times10^5$ cells/well).

Cell uptake assay was conducted in the same manner as in Test Example 1 except that each of the fluorescent peptide conjugates 1, 2, 3 and 4 was added so that the final concentration would be 1.5 μM. Then, in the same manner as in Test Example 1, the cells were recovered and analyzed by flow cytometry.

The results are shown in Fig. 1. The vertical axis indicates the number of cells (count), and the horizontal axis indicates fluorescence intensity of the respective cells. As shown in Fig. 2, with the fluorescent peptide conjugate 2 (Alexa-Ala-RFAA (C8)-Phe-OMe), as compared with the fluorescent peptide conjugates 3 and 4, the proportion of cells emitting fluorescence of Alexa Fluor 647 was higher by 16 times or more.
Further, with the fluorescent peptide conjugate 2, as compared with the fluorescent peptide conjugate 1, the proportion of cells emitting fluorescence of Alexa Fluor 647 was higher by 6 times or more. From these results, it was suggested that Alexa-Ala-RFAA-Phe-OMe, a peptide having a fluoroalkyl group, exhibits a higher cell uptake efficiency and is more excellent in cell permeability, than a peptide having no fluoroalkyl group.

[Example 9]

A tripeptide having a tridecafluorohexyl group (H-Ala-RFAA-Phe-OMe) was synthesized.

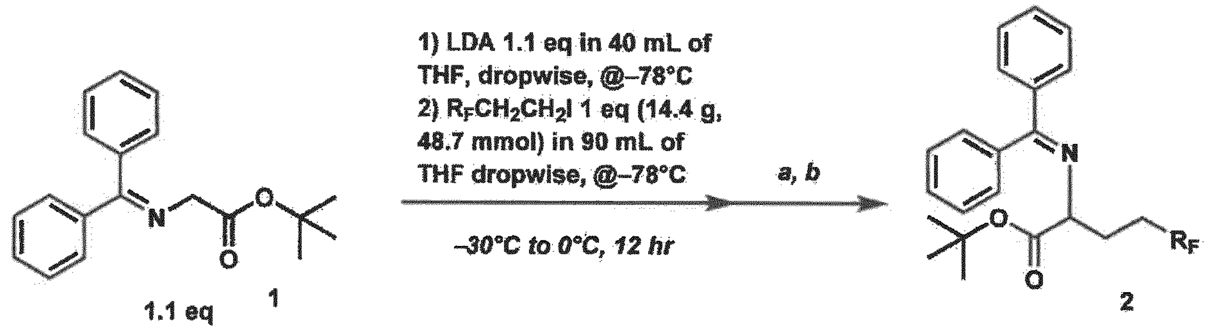


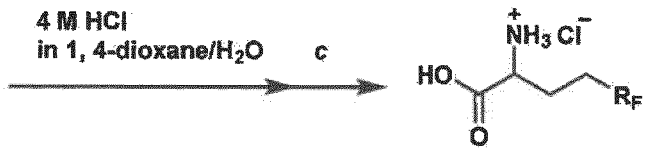


2 step yield 50.2% 3

a: quenched w/ water @0°C, extracted w/ DCM, washed with brine, evap, No TM in organic layer
b: aqueous phase was evaporated, then extracted w/ AK225, evap, dried in vacuo
c: diluted w/brine, filtration, washed w/acetone, filtrate was dried in vacuo

The compound 1 (200 mg) dissolved in THF (1 mL) was added to LDA(2.2 equivalents) and kept at -78°C in dry THF (1 mL) in an argon atmosphere for 30 minutes. Then, $RFCH_2CH_2I$ (1.1 equivalents) was added to the mixture and stirred for 3 hours. Then, the temperature of the mixture was slowly increased to -30°C, the mixture was stirred overnight, and water (5 mL) was added to the mixture at 0°C to terminate the reaction. Then, the mixture was extracted with $CH_2Cl_2$ (200 mLx3). The product was purified by column chromatography with alumina to obtain a white solid (yield: 54.1%).

$^1H$ NMR (400 MHz $CDCl_3$) δ=1.45 (s, 9H), 2.78-2.66 (m, 2H), 3.23-3.27 (m, 2H), 4.03 (t, 1H), 7.2-7.7 (m, 10H)

$^{19}F$ NMR (376 MHz $CDCl_3$) δ=-126.2 (m, 2F), -123.4 (m, 2F), -122.9 (m, 2F), -121.9 (m, 2F), 114.9 (m, 1F), 114.2 (m, 1F), -80.8 (t, 3F)

HCl (6M, 50 mL) and a solution of the compound 2 (50.8 mmol) dissolved in 1,4-dioxane (200 mL) were heated at 80°C for 24 hours. The solution was subjected to filtration, and the precipitate was washed with acetone several times. The obtained white solid had a sufficient purity to be used in the next step without being further purified (yield: 50.2%).

$^1H$ NMR (400 MHz MeOH-d4) δ=2.11 (m, 2H), 2.35 (m, 1H), 2.52 (m, 1H), 3.68 (m, 1H)

$^{19}F$ NMR (376 MHz MeOH-d4) δ=-127.2 (m, 2F), -124.4 (m, 2F), -123.8 (m, 2F), -122.8 (m, 2F), -115.7 (m, 2F), -82.3 (t, 3F)

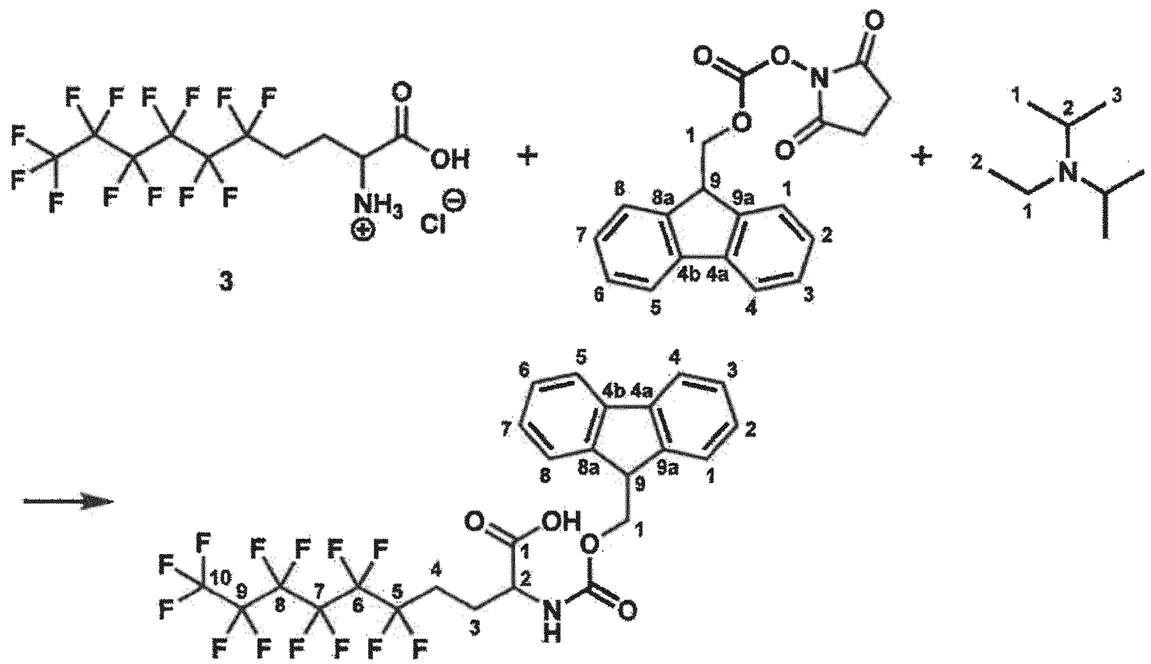


Into a 50 mL volume two-necked round-bottom flask, a stirrer was put, and the compound 3 (100 mg, 0.22 mmol) and DCM (10 mL) were added. Fmoc-OSn (0.24 mmol) and DIPEA (1.3 equivalents) were added at room temperature, and the reaction mixture was stirred at room temperature for 20 hours. The reaction mixture was directly evaporated and the residue was purified by silica gel column chromatography (MeOH/$CHCl_3$=1/9) to obtain a white solid (yield: 64.2%).

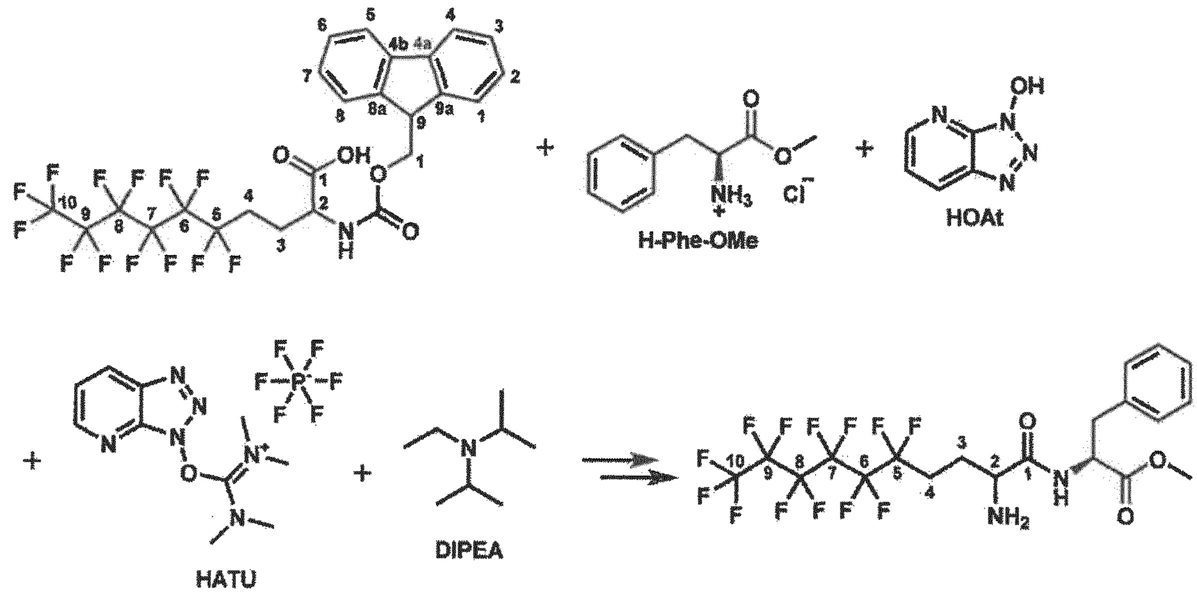


Into a 25 mL volume two-necked round-bottom flask, Fmoc-RFAA-OH (90.4 mg, 0.14 mmol), HOAt (1.2 equivalents) and DIPEA(1.3 equivalents) were added. To the mixture, HATU (1.2 equivalents) dissolved in DCM (5 mL) and H-Phe-OMe (HCl salt, 1.2 equivalents) were added at 0°C, and the mixture was allowed to room temperature and stirred for 4 hours. Then, HCl (1N) was added to terminate the reaction, and the mixture was partitioned between HCl (1N) and DCM. The resulting organic phase put together was evaporated, and the residue was diluted with ethyl acetate. The organic phase was washed with HCl (1N), a saturated aqueous $NaHCO_3$ solution and brine, dried over $Na_2SO_4$, and evaporated to obtain a white solid. To the white solid, a 20% piperidine DMF solution (25 mL) was added, followed by stirring at room temperature for 1 hour. The solvent was removed by vacuum drying to obtain a white solid, which was purified by silica gel column chromatography ($CHCl_3$:MeOH=10:1) to obtain H-RFAA-Phe-OMe (41.4 mg, yield: 64.8%).

$^{1}$H NMR (400 MHz Acetone-d6) δ=1.96 (m, 2H), 2.32 (m, 2H), 2.81 (br s, NH2), 2.99-3.15 (m, 2H), 3.15 (s, 3H), 4.05 (t, 1H), 4.71 (m, 1H)7.2-7.7 (m, 5H)

$^{19}$F NMR (376 MHzAcetone-d6) δ=-126.7 (m, 2F), -123.8 (m, 2F), -123.4 (m, 2F), -122.4 (m, 2F), -114.6 (m, 2F), -81.4 (t, 3F)

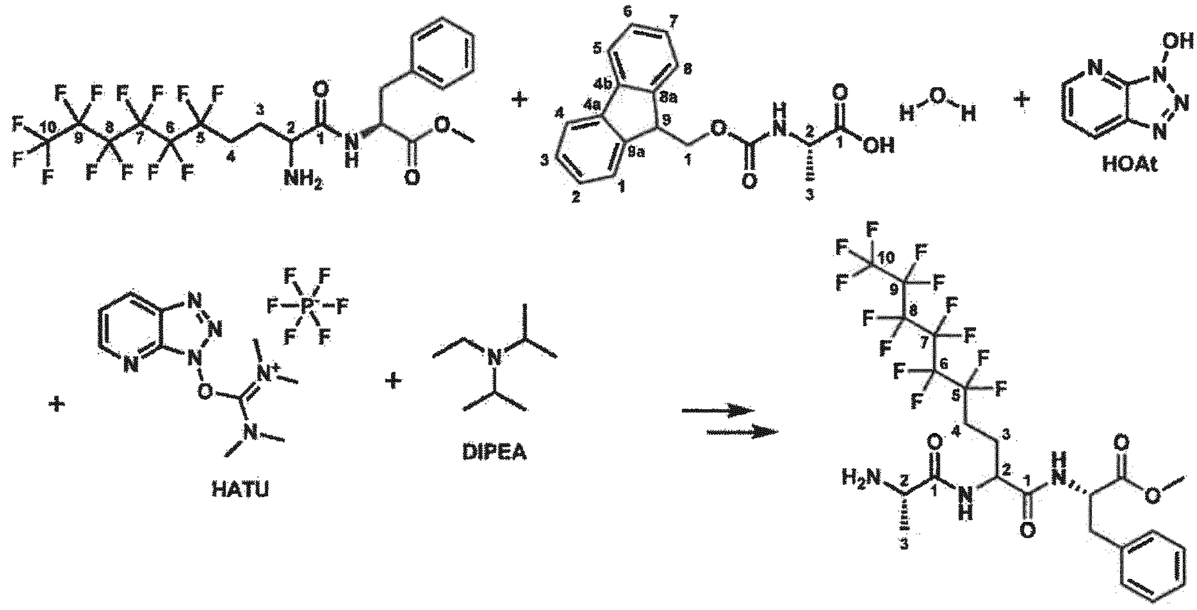


Into a 25 mL volume two-necked round-bottom flask, Fmoc-RFAA-OH (44.8 μmol), HOAt (1.2 equivalents) and DIPEA (1.3 equivalents) were added. HATU (1.2 equivalents) dissolved in DCM (5 mL) and Fmoc-AlaOH (1.2 equivalents) were added to the mixture at 0°C, and the mixture was allowed to room temperature and stirred for 4 hours. HCl (1N) was added to terminate the reaction, and the mixture was partitioned between HCl (1N) and DCM. The resulting organic phase put together was evaporated, and the residue was diluted with ethyl acetate. The organic phase was washed with HCl (1N), a saturated aqueous $NaHCO_3$ solution and brine, dried over $Na_2SO_4$, and evaporated to obtain a white solid. To the crude mixture, 5 mL of a 20% piperidine DMF solution was added, followed by stirring at room temperature for one hour. The solvent was distilled off under reduced pressure to obtain a white solid, which was purified by HPLC.

ESI-MS
$[M+H]^+$:m/z calcd.for 754.16, found 654.52

[Comparative Example 4]

A tripeptide having an octyl group (Boc-nOctyl-Phe-OMe) was synthesized.

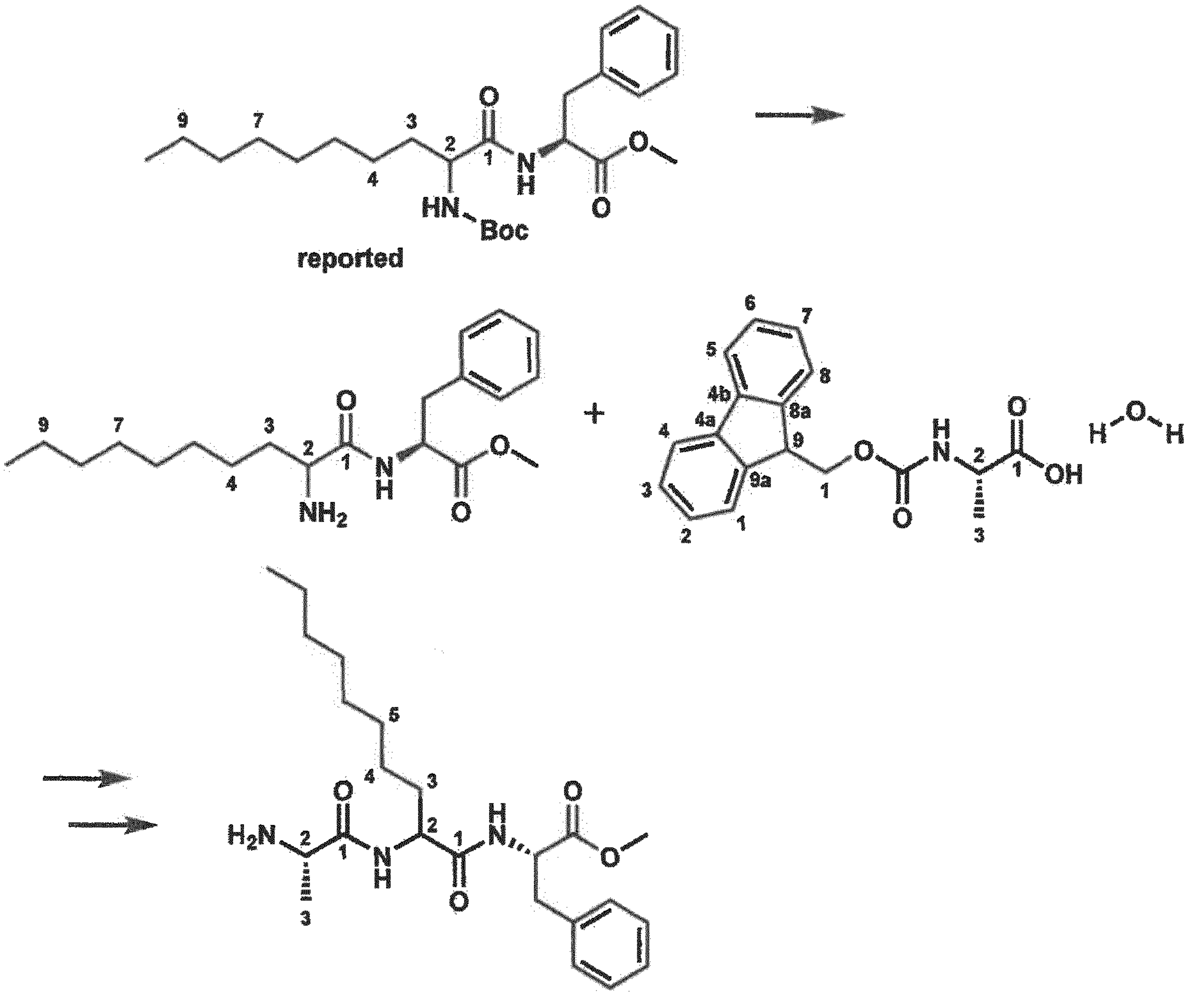


Boc-nOctyl-Phe-OMe was produced by the method in accordance with known literature (Liebigs Annalen der Chemie, 1990, 12p, p. 1175-1183). Removal of Boc by deprotection from the dipeptide was conducted by the same standard procedure as above (yield: 100%). Further, the synthesis of the tripeptide was conducted in accordance with the above procedure.

ESI-MS
$[M+H]^+$:m/z calcd.for 420.29, found 420.72

[Example 10]

A dipeptide having a heptadecafluorooctyl group (Boc-RFAA (C8)-Gly-OMe) was synthesized.

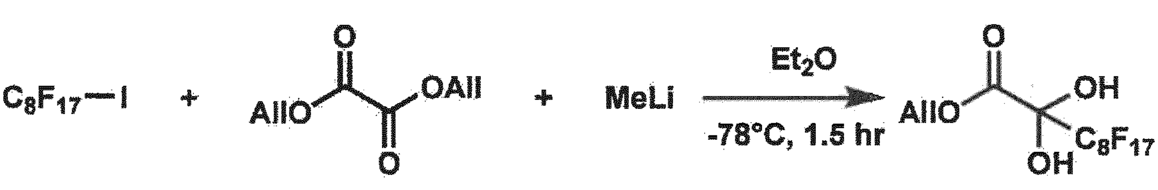


In the same manner as in Example 7 except that diallyl oxalate (3.4 g) was used instead of dibenzyl oxalate, crude allyl 3,3,4,4,5,5,6,6,7,7,8,8,9,9,10,10,10-hexadecafluoro-2,2-dihydrodecanoate was obtained with a yield of 80.9%. The obtained product was subjected to the next step without being purified.
$^1$H NMR (400 MHz Acetone-d6) $\delta$=6.25-5.71 (m, 1H), 5.65-5.03 (m, 2H), 4.97-4.52 (m, 2H)
$^{19}$F NMR (400 MHz Acetone-d6) $\delta$=81.72 (m, 3F), -120.30 (s, 4F), -122.24 (s, 6F), -123.26 (s, 2F), -126.79 (d, J=54.5 Hz, 2F)

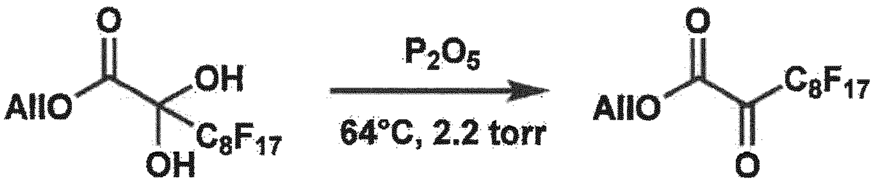


In the same manner as in Example 7 except that the obtained crude product was purified by sublimation at 64°C under 2.2 mmHg, allyl 3,3,4,4,5,5,6,6,7,7,8,8,9,9,10,10,10-hexadecafluoro-2-oxodecanoate was obtained with a yield of 60.3%.

$^1H$ NMR (400 MHz, $CDCl_3$) δ=6.08-5.79 (m, 1H), 5.59-5.13 (m, 2H), 4.89-4.80 (m, 2H)

$^{19}F$ NMR (400 MHz, $CDCl_3$) δ=-81.03 (t, J=10.0 Hz, 3F), -117.88 (t, J=12.9 Hz, 2F), -121.25 (m, 4F), -121.96 (m4F), -122.85 (s, 2F), -126.31 (d, J=5.7 Hz, 2F)

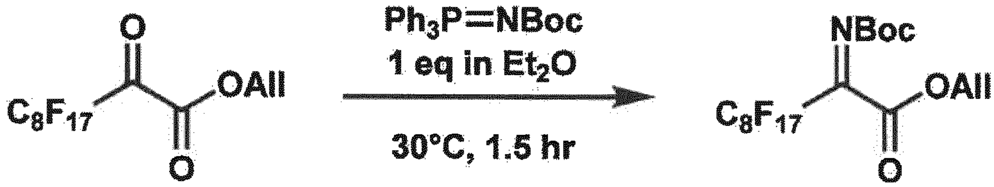


Then, in the same manner as in Example 7 except that in the silica gel chromatography, an eluent was one having 1% triethylamine added to hexane:ethyl acetate=9:1, α-imino ester was obtained with a yield of 95.1%.

$^1H$ NMR (400 MHz, $CDCl_3$) δ=6.01-5.82 (m, 1H), 5.49-5.30 (m, 2H), 4.81 (d, J=5.9 Hz, 2H), 1.63-1.49 (m, 9H)

$^{19}F$ NMR (400 MHz, $CDCl_3$) δ=-79.98 to -82.00 (m, 3F), -112.58 (m, 2F), -120.10 (s, 2F), -121.11 (s, 2F), -121.80 (m, 4F), -122.71 (s, 2F), -126.38 (m, 2F)

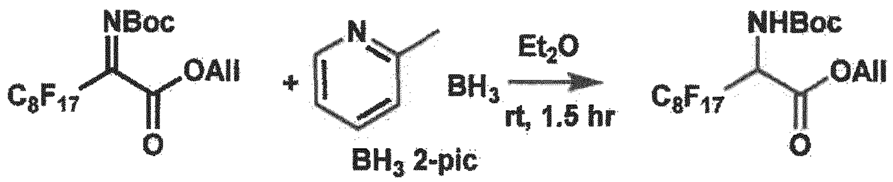


To a dry diethyl ether (30 mL) solution of the obtained imino ester (2.0 g, 3.2 mmol), 2-picoline borane (1 equivalent) was added at 0°C with stirring. The reaction mixture was stirred at room temperature for 1.5 hours and diluted with HCl (1N) (20 mL). The resulting organic phase was washed with HCl (1N) twice and concentrated under reduced pressure, and the obtained crude product was purified by silica gel column chromatography (hexane:ethyl acetate=9:1) to obtain α-butoxycarbonylamino ester as a yellow solid (yield: 54%).

$^1H$ NMR (400 MHz, $CDCl_3$) δ6.01-5.79 (m, 1H), 5.51-4.97 (m, 4H), 4.84-4.62 (m, 2H), 1.57-1.35 (m, 9H)

$^{19}F$ NMR (400 MHz, $CDCl_3$) δ-80.96 (t, J=10.0 Hz, 3F), -115.81 (d, J=280 Hz, 1F), -118.10 (d, J=281 Hz, 1F), -120.88 to -123.39 (m, 10F), -126.23 (m, 2F)

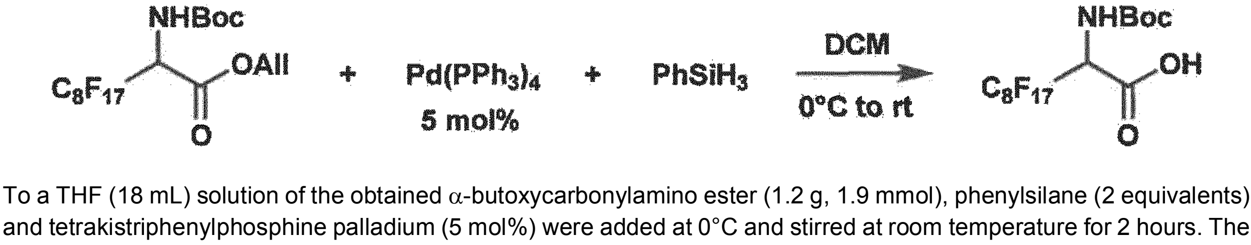


To a THF (18 mL) solution of the obtained α-butoxycarbonylamino ester (1.2 g, 1.9 mmol), phenylsilane (2 equivalents) and tetrakistriphenylphosphine palladium (5 mol%) were added at 0°C and stirred at room temperature for 2 hours. The reaction mixture was diluted with HCl (1N) (10 mL) and extracted with DCM twice. The resulting organic phase was concentrated under reduced pressure, and the obtained crude product was purified by silica gel column chromatography (chloroform:methanol=6:1/1% acetic acid) to obtain 2-((tert-butoxycarbonyl)amino)-3,3,4,4,5,5,6,6,7,7,8,8,9,9,10,10,10-heptadecafluorododecanoic acid as a pale yellow liquid (yield: 74%).

$^1H$ NMR (400 MHz, $CDCl_3$) δ=5.03 (m, J=8.4 Hz, 1H), 1.38 (s, 9H)

$^{19}F$ NMR (400 MHz, $CDCl_3$) δ=-80.87 (t, J=10.0 Hz, 3F), -115.78 (d, J=281.1 Hz, 1F), -118.12 (d, J=281.1 Hz,

1F), -120.16 to -123.43 (m, 10F), -126.19 (s, 2F)

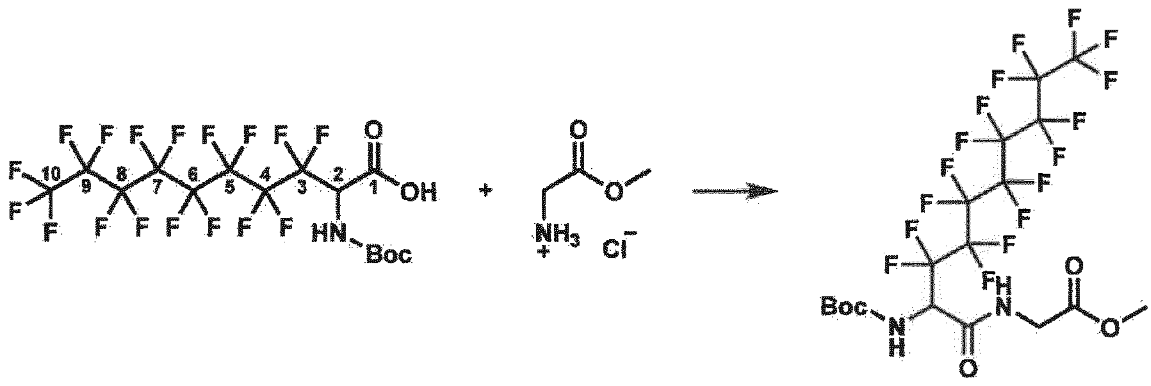

In a dried 25 mL volume two-necked eggplant flask, 2-((tert-butoxycarbonyl)amino)-3,3,4,4,5,5,6,6,7,7,8,8,9,9,10,10,10-heptadecafluorododecanoic acid (15.4 mg, 26.0 μmol), DCM (2 mL), DIPEA (57 μmol), glycine methyl ester hydrochloride (29 μmol) and ethyl (hydroxyimino)cyanoacetate (oxyma) (CAS RN:3849-21-6) (29 μmol) were mixed and stirred. The reaction mixture was cooled to 0°C, and (1-cycno-2-ethoxy-2-oxoethylidenaminooxy)dimethylaminomorpholinocarbenium hexafluorophosphate (COMU) (CAS RN:1075198-30-9) (29 μmol) was added, followed by stirring at room temperature for 1.5 hours. Then, the reaction mixture was quenched with HCl (1N) and extracted with DCM three times. The resulting organic phase put together was concentrated under reduced pressure, diluted with ethyl acetate and washed with HCl (1N), a saturated aqueous sodium hydrogen carbonate solution and a saturated saline solution. The resulting organic phase after washed was dried over sodium sulfate and subjected to filtration, and the filtrate was concentrated under reduced pressure to obtain crude Boc-RFAA (C8)-Gly-OMe. The crude product was purified by silica gel chromatography (ethyl acetate/hexane=1/3) to obtain Boc-RFAA (C8)-Gly-OMe (yield: 92%).

$^{1}H$ NMR (400 MHz, Acetone-d6) δ=8.25 (t, J=5.3 Hz, 1H), 6.66 (d, J=9.6 Hz, 1H), 5.44-5.19 (m, 1H), 4.07 (d, J=5.5 Hz, 2H), 3.68 (s, 3H), 1.42 (s, 9H)

$^{19}F$ NMR (400 MHz, Acetone-d6) δ=-81.53 (s, 3F), -115.31 (d, J=281.1 Hz, 1F), -119.15 to -120.92 (m, 1F), -120.92 to -124.38 (10F), -125.54 to -127.82 (m, 2F)

[Example 11]

A dipeptide having a heptadecafluorooctyl group (Boc-RFAA (C8)-Ala-OMe) was synthesized.

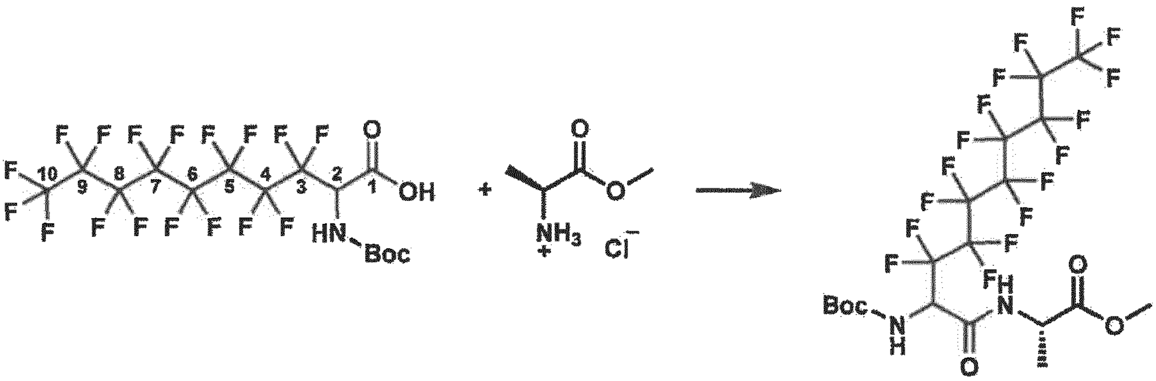

In the same manner as in Example 10 except that alanine methyl ester hydrochloride was used instead of glycine methyl ester hydrochloride, Boc-RFAA (C8)-Ala-OMe (47:53 mixture of diastereomers) was obtained (yield: 74%) from 2-((tert-butoxycarbonyl)amino)-3,3,4,4,5,5,6,6,7,7,8,8,9,9,10,10,10-heptadecafluorododecanoic acid (15 μmol).

$^{1}H$ NMR (400 MHz, Acetone-d6) δ=8.22-8.27 (d, J=7.1 Hz, 1H), 6.82-6.43 (m, 1H), 5.44-5.19 (m, 1H), 4.64-4.40 (m, 1H), 3.68 (s, 3H), 1.42 (s, 9H), 1.38-1.40 (d, 3H)

$^{19}F$ NMR (400 MHz, Acetone-d6) δ=-80.91 (t, J=10.0 Hz, 3F), -113.87 to -115.77 (m, 1F), -119.22 to -119.98

(m, 1F), -120.67 to -121.35 (m, 2F), -121.48 (m, 6F), -122.03 to -122.81 (m, 2F), -126.00 (m, 2F)

[Example 12]

A dipeptide having a heptadecafluorooctyl group (Boc-RFAA (C8)-Leu-OMe) was synthesized.

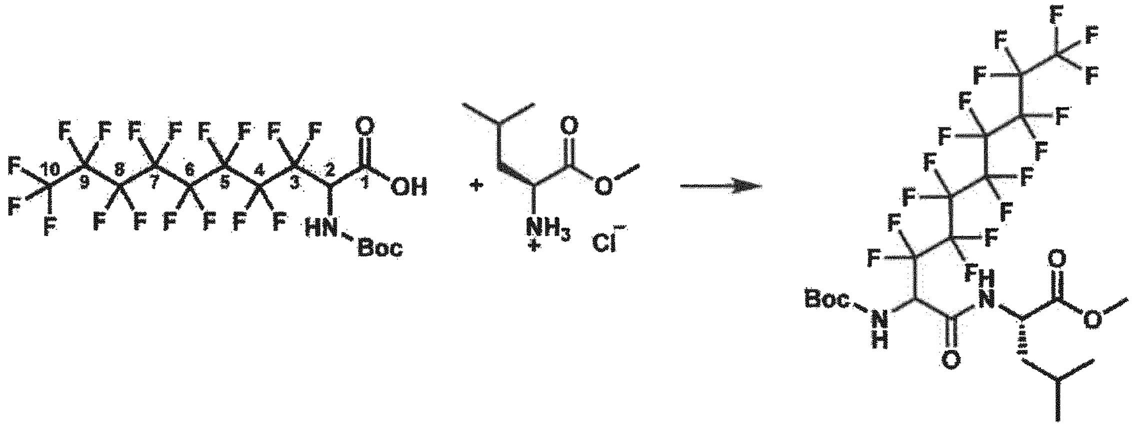

In the same manner as in Example 10 except that leucine methyl ester hydrochloride was used instead of glycine methyl ester hydrochloride and that no silica gel chromatography was conducted, Boc-RFAA (C8)-Leu-OMe (49:51 mixture of diastereomers) (yield: 83%) was obtained from 2-((tert-butoxycarbonyl)amino)-3,3,4,4,5,5,6,6,7,7,8,8,9,9,10,10,10-heptadecafluorododecanoic acid (15 μmol).

$^{1}H$ NMR (400 MHz, Acetone-d6) δ=8.19 (d, J=7.5 Hz, 1H), 6.64 (d, J=10.1 Hz, 1H), 5.45-5.13 (m, 1H), 4.68-4.47 (m, 1H), 3.68 (s, 3H), 1.81-1.66 (m, 1H), 1.42 (s, 9H), 0.96-0.88 (m, 6H)

$^{19}F$ NMR (400 MHz, Acetone-d6) δ=-81.56 (t, J=10.0 Hz, 3F), -115.49 (m, J=272.51F), -119.40 to -120.88 (m, 1F), -121.33 to -123.04 (m, 10F), -126.63 (m, 2F)

[Example 13]

A dipeptide having a heptadecafluorooctyl group (Boc-RFAA (C8)-Lys (Boc)-OMe) was synthesized.

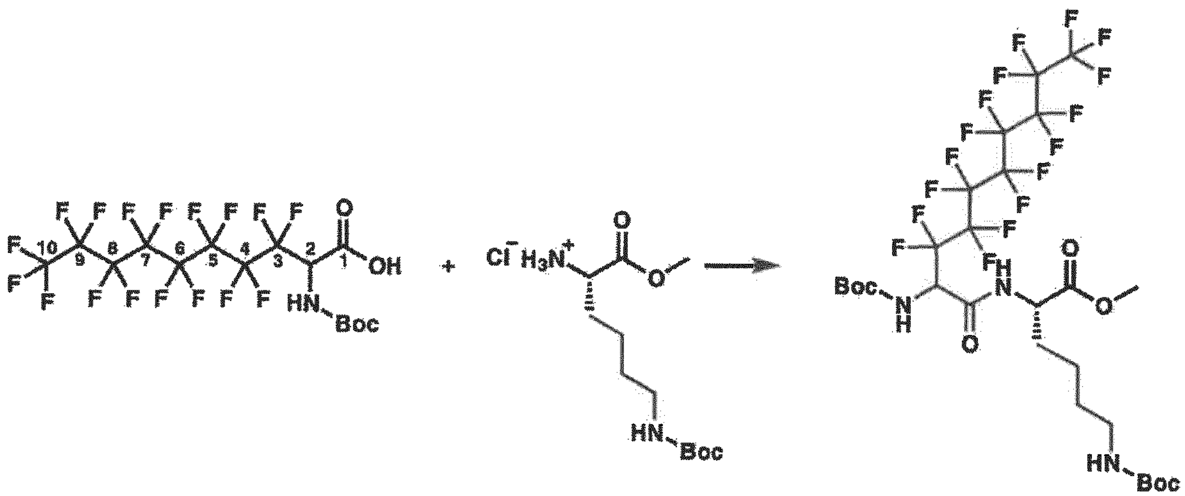

In the same manner as in Example 10 except that lysine (Boc)methyl ester hydrochloride was used instead of glycine methyl ester hydrochloride, Boc-RFAA (C8)-Lys (Boc)-OMe was obtained (yield: 69%).

$^{1}H$ NMR (400 MHz, $CDCl_3$) δ 7.71 (d, J=3.7 Hz, 1H), 7.51-7.54 (m, 1H), 6.94 (d, J=8.2 Hz, 1H), 5.67 (d, J=10.1 Hz, 1H), 5.13 (s, 1H), 4.57-4.62 (m, 2H), 3.75 (s, 3H), 1.45 (s, 18H), 1.10-1.90 (m, 6H)

$^{19}F$ NMR (400 MHz, $CDCl_3$) δ -80.63 (s, 3F), -114.56 (d, J=281.1 Hz, 1F), -119.10 (d, J=284.0 Hz, 1F), -122.61--120.86 (m, 10F), -126.02 (s, 2F)

LRMS (ESI-TOF)

$[M+Na]^+$:calcd.for $C_{27}H_{34}F_{17}N_3NaO_7$ 858.20, found 858.03

[Comparative Example 5]

Ac-L-Ala-L-Ala-NHBn was synthesized.

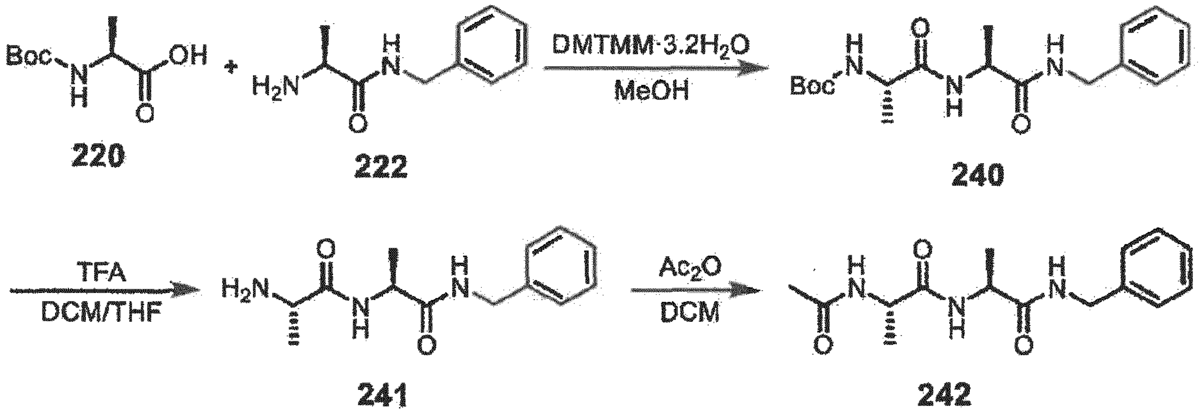


220 222 240 241 242

The compound 220 (61 mg, 0.32 mmol) and the compound 222 (68 mg) were dissolved in 3 mL of methanol, and to the solution, DMTMM (4-(4,6-dimethoxy-1,3,5-triazin-2-yl)-4-methylmorpholinium chloride)·$3.2H_2O$ (135 mg) was added. The reaction mixture was stirred at room temperature for 12 hours and evaporated in vacuum. To the reaction mixture, DCM was added, and the solution was washed with a 1M aqueous $Na_2CO_3$ solution, water, a 1M aqueous HCl solution, water and brine. The resulting organic phase was dried over $Na_2SO_4$ and evaporated in vacuum to obtain compound 240 (92 mg, yield: 83%).

The compound 240 (92 mg, 0.26 mmol) was dissolved in 4 mL of DCM and 1 mL of THF. To the solution, 1.25 mL of TFA was added at 0°C, and the mixture was stirred for 15 minutes. Then, the reaction mixture was allowed to room temperature and stirred for 3 hours. To the reaction mixture, a 1M aqueous $NaHCO_3$ solution was added, and the obtained solution was stirred for 2 hours. The mixture was extracted with DCM four times. The resulting organic phase was dried over $Na_2SO_4$ and evaporated in vacuum to obtain compound 241.

To the compound 241 (73 mg, 0.29 mmol) in 3 mL of DCM, acetic anhydride (33 μL) was added. The reaction mixture was stirred at room temperature for 24 hours and evaporated in vacuum. The residue was dissolved in 10 mL of a 40% aqueous acetonitrile solution and purified by a HPLC reversed-phase column to obtain compound 242 (9 mg, yield: 11%).

$^1H$ NMR ($CD_3OD$, 400 MHz):δ7.33-7.20 (m, 5H), 4.42-4.33 (m, 3H), 4.29 (q, J=6.9 Hz, 1H), 1.96 (s, 3H), 1.38 (d, J=6.87 Hz, 3H), 1.33 (d, J=7.3 Hz, 3H).

MS (MALDI-TOF MS.m/z)

$[M+Na]^+$:calcd.for $C_{15}H_{21}N_3O_3Na$ 314.15, found 313.88.

[Example 14]

Ac-D,L-Ala ($F_3$)-L-Ala-NHBn was synthesized.

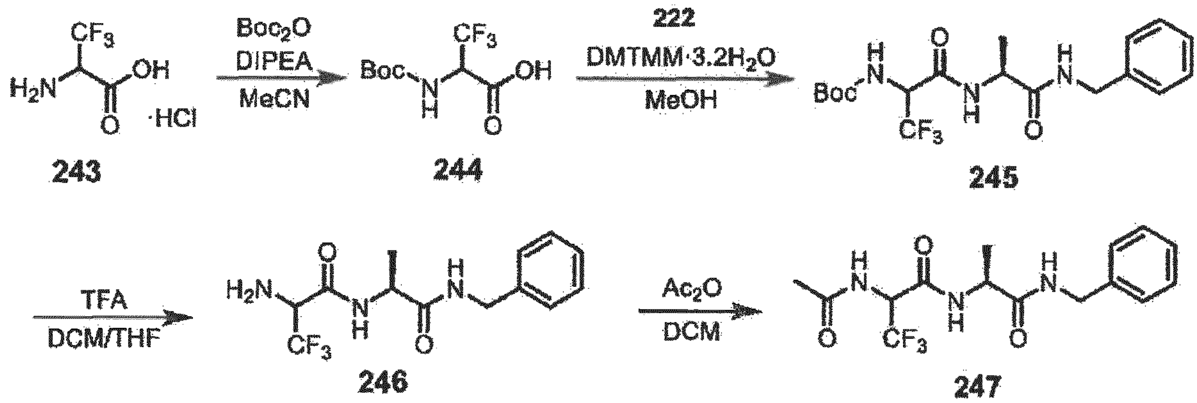


243 244 245 246 247

D,L-trifluoroalanine hydrochloride (compound 243) (52 mg, 0.28 mmol) was dissolved in 3 mL of acetonitrile. To the solution, DIPEA (57 μL) and di-tert-butyl dicarbonate (77 μL) were added at 0°C. The reaction mixture was allowed to room temperature over a period of 21 hours. The solution was evaporated in vacuum, and water was added to the residue. The solution was extracted with diethyl ether three times. To the aqueous phase, a 1M aqueous HCl solution was added, and the solution was extracted with diethyl ether three times. The resulting organic phase put together was dried over $Na_2SO_4$ and evaporated in vacuum to obtain compound 244 as a white solid (62 mg, yield: 91%).

The compound 244 (40 mg, 0.16 mmol) and the compound 222 (29 mg) were dissolved in 1.5 mL of methanol, and to the solution, DMTMM·3.2$H_2O$ (62 mg) was added. The reaction mixture was stirred at room temperature for 11.5 hours and evaporated in vacuum. To the reaction mixture, DCM was added, and the obtained solution was washed with a 1M aqueous $Na_2CO_3$ solution, water, a 1M aqueous HCl solution, water and brine. The resulting organic phase was dried over $Na_2SO_4$ and evaporated in vacuum to obtain compound 245 (45 mg, yield: 68%).

The compound 245 (45 mg, 0.11 mmol) was dissolved in 4 mL of DCM and 1 mL of THF. To the solution, 1.25 mL of TFA was added at 0°C, and the mixture was stirred for 15 minutes. The reaction mixture was allowed to room temperature and stirred for 3 hours. To the reaction mixture, a 1M aqueous $NaHCO_3$ solution was added, and the obtained solution was stirred for 2 hours. The mixture was extracted with DCM four times. The resulting organic phase was dried over $Na_2SO_4$ and evaporated in vacuum to obtain compound 246 (41 mg, quantitative yield).

To the compound 246 (41 mg, 0.14 mmol) in 3 mL of DCM, acetic anhydride (67 μL) was added. The reaction mixture was stirred at room temperature for 24 hours and evaporated in vacuum. The residue was dissolved in 10 mL of a 46% aqueous acetonitrile solution and purified by a HPLC reversed-phase column to obtain compound 247 as a white solid (0.3 mg, yield: 1%). The molecules were obtained as a diastereomer mixture and were used for permeability assay as they were.

$^1H$ NMR ($CD_3OD$, 400 MHz):δ7.33-7.22 (m, 5H), 5.37-5.29 (m, 1H), 4.45-4.38 (m, 3H), 2.66 (s, 3H), 1.40-1.38 (m, 3H).

MS (MALDI-TOF MS.m/z)

$[M+Na]^+$:calcd.for $C_{15}H_{18}F_3N_3O_3Na$ 368.12, found 367.92.

[Comparative Example 6]

Ac-L-Ala-L-Phe-iBu was synthesized.

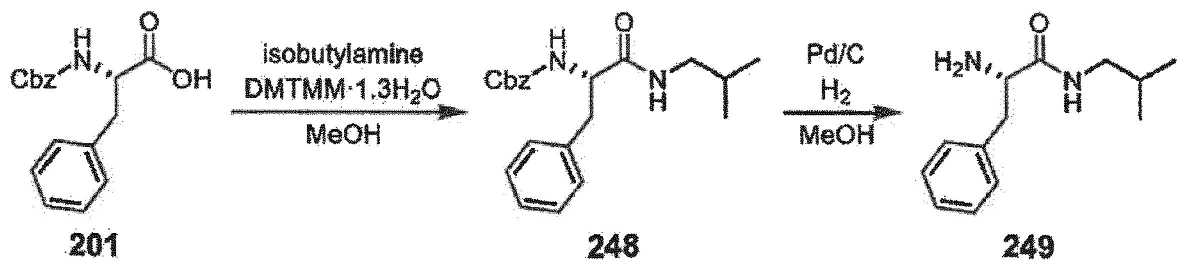


201 248 249

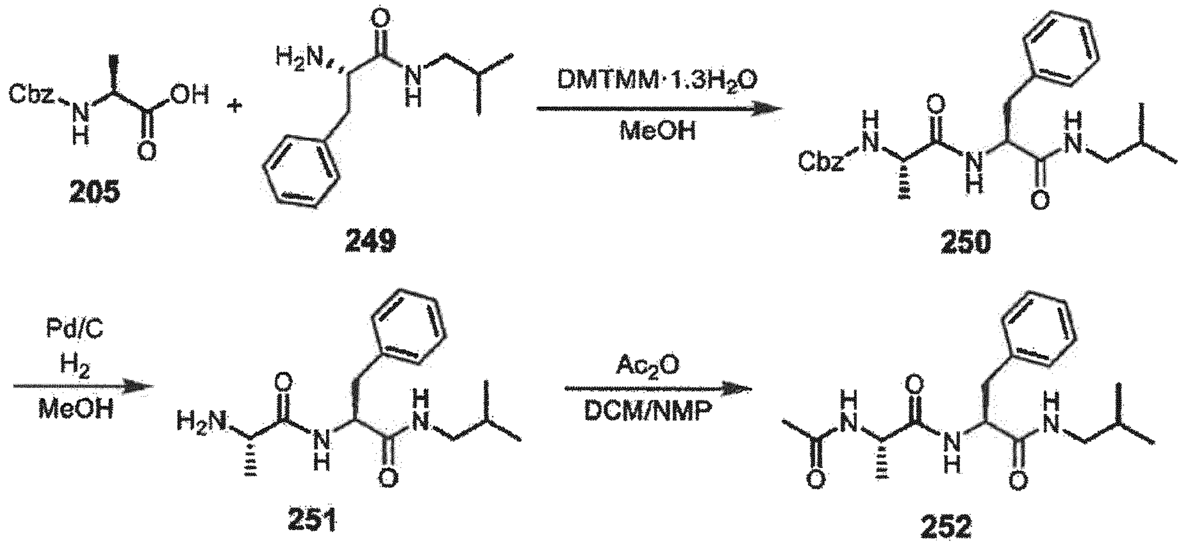


205 249 250 251 252

Compound 201 (700 mg, 2.34 mmol) and isobutylamine (181 μL) were dissolved in 23 mL of methanol, and to the solution, DMTMM·1.3$H_2O$ (838 mg) was added. The reaction mixture was stirred at room temperature for 5 hours and evaporated in vacuum. To the reaction mixture, DCM was added, and the obtained solution was washed with a 1M aqueous $Na_2CO_3$ solution, water, a 1M aqueous HCl solution, water and brine.

The resulting organic phase was dried over $Na_2SO_4$ and evaporated in vacuum. The residue was purified by silica gel column chromatography (hexane/ethyl acetate=4:6) to obtain compound 248 (523 mg, yield: 65%).

Into a recovery flask, the compound 248 (523 mg, 1.48 mmol), palladium carbon 10% (55 mg) and 7.4 mL of methanol were added. $H_2$ was introduced into the flask, and the mixture was stirred at room temperature for 15 hours. The reaction mixture was filtrated through celite. The solvent was removed under reduced pressure to obtain compound 249 (319 mg, yield: 98%).

Compound 205 (45 mg, 0.2 mmol) and the compound 249 (53 mg) was dissolved in 2 mL of methanol, and the resulting solution was stirred at room temperature. To the solution, DMTMM·1.3$H_2O$ (74 mg) was added. The reaction mixture was stirred at room temperature for 23 hours and evaporated in vacuum. To the reaction mixture, DCM was added, and the obtained solution was washed with a 1M aqueous $Na_2CO_3$ solution, water, a 1M aqueous HCl solution, water and brine. The resulting organic phase was dried over $Na_2SO_4$ and evaporated in vacuum. The residue was purified by silica gel column chromatography (DCM/methanol=19:1) to obtain compound 250 (9.1 mg, yield: 11%).

Into a recovery flask, the compound 250 (9.1 mg, 21 μmol), 10% palladium carbon (2.8 mg) and 2 mL of methanol were added. To the flask, $H_2$ was introduced, and the mixture was stirred at room temperature for 22 hours. The reaction mixture was subjected to filtration through celite. The solvent was removed under reduced pressure to obtain compound 251 (7.4 mg, quantitative yield).

To the compound 251 (4 mg, 14 μmol) in 1 mL of DCM and 0.2 mL of NMP (N-methylpyrrolidone), acetic anhydride (23 μL) was added. The reaction mixture was stirred at room temperature for 1.5 hours and evacuated in vacuum. The residue was dissolved in 3.5 mL of a 14% aqueous acetonitrile solution and purified by a HPLC reversed-phase column to obtain compound 252 (2.0 mg, yield: 43%).

$^1$H NMR ($CDCl_3$, 400 MHz):δ7.32-7.19 (m, 10H), 6.62 (d, J=7.7 Hz, 1H), 5.90-5.86 (m, 2H), 4.57 (q, J=7.7 Hz, 1H), 4.39 (dq, J=6.9, 7.1 Hz, 1H), 3.14 (dd, J=6.4, 13.9 Hz, 1H), 3.06-2.98 (m, 3H), 1.94 (s, 3H), 1.70-1.62 (m, 1H), 1.33 (d, J=7.1 Hz, 3H), 0.79 (t, J=6.3 Hz, 6H).

MS (MALDI-TOF MS.m/z)

$[M+Na]^+$:calcd.for $C_{18}H_{27}N_3O_3Na$ 356.20, found 356.23.

[Example 15]

Ac-D,L-Ala ($F_3$)-L-Phe-iBu was synthesized.

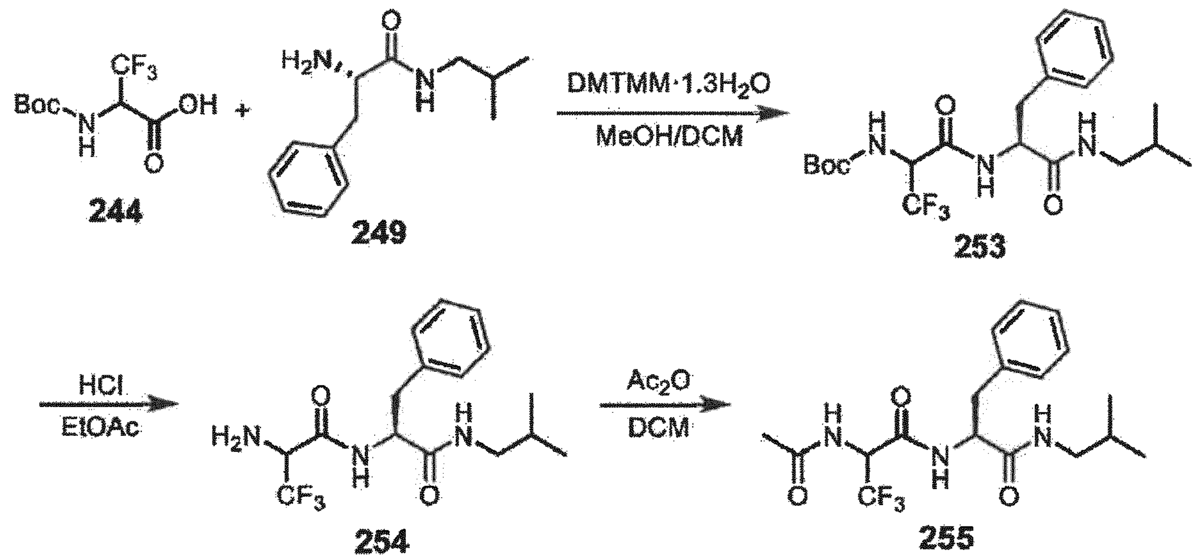


244 249 253 254 255

The compound 244 (41 mg, 0.17 mmol) and the compound 249 (45 mg) were dissolved in 1 mL of methanol and 0.7 mL of DCM, and to the obtained solution, DMTMM·1.3$H_2O$ (59 mg) was added. The reaction mixture was stirred overnight at room temperature and evaporated in vacuum. DCM was added to the reaction mixture, and the obtained solution was washed with a 1M aqueous $NaHCO_3$ solution, a saturated aqueous $NH_4Cl$ solution and brine. The resulting organic phase was dried over $Na_2SO_4$ and evaporated in vacuum. The residue was purified by silica gel column chromatography (DCM/methanol=19:1) to obtain compound 253 (47 mg, yield: 63%).

Into the compound 253 (47 mg, 0.11 mmol) in 3 mL of ethyl acetate, 4M HCl (3 mL) in ethyl acetate was added, and the obtained solution was stirred at room temperature for 20 minutes. The obtained solution was evaporated in vacuum to obtain compound 254 (45 mg, quantitative yield).

Into the compound 254 (30 mg, 79 μmol) in 2 mL of DCM, acetic anhydride (45 μL) was added. The reaction mixture was stirred at room temperature for 3 hours and evaporated in vacuum. The residue was dissolved in acetonitrile/$H_2O$/MeOH (=2.4 mL:3.6 mL:2 mL) and purified by a HPLC reversed-phase column to obtain compound 255 (9.4 mg, yield: 30%). The molecules were obtained as a diastereomer mixture and were used for permeability assay as they were.

$^1$H NMR ($CDCl_3$, 400 MHz):δ7.34-7.21 (m, 5H), 6.82-6.76 (m, 1H), 6.41 (d, J=6.4 Hz, 1H), 5.3 (s, 1H), 5.17 (q, J=7.3 Hz, 1H), 4.59-4.53 (m, 1H), 3.21-3.12 (m, 1H), 3.01-2.92 (m, 3H), 1.42 (dd, J=6.9, 12.4 Hz, 1H), 0.76 (dd, J=6.4, 10.5 Hz, 6H).

MS (MALDI-TOF MS.m/z)

$[M+Na]^+$:calcd.for $C_{18}H_{24}F_3N_3O_3Na$ 410.17, found 410.19.

[Comparative Example 7]

Ac-L-Val-L-Ala-$NMe_2$ was synthesized.

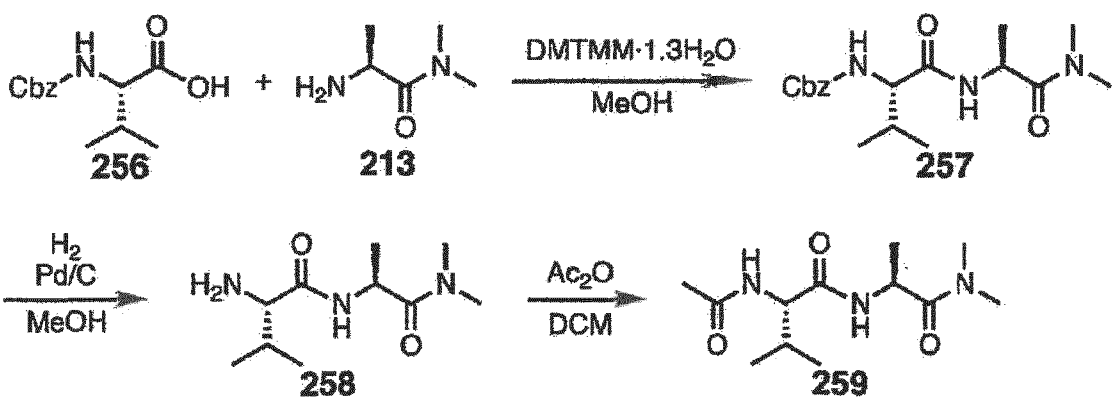


256 213 257 258 259

N-carboxybenzoxy-L-valine (compound 256) (60 mg, 0.24 mmol) and compound 213 (33 mg) were dissolved in 2.2 mL of methanol, and to the obtained solution, DMTMM·1.3$H_2O$ (91 mg) was added. The reaction mixture was stirred overnight at room temperature and evaporated in vacuum. DCM was added to the reaction mixture, and the obtained solution was washed with a 1M aqueous $Na_2CO_3$ solution, water, a 1M aqueous HCl solution, water and brine. The resulting organic phase was dried over $Na_2SO_4$ and evaporated in vacuum to obtain compound 257 (75 mg, yield: 89%).

Into a recovery flask, the compound 257 (773 mg, 3.10 mmol), palladium carbon 10% (7.5 mg) and 2.1 mL of methanol were added. To the flask, $H_2$ was introduced, and the mixture was stirred at room temperature for 18 hours. The reaction mixture was subjected to filtration through celite. The solvent was removed under reduced pressure to obtain compound 258 (35 mg, yield: 76%).

Into the compound 258 (35 mg, 0.16 mmol) in 0.8 mL of DCM, acetic anhydride (18 μL) was added. The reaction mixture was stirred at room temperature for 20 hours and evaporated in vacuum. The residue was dissolved in 4 mL of a 10% aqueous acetonitrile solution and purified by a HPLC reversed-phase column to obtain compound 259 as a white solid (27 mg, yield: 65%).

$^1H$ NMR ($CDCl_3$, 400 MHz):δ7.10 (d, J=7.3 Hz, 1H), 6.41 (d, J=8.7 Hz, 1H), 4.87 (quin, J=6.9, 1H), 4.37 (dd, J=6.1, 2.6 Hz, 1H), 3.09 (s, 3H), 2.99 (s, 3H), 2.10-2.02 (m, 4H), 1.34 (d, J=6.9 Hz, 3H), 0.94 (d, J=6.0 Hz, 3H), 0.92 (d, J=5.5 Hz, 3H).

MS (MALDI-TOF MS.m/z)

$[M+Na]^+$:calcd.for $C_{12}H_{23}N_3O_3Na$ 280.16, found 280.05.

[Example 16]

Ac-D,L-Val ($F_6$)-L-Ala-$NMe_2$ was synthesized.

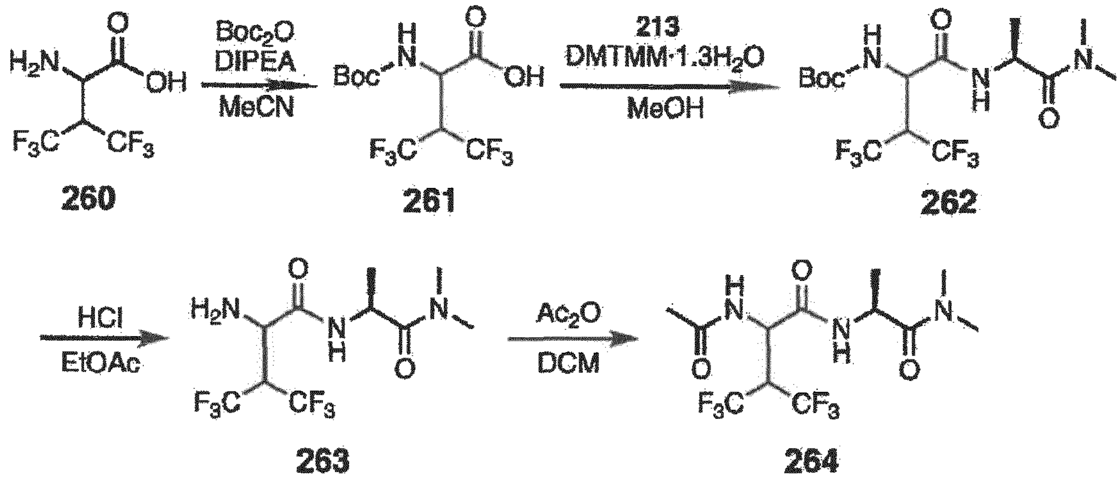


260 261 262 263 264

D,L-hexafluorovaline (compound 260) (50 mg, 0.22 mmol) was dissolved in 2.5 mL of acetonitrile. To the obtained solution, di-tert-butyl dicarbonate (49 μL) was added at 0°C. The reaction mixture was allowed to room temperature over a period of 11.5 hours. To the solution, DIPEA (38 μL) was added, and the reaction mixture was stirred at room temperature for 6.5 hours. The solution was evaporated in vacuum, and water was added to the residue. The resulting solution was extracted with diethyl ether. To the resulting aqueous phase, a 1M aqueous HCl solution was added, and the obtained solution was extracted with diethyl ether three times. The resulting organic phase put together was dried over $Na_2SO_4$ and evaporated in vacuum to obtain compound 261 (63 mg, yield: 87%).

The compound 261 (50 mg, 0.15 mmol) and the compound 213 (21 mg) were dissolved in 0.7 mL of methanol, and to the obtained solution, DMTMM·1.3$H_2O$ (56 mg) was added. The reaction mixture was stirred at room temperature for 17 hours and evaporated in vacuum. DCM was added to the reaction mixture, and the obtained solution was washed with a 1M aqueous $Na_2CO_3$ solution, water, a 1M aqueous HCl solution, water and brine. The resulting organic phase was dried over $Na_2SO_4$ and evaporated in vacuum to obtain compound 262 (50 mg, yield: 77%).

To the compound 262 (50 mg, 0.12 mmol), 4M HCl (2.5 mL) in ethyl acetate was added, and the obtained solution was stirred at room temperature for 2 hours. To the reaction mixture, ethyl acetate was added, and the obtained solution was extracted with a 1M aqueous HCl solution twice. To the resulting aqueous phase, a 1M aqueous NaOH

solution was added until the pH reached 11. The obtained solution was extracted with DCM three times, and the resulting organic phase was dried over $Na_2SO_4$. The solvent was removed under reduced pressure to obtain compound 263 (38 mg, quantitative yield).

To the compound 263 (38 mg, 0.12 mmol) in DCM (1.2 mL), acetic anhydride (13 μL) was added. The reaction mixture was stirred at room temperature for 13 hours and evaporated in vacuum. The residue was dissolved in 8 mL of a 30% aqueous acetonitrile solution and purified by a HPLC reversed-phase column to obtain compound 264 as a white solid (27 mg, yield: 63%). The molecules were obtained as a diastereomer mixture and were used for permeability assay as they were.

$^1H$ NMR ($CDCl_3$, 400 MHz):δ7.57 (d, J=6.4 Hz, 0.5H), 7.39 (d, J=6.9 Hz, 0.5H), 6.22 (d, J=9.6 Hz, 0.5H), 6.08 (d, J=9.6 Hz, 0.5H), 5.41 (t, J=9.2 Hz, 1H), 4.86-4.75 (m, 1H), 4.35-4.22 (m, 1H), 3.07 (d, J=5.0 Hz, 3H), 2.98 (d, J=3.2 Hz, 3H), 2.14 (d, J=2.14 Hz, 3H), 1.32 (t, J=7.3 Hz, 3H).

MS (MALDI-TOF MS.m/z)

$[M+Na]^+$:calcd.for $C_{12}H_{17}F_6N_3O_3Na$ 388.11, found 388.18.

[Comparative Example 8]

Ac-L-Val-L-Phe-iBu was synthesized.

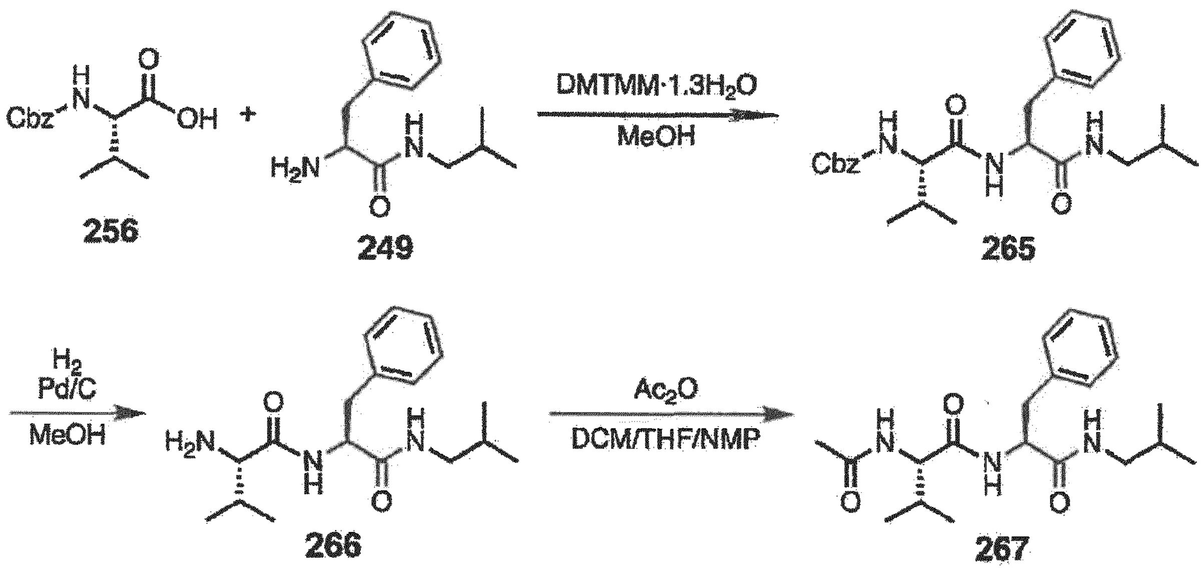


256 249 265 266 267

The compound 256 (52 mg, 0.2 mmol) and the compound 249 (53 mg) were dissolved in 2 mL of methanol, and the obtained solution was stirred at room temperature. To the solution, DMTMM·$1.3H_2O$ (72 mg) was added. The reaction mixture was stirred at room temperature for 23 hours and evaporated in vacuum. DCM was added to the reaction mixture, and the obtained solution was washed with a 1M aqueous $Na_2CO_3$ solution, water, a 1M aqueous HCl solution, water and brine. The resulting organic phase was dried over $Na_2SO_4$ and evaporated in vacuum. The residue was purified by silica gel column chromatography (DCM/methanol=19:1) to obtain compound 265 (60 mg, yield: 67%).

Into a recovery flask, the compound 265 (60 mg, 0.13 mmol), palladium carbon 10% (6 mg) and 1.3 mL of methanol were added. To the flask, $H_2$ was introduced, and the mixture was stirred at room temperature for 22 hours. The reaction mixture was subjected to filtration through celite. The solvent was removed under reduced pressure to obtain compound 266 (39 mg, yield: 92%).

The compound 266 (15 mg, 47 μmol) was dissolved in 1 mL of DCM, 0.4 mL of NMP and 0.2 mL of THF. To the obtained solution, acetic anhydride (23 μL) was added. The reaction mixture was stirred at room temperature for 1.5 hours and evaporated in vacuum. The residue was dissolved in acetonitrile/$H_2O$/MeOH (=1.8 mL:2.9 mL:1.5 mL) and purified by a HPLC reversed-phase column to obtain compound 267 (7.2 mg, yield: 43%).

$^1H$ NMR ($CDCl_3$, 400 MHz):δ7.30-7.18 (m, 5H), 6.51 (d, J=7.3 Hz, 1H), 5.92 (d, J=7.8 Hz, 1H), 5.75 (s, 1H), 4.58 (dt, J=6.0, 8.2 Hz, 1H), 4.20 (dd, J=6.0, 8.2 Hz, 1H), 3.11 (dd, J=6.0, 13.7 Hz, 1H), 3.02-2.92 (m, 3H), 2.10-2.03 (m, 1H), 1.97 (s, 3H), 1.66-1.59 (m, 1H), 0.88 (dd, J=6.9, 11.5 Hz, 6H), 0.76 (t, J=7.3 Hz, 6H).

MS (MALDI-TOF MS.m/z)

$[M+Na]^+$:calcd.for $C_{20}H_{31}N_3O_3Na$ 384.23, found 384.13.

[Example 17]

Ac-D,L-Val $(F_6)$-L-Phe-iBu was synthesized.

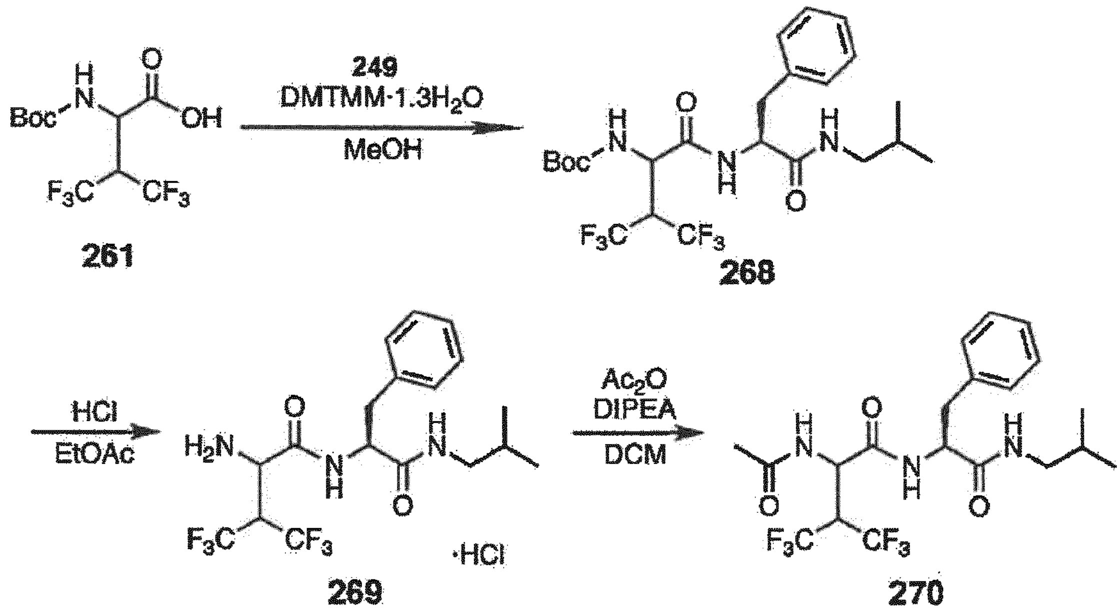


The compound 261 (70 mg, 0.22 mmol) and the compound 249 (57 mg) were dissolved in 1.2 mL of methanol, and to the solution, DMTMM·$1.3H_2O$ (83 mg) was added. The reaction mixture was stirred at room temperature for 5 hours and evaporated in vacuum. DCM was added to the reaction mixture, and the obtained solution was washed with a saturated aqueous $NaHCO_3$ solution, a 1M aqueous HCl solution and brine. The resulting organic phase was dried over $Na_2SO_4$ and evaporated in vacuum. The residue was purified by silica gel column chromatography (DCM/methanol=19:1) to obtain compound 268 (79 mg, yield: 69%).

To the compound 268 in 3 mL of ethyl acetate, 4M HCl (3 mL) in ethyl acetate was added. The obtained solution was stirred at room temperature for 1.5 hours. The solvent was removed under reduced pressure to obtain compound 269 (53 mg, yield: 76%).

To the compound 269 (40 mg, 86 μmol) in 2 mL of DCM, DIPEA (16 μL) and acetic anhydride (45 μL) were added. The reaction mixture was stirred at room temperature for 8.5 hours and evaporated in vacuum. The residue was dissolved in acetonitrile/$H_2O$/MeOH (=3 mL:3 mL:8 mL) and purified by a HPLC reversed-phase column to obtain compound 270 as a white solid (5.7 mg, yield: 13%). The molecules were obtained as a diastereomer mixture and were used for permeability assay as they were.

$^1H$ NMR ($CD_3OD_3$, 400 MHz):δ7.29-7.17 (m, 5H), 5.29 (dq, J=7.8, 23.8 Hz, 1H), 4.63-4.58 (m, 1H), 3.15-3.05 (m, 1H), 3.01-2.86 (m, 3H), 2.01 (d, J=4.6hz, 3H), 1.72-1.62 (m, 1H), 0.82-0.78 (m, 6H).

MS (MALDI-TOF MS.m/z)

$[M+Na]^+$:calcd.for $C_{20}H_{25}F_6N_3O_3Na$ 492.17, found 491.97.

[Test Example 3]

With respect to the peptides synthesized in Examples 15 to 17 and Comparative Examples 6 to 8, PAMPA assay was conducted. Further, with respect to the peptides synthesized in Examples 14, 15 and 17 and Comparative Examples 5,6 and 8, MDCK-II assay was conducted. For the MDCK-II assay, propranolol (CAS No: 318-98-9) was used as a positive control ("PC") and Norfloxacin (CAS No: 70458-96-7) as a negative control ("NC").

<PAMPA (parallel artificial membrane permeability assay)>

Permeability of the peptides was evaluated by PAMPA. In the PAMPA, 300 μL of a 5% DMSO-containing PBS was added to the respective wells of an accepter plate (MultiScreen 96-well transport receiver plate, manufactured by Merck), and 150 μL of a peptide solution (20 μM) dissolved in 5% DMSO/PBS was added to the respective wells of a donor plate (MultiScreen-IP filter plate, 0.45 μm, manufactured by Merck). A dodecane solution of 1% lecithin (from soybean) was subjected to ultrasonic treatment for 30 minutes before use, and 5 μL of the solution was applied to the membrane support (PVDF) of the respective wells of the donor plate. The donor plate was placed on the acceptor plate, and the plates were left to stand in an incubator at 25°C for 18 hours. The peptide concentration was determined by LC/MS. The experiment was conducted repeatedly three times. The permeability ($P_e$) was calculated in accordance with the following formulae.

$$P_e = -\frac{\ln[1 - C_A(t)/C_{equilibrium}]}{A \times (1/V_D + 1/V_A) \times t}$$

$$C_{equilibrium} = \frac{C_D(t) \times V_D + C_A(t) \times V_A}{V_D + V_A}$$

A: filter area (0.3 $cm^2$)
VD: volume of donor well (0.15 $cm^3$)
VA: volume of acceptor well (0.3 $cm^3$)
t: incubation time (s) (18 hours = 64800 s)
CD (t): compound concentration in donor well at time t
CA (t): compound concentration in acceptor well at time t

<MDCK-II assay>

MDCK-II cells were seeded on Cell Culture Inserts (Falcon) at $5.04 \times 10^4$ cells/mL, and 5 days after seeding, cell monolayer assay (MDCK-II assay) was conducted. The peptide stock solution was prepared at 2 mM in DMSO solution, and diluted with HBSS containing 20 mM of HEPES (pH 7.5) to prepare a 2 μM peptide solution using as a solvent 0.1% DMSO/HBSS (+), as a donor solution. The acceptor solution was a 0.1% DMSO solution using as a solvent HBSS (+) containing 20 mM of HEPES (pH 7.5). The apparent permeability ($P_{app}$) was determined by incubation from the apical side to the basolateral side of the peptide solution at 37°C at 5% $CO_2$ for 2 hours. The peptide concentration was analyzed by LC/MS. The experiment was conducted repeatedly three times. The permeability ($P_{app}$) was calculated in accordance with the following formula.

$$P_{app} = \frac{Q(t)/t}{A \times C_0}$$

$$Q(t) = C_B(t) \times V_B$$

A: filter area (0.3 $cm^2$)
VB: basolateral well volume (0.75 $cm^3$)
t: incubation time (s) (2 hours =7200 s)
C0: initial concentration in apical chamber (2 μM)
CB (t): basolateral compound concentration at time t

The results of the PAMPA assay are shown in Table 3, and the results of the MDCK-II assay are shown in Table 4. As a result, as compared with the peptides in Comparative Examples 5 to 8, the fluorine-containing peptides in Examples 14 to 17, having fluorine atoms introduced to side chains of the peptides in Comparative Examples 5 to 8, had improved cell permeability.

[Table 3]

| | Peptide | Pe $10^{-6}$ cm $s^{-1}$ | Stdev | |
|---|---|---|---|---|
| Comparative Example 6 | Ac-L-Ala-L-Phe-iBu | 0.02 | 0.00 | P <0.01 |
| Example 15 | Ac-L-Ala (F3)-L-Phe-iBu | 0.19 | 0.00 | |
| Comparative Example 7 | Ac-L-Val-L-Ala-NMe2 | At most 0.003 | - | P <0.05 |
| Example 16 | Ac-L-Val (F6)-L-Ala-NMe2 | 0.04 | 0.01 | |
| Comparative Example 8 | Ac-L-Val-L-Phe-iBu | 0.07 | 0.01 | P <0.01 |
| Example 17 | Ac-L-Val (F6)-L-Phe-iBu | 1.57 | 0.05 | |

[Table 4]

| | Peptide | Papp $10^{-6}$ cm $s^{-1}$ | Stdev | |
|---|---|---|---|---|
| Comparative Example 5 | Ac-L-Ala-L-Ala-NHBn | 1.23 | 0.24 | P <0.01 |
| Example 14 | Ac-D,L-Ala (F3)-L-Ala-NHBn | 3.26 | 0.28 | |
| Comparative Example 6 | Ac-L-Ala-L-Phe-iBu | 2.52 | 0.50 | P <0.01 |
| Example 15 | Ac-L-Ala (F3)-L-Phe-iBu | 9.86 | 0.55 | |
| Comparative Example 8 | Ac-L-Val-L-Phe-iBu | 7.12 | 0.18 | P <0.01 |
| Example 17 | Ac-L-Val (F6)-L-Phe-iBu | 8.38 | 0.21 | |

INDUSTRIAL APPLICABILITY

The present invention relates to a peptide having an amino acid residue having a fluoroalkyl group as its side chain, and a method for producing it. The peptide according to the present invention is excellent in cell permeability and is thereby expected to be useful in medical fields as a physiologically active substance, for example, as a carrier to introduce a therapeutic component to target cells.

## Claims

**1.** A method for producing a fluoroalkyl group-containing peptide, which comprises condensing a compound represented by the following formula (6-2) or (6-4):

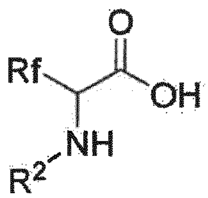

(6-2)

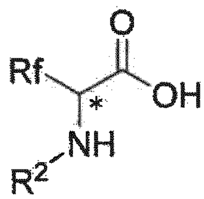

(6-4)

wherein asterisk means that the asymmetric carbon atom marked with the asterisk has an absolute configuration of S or R,

Rf is a $C_{1-30}$ alkyl group which is substituted with at least two fluorine atoms, and which may further be substituted with a halogen atom other than a fluorine atom (when the $C_{1-30}$ alkyl group is a $C_{2-30}$ alkyl group, it may have 1 to 5 etheric oxygen atoms between carbon atoms), and
$R^2$ is a protecting group for the amino group,
with a fluorinated amino acid having its carboxy group protected, an amino acid having its carboxy group

protected, a fluorinated peptide having its C-terminal protected, or a peptide having its C-terminal protected.

**2.** A method for producing a fluoroalkyl group-containing peptide, which comprises condensing a compound represented by the following formula (6-1) or (6-3):

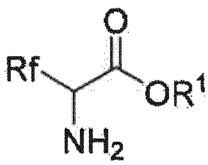
(6-1)

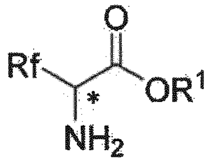
(6-3)

wherein asterisk means that the asymmetric carbon atom marked with the asterisk has an absolute configuration of S or R,

Rf is a $C_{1-30}$ alkyl group which is substituted with at least two fluorine atoms, and which may further be substituted with a halogen atom other than a fluorine atom (when the $C_{1-30}$ alkyl group is a $C_{2-30}$ alkyl group, it may have 1 to 5 etheric oxygen atoms between carbon atoms), and
$R^1$ is a protecting group selected from a group represented by the following formula (p-1):

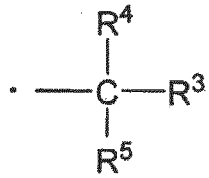
(p-1)

(wherein $R^3$ is a $C_{6-14}$ aryl group which may be substituted, $R^4$ and $R^5$ are each independently a hydrogen atom or a $C_{6-14}$ aryl group which may be substituted, and the black circle means a binding site), a 2-(9,10-dioxo)anthrylmethyl group, a benzyloxymethyl group and a phenacyl group,
with a fluorinated amino acid having its amino group protected, an amino acid having its amino group protected, a fluorinated peptide having its N-terminal protected, or a peptide having its N-terminal protected.

**3.** A method for producing a fluoroalkyl group-containing peptide, which comprises protecting, of a compound represented by the following formula (7) or (7-1):

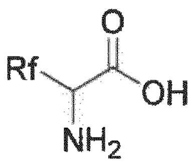
(7)

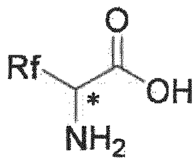
(7-1)

wherein asterisk means that the asymmetric carbon atom marked with the asterisk has an absolute configuration of S or R, and

Rf is a $C_{1-30}$ alkyl group which is substituted with at least two fluorine atoms, and which may further be substituted with a halogen atom other than a fluorine atom (when the $C_{1-30}$ alkyl group is a $C_{2-30}$ alkyl group, it may have 1 to 5 etheric oxygen atoms between carbon atoms),
the amino group with a protecting group, and then condensing the compound with a fluorinated amino acid having its carboxy group protected, an amino acid having its carboxy group protected, a fluorinated peptide having its C-terminal protected, or a peptide having its C-terminal protected, or
the carboxy group with a protecting group, and then condensing the compound with a fluorinated amino acid having its amino group protected, an amino acid having its amino group protected, a fluorinated peptide having

its N-terminal protected, or a peptide having its N-terminal protected.

**4.** The method for producing a fluoroalkyl group-containing peptide according to any one of Claims 1 to 3, which further comprises removing the protecting group for the amino group or for the carboxy group of the produced fluoroalkyl group-containing peptide by deprotection.

**5.** A peptide having two or more amino acids bonded by a peptide bond, wherein at least one of amino acid residues constituting the peptide has, as its side chain, a $C_{1-30}$ alkyl group substituted with at least two fluorine atoms (when the $C_{1-30}$ alkyl group has two or more carbon atoms, it may have an etheric oxygen atom between carbon atoms).

**6.** The peptide according to Claim 5, wherein the $C_{1-30}$ alkyl group substituted with at least two fluorine atoms may further be substituted with a halogen atom other than a fluorine atom.

**7.** The peptide according to Claim 5 or 6, wherein the side chain consisting of the $C_{1-30}$ alkyl group substituted with at least two fluorine atoms is a group represented by the following formula (f-1) or (f-2):

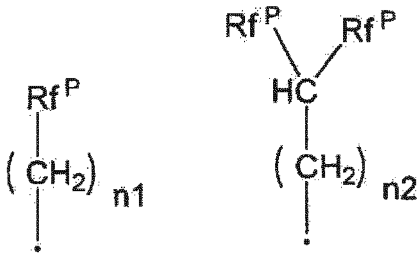

(f-1) (f-2)

wherein $Rf^P$ is a perhalogenated $C_{1-10}$ alkyl group containing at least two fluorine atoms (when the $C_{1-10}$ alkyl group has two or more carbon atoms, it may have an etheric oxygen atom between carbon atoms), n1 is an integer of from 0 to 10, n2 is an integer of from 0 to 9, and the black circle means a binding site.

**8.** The peptide according to any one of Claims 5 to 7, of which the C-terminal or the N-terminal may be protected with a protecting group.

**9.** The peptide according to any one of Claims 5 to 8, which is a tripeptide represented by the following formula (101) or (102):

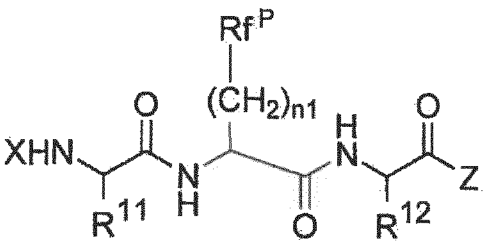

(101)

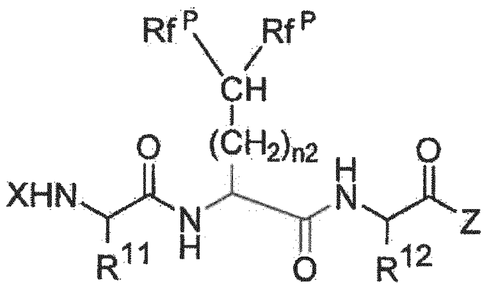

(102)

wherein $Rf^P$ is a perhalogenated $C_{1-10}$ alkyl group containing at least two fluorine atoms (when the $C_{1-10}$ alkyl group has two or more carbon atoms, it may have an etheric oxygen atom between carbon atoms), n1 is an integer of from 0 to 10, n2 is an integer of from 0 to 9, $R^{11}$ and $R^{12}$ are each independently a $C_{1-6}$ alkyl group or a benzyl group, X is a 9-fluorenylmethyloxycarbonyl group or a tert-butoxycarbonyl group, and Z is a $C_{1-6}$ alkoxy group.

**10.** The peptide according to any one of Claims 5 to 9, which is a cell penetrating peptide.

Fig. 1

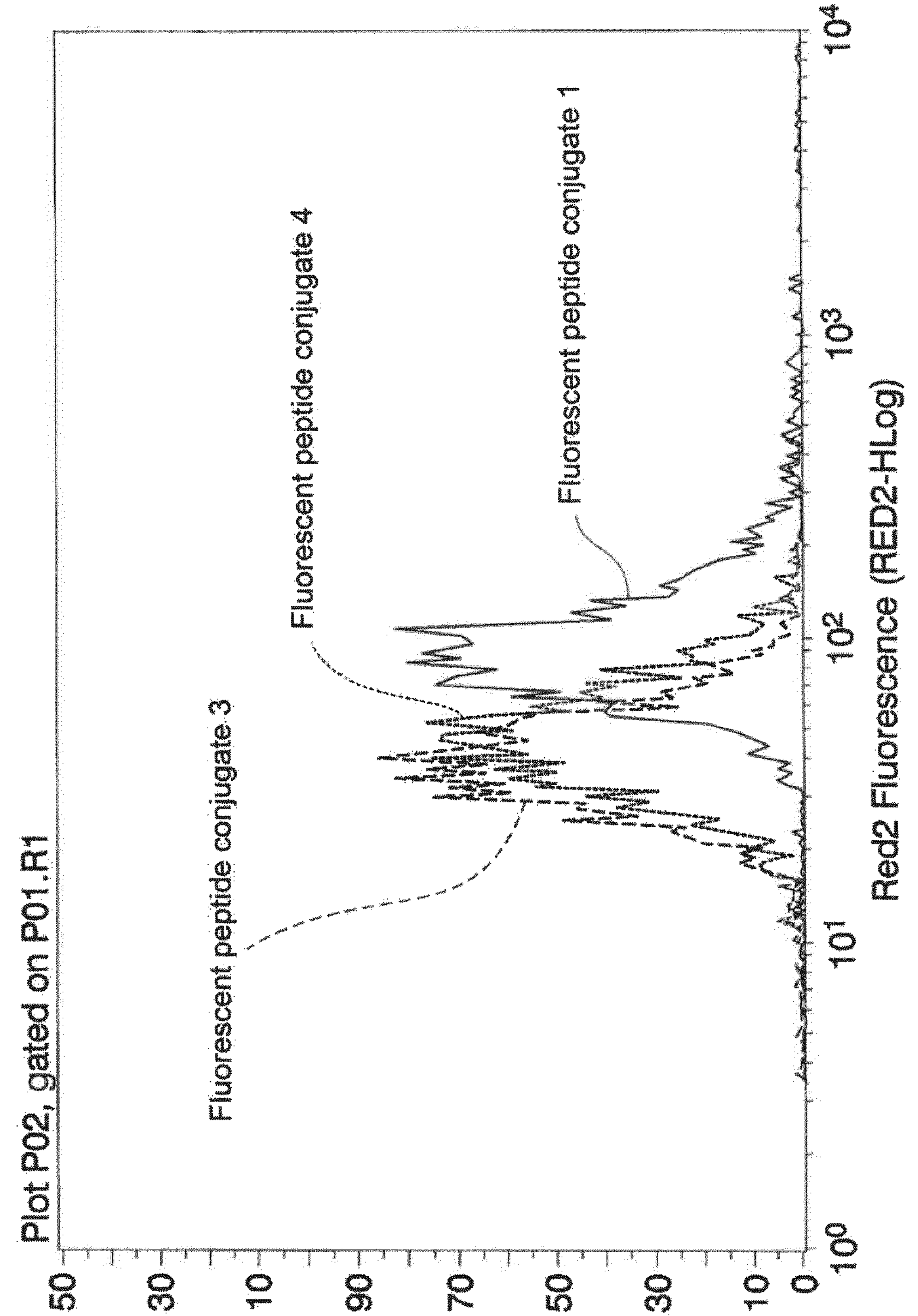
Plot P02, gated on P01.R1
Count
150
130
110
90
70
50
30
10
0
$10^0$
$10^1$
$10^2$
$10^3$
$10^4$
Red2 Fluorescence (RED2-HLog)
Fluorescent peptide conjugate 3
Fluorescent peptide conjugate 4
Fluorescent peptide conjugate 1

Fig. 2

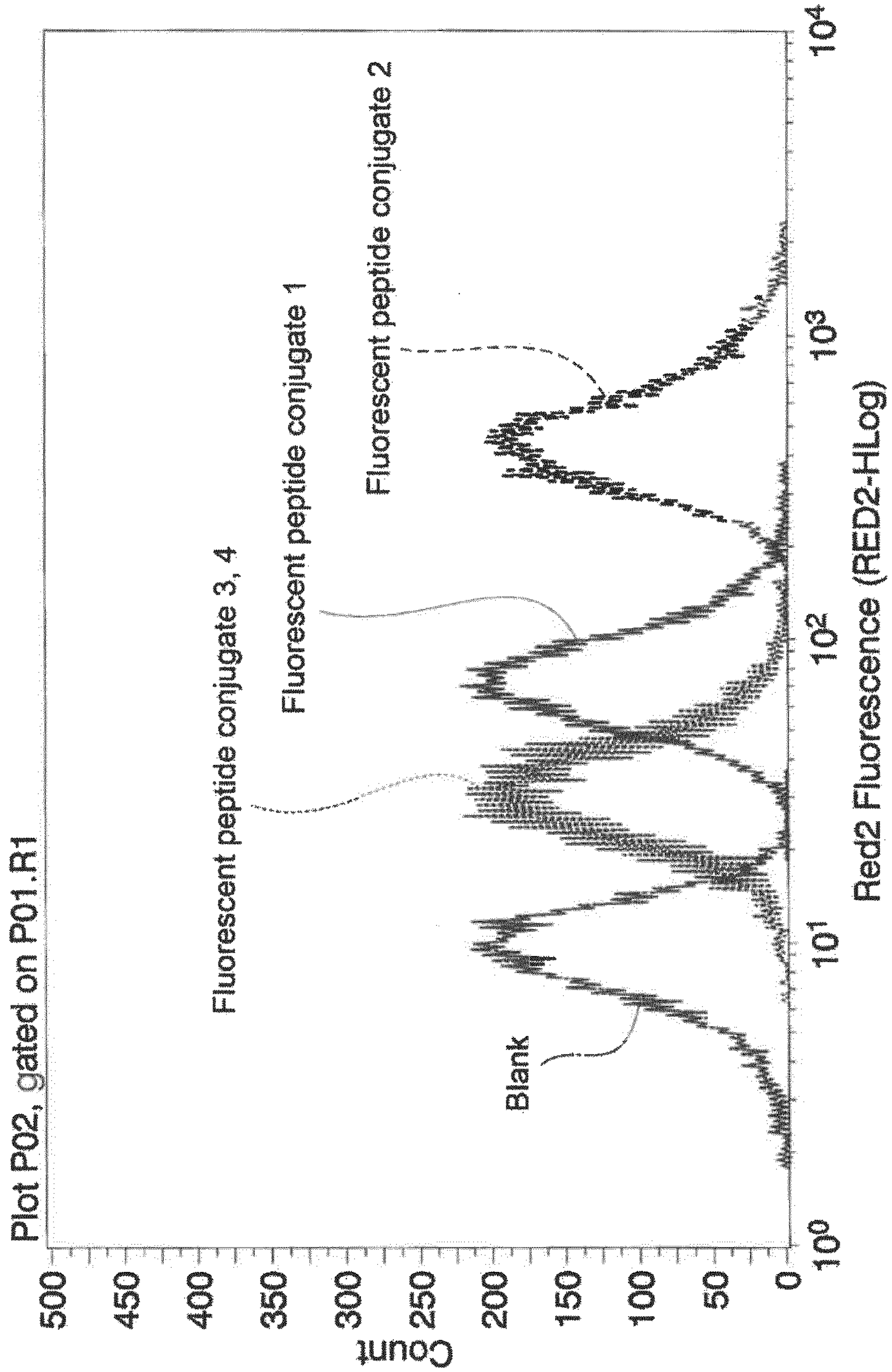
Plot P02, gated on P01.R1
Count
500
450
400
350
300
250
200
150
100
50
0
$10^0$
$10^1$
$10^2$
$10^3$
$10^4$
Red2 Fluorescence (RED2-HLog)
Blank
Fluorescent peptide conjugate 3, 4
Fluorescent peptide conjugate 1
Fluorescent peptide conjugate 2

| INTERNATIONAL SEARCH REPORT | International application No. PCT/JP2020/025912 |
|---|---|

**A. CLASSIFICATION OF SUBJECT MATTER**

C07C 231/12(2006.01)i; C07C 237/12(2006.01)i; C07C 269/06(2006.01)i; C07C 271/22(2006.01)i; C07K 1/06(2006.01)i; C07K 1/10(2006.01)i; C07K 5/00(2006.01)i; C07K 5/08(2006.01)i; C07K 5/083(2006.01)i

FI: C07K1/06; C07K5/00; C07K5/083; C07C269/06; C07C271/22; C07C231/12; C07C237/12; C07K5/08; C07K1/10

According to International Patent Classification (IPC) or to both national classification and IPC

**B. FIELDS SEARCHED**

Minimum documentation searched (classification system followed by classification symbols)

C07C231/12; C07C237/12; C07C269/06; C07C271/22; C07K1/06; C07K1/10; C07K5/00; C07K5/08; C07K5/083

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | CHIU, Hsien-Po et al., "Helix Propensity of Highly Fluorinated Amino Acids", J. AM. CHEM. SOC., 2006, 128, 15556-15557, page 15556 | 1-10 |
| X | LEE, Kyung-Hoon et al., "Fluorous Effect in Proteins: De Novo Design and Characterization of a Four-α-Hel ix Bundle Protein Containing Hexafluoroleucine", Biochemistry, 2004, 43, 16277-16284, abstract, fig. 2, page 16278 | 1-10 |
| A | US 2010/0099845 A1 (UNIVERSITY OF UTAH RESEARCH FOUNDATION) 22.04.2010 (2010-04-22) paragraph [0089] | 10 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 August 2020 (24.08.2020) | 08 September 2020 (08.09.2020) |
| Name and mailing address of the ISA/ Japan Patent Office 3-4-3, Kasumigaseki, Chiyoda-ku, Tokyo 100-8915, Japan | Authorized officer<br>Telephone No. |

Form PCT/ISA/210 (second sheet) (January 2015)

**INTERNATIONAL SEARCH REPORT**

International application No.
PCT/JP2020/025912

C (Continuation). DOCUMENTS CONSIDERED TO BE RELEVANT

| Category* | Citation of document, with indication, where appropriate, of the relevant passages | Relevant to claim No. |
|---|---|---|
| A | US 2009/0259020 A1 (UNIVERSITY OF UTAH RESEARCH FOUNDATION) 15.10.2009 (2009-10-15) paragraph [0067] | 10 |
| A | ABE, Hajime et al., "Pd-Catalyzed Asymmetric Hydrogenation of α-Fluorinated Iminoesters in Fluorinated Alcohol: A New and Catalytic Enantioselective Synthesis of Fluoro α-Amino Acid Derivatives", ORGANIC LETTERS, 2001, vol. 3, no. 3, pp. 313-315, table 2 | 1-10 |
| P,X | 三上 峻輝 ほか, 新規含フッ素アミノ酸の合成と細胞膜透過性ペプチドへの応用, 第 42 回フッ素化学討論会 講演要旨集, 21 November 2019, pp. 142-143, P38, 2. Results and discussion, non-official translation (MIKAMI, Toshiki et al., "Synthesis of novel fluorine-containing amino acids and application to cell membrane permeable peptides", Lecture abstracts of the 42nd Fluorine Conference of Japan) | 1-10 |

Form PCT/ISA/210 (continuation of second sheet) (January 2015)

**INTERNATIONAL SEARCH REPORT**
Information on patent family members

International application No.
PCT/JP2020/025912

| Patent Documents referred in the Report | Publication Date | Patent Family | Publication Date |
| --- | --- | --- | --- |
| US 2010/0099845 A1 | 22 Apr. 2010 | WO 2008/034095 A2 | |
| US 2009/0259020 A1 | 15 Oct. 2009 | WO 2008/034093 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019124016 A
- JP 2019209908 A
- US 6316003 B
- US 6306993 B
- WO 2008089491 A


### Non-patent literature cited in the description


- **MIYAJI et al.** *Drug Metabolism and Disposition,* 2011, vol. 39, 1946-1953
- **SAKAI et al.** *Tetrahedron,* 1996, vol. 52 (1), 233-244
- **FARACI ; WALSH.** *Biochemistry,* 1989, vol. 28 (2), 431-437
- **ZHANG et al.** *MRS Communications,* 2018, vol. 8, 303-313
- **CAI et al.** *ACS Applied Materials and Interfaces,* 2016, vol. 8, 5821-5832
- *Journal of Fluorine Chemistry,* 1984, vol. 26, 341-358
- *Chemical and Pharmaceutical Bulletin,* 1987, vol. 35, 468
- *Liebigs Annalen der Chemie,* 1990, vol. 12, 1175-1183